# EUROPEAN PATENT SPECIFICATION

(11) **EP 2 787 978 B1**
(45) Date of publication and mention of the grant of the patent: **21.09.2016**
(21) Application number: 12813099.4
(22) Date of filing: 07.12.2012
(51) Int. Cl.: A61K 9/16, A61K 9/20, A61K 9/48

(54) **PHARMACEUTICAL DOSAGE FORMS COMPRISING POLY(EPSILON-CAPROLACTONE) AND POLYETHYLENE OXIDE**
PHARMAZEUTISCHE DARREICHUNGSFORMEN MIT POLY(EPSILON-CAPROLACTON) UND POLYETHYLENOXID
FORMES DOSIFIÉES PHARMACEUTIQUES COMPRENANT DE LA POLY(OXEPAN-2-ONE) ET UN POLYÉTHYLÈNE OXYDE

(30) Priority: 09.12.2011 US 201161569193 P
(43) Date of publication of application: 15.10.2014
(73) Proprietor: Purdue Pharma LP, Stamford, CT 06901-3431 (US)
(72) Inventor: MULEY, Sheetal, Piscataway, NJ 08854 (US)
(74) Representative: Ehlich, Eva Susanne
(86) International application number: PCT/IB2012/002681
(87) International publication number: WO 2013/084059

(56) References cited:
- EP-A1- 1 897 545
- EP-A2- 0 147 335
- WO-A1-2010/032128
- WO-A2-98/20073
- WO-A2-2005/081825
- WO-A2-2007/070632
- DE-A1-102005 005 446
- US-A1- 2007 014 846

## Description

### TECHNICAL FIELD OF THE INVENTION

The present invention relates to tamper resistant pharmaceutical dosage forms including an active agent, and processes of manufacture, uses thereof, and corresponding methods of treatment therewith.

### BACKGROUND OF THE INVENTION

Pharmaceutical products and in particular extended release dosage forms, which usually comprise a larger amount of active agent in a single dose, are increasingly the subject of abuse. For example, a particular dose of active agent, e.g. opioid analgesic, may be more potent when administered parenterally as compared to the same dose administered orally. Some formulations can be tampered with to provide the active agent, e.g. the opioid analgesic, contained therein for illicit use.

Extended release opioid analgesic formulations are sometimes crushed or subject to extraction with solvents (e.g. ethanol) by drug abusers to provide the opioid contained therein for immediate release upon oral or parenteral administration.

Extended release dosage forms that can liberate a portion of the active agent upon exposure to ethanol can also result in a patient receiving the dose more rapidly than intended if the patient concomitantly uses alcohol with the dosage form.

There continues to exist a need in the art for extended release pharmaceutical dosage forms comprising an active agent that resist illicit use. In particular, there continues to exist a need for extended release pharmaceutical dosage forms comprising an active agent, e.g. an opioid analgesic, with resistance to crushing and/or without significantly changed active agent release properties when in contact with alcohol.

WO 2010/032128 (A1) relates to pharmaceutical dosage forms, for example to pharmaceutical dosage forms comprising poly(ε-caprolactone), and processes of manufacture, uses, and methods of treatment thereof, in particular to a solid oral extended release pharmaceutical dosage form, comprising a melt formed multi particulate extended release matrix formulation, comprising at least one poly(ε-caprolactone), and at least one active agent.

US 2007/014846 (A1) relates to pharmaceutical compositions in particulate form or in solid dosage forms comprising a combination of fenofibrate and the HMG CoA reductase inhibitor atorvastatin or a pharmaceutically active salt thereof, which upon oral administration provides a relative AUC0-24 value (AUCfibric acid/AUCatorvastatin) of between about 250 and about 10,000. The solid compositions are manufactured without any need of addition of water or aqueous medium.

DE10 2005 005446 (A1) relates to a dosage form comprising a physiologically active sustance (A) with at least partially sustained release; optionally one or more physiologically accepetable excipients (B); and a synthetic or a natural polymer (C); wherein the dosage form exhibits a resistance to crushing of at least 400 N.

### OBJECTS AND SUMMARY OF THE INVENTION

It is an object of certain embodiments of the present invention to provide a solid extended release dosage form comprising an active agent, e.g. an opioid analgesic, which is tamper resistant.

It is an object of certain embodiments of the present invention to provide a solid extended release dosage form comprising an active agent, e.g. an opioid analgesic, which is resistant to crushing.

It is an object of certain embodiments of the present invention to provide a solid extended release dosage form comprising an active agent, e.g. an opioid analgesic, which is resistant to alcohol extraction.

It is an object of certain embodiments of the present invention to provide a solid extended release dosage form comprising an active agent, e.g. an opioid analgesic, which is resistant to crushing and resistant to alcohol extraction.

It is an object of certain embodiments of the present invention to provide a solid extended release dosage form comprising an active agent, e.g. an opioid analgesic, in an extended release matrix formulation, wherein the extended release matrix formulation is manufactured by a continuous process, e.g. by a melt extrusion method, which is resistant to crushing and/or resistant to alcohol extraction.

It is an object of certain embodiments of the present invention to provide a solid extended release dosage form comprising an active agent, e.g. an opioid analgesic, in an extended release matrix formulation, wherein the extended release matrix formulation is manufactured by a continuous process, e.g. by a melt extrusion method, wherein the extended release matrix formulation includes poly(ε-caprolactone) and polyethylene oxide and is resistant to alcohol extraction.

These objects and others are accomplished by the present invention, which according to one aspect relates to a solid extended release pharmaceutical dosage form comprising a mixture in the form of an extended release matrix formulation, the mixture comprising at least:
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) at least one active agent.

According to one aspect, the invention relates to a solid extended release pharmaceutical dosage form, comprising a mixture in the form of an extended release matrix formulation, the mixture comprising at least:
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) at least one active agent,
wherein the extended release matrix formulation is shaped by a melt extrusion method.

According to one aspect, the invention relates to a solid extended release pharmaceutical dosage form, comprising a mixture in the form of an extended release matrix formulation, the mixture comprising at least:
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) 5 mg to 500 mg of oxycodone hydrochloride; and
wherein the dosage form provides an in-vitro dissolution rate of the active agent, when measured by the USP Basket Method at 100 rpm at 900 ml simulated gastric fluid at 37 °C, from about 10 % to about 30 % (by wt) active agent released after 30 minutes, from about 20 % to about 50 % (by wt) active agent released after 60 minutes, from about 30 % to about 65 % (by wt) active agent released after 120 minutes, from about 45 % to about 85 % (by wt) active agent released after 240 minutes, and from about 60 % to about 95 % (by wt) active agent released after 360 minutes.

According to one aspect, the invention relates to a solid extended release pharmaceutical dosage form in the form of a tablet, a suppository or multi-particulates, comprising a mixture in the form of an extended release matrix formulation, the mixture comprising at least:
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) at least one active agent selected from opioid analgesics; and
wherein the tablet, a suppository or the multi-particulates provide an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 30 minutes of dissolution that deviates no more than 10 % points from the corresponding in-vitro dissolution rate measured in a USP Apparatus 1 (basket) at 100 rpm in 900 ml simulated gastric fluid (SGF) at 37° C without ethanol.

According to one aspect, the invention relates to a solid extended release pharmaceutical dosage form, comprising a mixture in the form of an extended release matrix formulation, the mixture comprising at least:
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) at least one active agent selected from opioid analgesics; and
wherein the dosage form, after crushing for 60 seconds in a coffee mill, provides an amount of material retained by a mesh #30 of at least about 80 % of the initial amount of the dosage form.

According to preferred embodiments, the invention relates to a solid oral extended release pharmaceutical dosage form.

Within the meaning of this invention, the term "extended release" refers to products that provide a release of the active agent of less than 100 % after 60 minutes *in* vitro when measured in a USP Apparatus 1 (basket) at 100 rpm in 900 ml simulated gastric fluid at 37° C.

Within the meaning of this invention, the term "immediate release" refers to products which provide a release of active agent of at least 100 % in 60 minutes *in vitro* when measured in a USP Apparatus 1 (basket) at 100 rpm in 900 ml simulated gastric fluid at 37° C.

Within the meaning of this invention the term "solid extended release pharmaceutical dosage form", in particular "solid oral extended release pharmaceutical dosage form" refer to the administration form comprising a unit dose of active agent in extended release form, i.e. in an extended release matrix formulation, and optionally other adjuvants and additives conventional in the art, such as a protective coating or an additional prolonged release coating or a capsule and the like, and optionally any other additional features or components that are used in dosage forms. Unless specifically indicated, the term "solid extended release pharmaceutical dosage form", in particular "solid oral extended release pharmaceutical dosage form" refer to said dosage form in intact form, i.e. prior to any tampering. The extended release pharmaceutical dosage form can, e.g., be a tablet comprising the extended release matrix formulation or a capsule comprising the extended release matrix formulation in the form of multi-particulates or a suppository. The "solid extended release pharmaceutical dosage form", in particular the "solid oral extended release pharmaceutical dosage form" may comprise a portion of active agent in extended release form and another portion of active agent in immediate release form, e.g. as an immediate release layer of active agent surrounding the dosage form or an immediate release component included within the dosage form.

Within the meaning of this invention, the term "extended release matrix formulation" refers to the shaped solid form of a mixture comprising at least one active agent and at least one poly(ε-caprolactone) and at least one polyethylene oxide. The shape can be a tablet or multi-particulates, or a suppository. The "extended release matrix formulation" can optionally comprise more than these components, namely one or more additional active agents and/or additional retardants and/or other materials and/or other adjuvants and/or other additives conventional in the art.

Within the meaning of this invention, the term "retardant" refers to a component which contributes to the prolongation of the dissolution rate of the active agent present in the extended release matrix formulation when measured in a USP Apparatus 1 (basket) at 100 rpm in 900 ml simulated gastric fluid at 37° C. Poly(ε-caprolactone) and polyethylene oxide as described herein are retardants within the meaning of the present invention.

Within the meaning of this invention, the term "active agent" is defined as a pharmaceutically active substance, which includes without limitation opioids, in particular opioid analgesics, but also pure opioid antagonists which provide no analgesic effect. Opioids used according to the invention may contain one or more asymmetric centers and may give rise to enantiomers, diastereomers, or other stereoisomeric forms. The present invention is intended to encompass the use of all such possible forms as well as their racemic and resolved forms and compositions thereof. When the compounds described herein contain olefinic double bonds or other centers of geometric asymmetry, is the term "active agent" is intended to include both E and Z geometric isomers. All tautomers of any such compounds are intended to be encompassed by the present invention as well.

Within the meaning of this invention, the term "opioid analgesic" includes single compounds and combinations of compounds selected from the group of opioids and which provide an analgesic effect such as one single opioid agonist or a combination of opioid agonists, and also combinations of opioid agonists and opioid antagonists which provide an analgesic effect.

Within the meaning of this invention the term "stereoisomers" is a general term for all isomers of individual molecules that differ only in the orientation of their atoms is space. It includes enantiomers and isomers of compounds with more than one chiral center that are not mirror images of one another (diastereomers).

Within the meaning of this invention, the term "chiral center" refers to a carbon atom to which four different groups are attached.

Within the meaning of this invention, the term "enantiomer" or "enantiomeric" refers to a molecule that is non-superimposable on its mirror image and hence optically active wherein the enantiomer rotates the plane of polarized light in one direction by a certain degree and its mirror image rotates the plane of polarized light by the same degree but in the opposite direction.

Within the meaning of this invention, the term "racemic" refers to a mixture of equal parts of enantiomers and which is optically inactive.

Within the meaning of this invention, the term "resolution" refers to the separation or concentration or depletion of one of the two enantiomeric forms of a molecule.

Within the meaning of this invention, the term "opioid antagonist" includes single compounds and combinations of compounds selected from the group of receptor antagonists that act at least partially on opioid receptors, but do not provide an analgesic effect.

The term "poly(ε-caprolactone)" may, for the purpose of the invention, be abbreviated by PCL and refers to a PCL-homopolymer. The molecular weight of poly(ε-caprolactone) for the purpose of the present invention relates to a number average molecular weight. The molecular weight of up to about 10,000 is defined by a molecular weight determined using the viscosity at 25 degrees Celsius. The molecular weight above 10,000 and up to 80,000 is defined by a molecular weight determined using the melt flow index. The molecular weight above 80,000 is defined by a molecular weight determined using the inherent viscosity at 25 degrees Celsius measured by an Ubbelohde capillary viscometer method in chloroform. Poly(ε-caprolactone) is also considered to have an approximate number average molecular weight of up to 80,000 in accordance with the definition when it has a molecular weight of up to 80,000 in accordance with the inherent viscosity when determined by Ubbelohde capillary viscometer method in chloroform. Poly(ε-caprolactone) is considered to have an approximate number average molecular weight of 10,000 when the viscosity is 400-1000 MPA at 25°C. Poly(ε-caprolactone) is considered to have an approximate number average molecular weight of 37,000 when the melt flow index is 40 g/10 minutes at 160°C and 2.16 kg. Poly(ε-caprolactone) is considered to have an approximate number average molecular weight of 42,500 when the melt flow index is 1.8 G/10 minutes at 80°C and 44 psi. Poly(ε-caprolactone) is considered to have an approximate number average molecular weight of 80,000 when the melt flow index is 1.0 G/10 minutes at 80°C and 44 psi. Poly(ε-caprolactone) is considered to have an approximate number average molecular weight of 78,000 when the inherent viscosity is 1.04 dl/g at 25°C when determined by Ubbelohde capillary viscometer method in chloroform at a concentration of 0.1 g/dl. Poly(ε-caprolactone) is considered to have an approximate number average molecular weight of 98,000 when the inherent viscosity is 1.24 d1/g at 25°C when determined by Ubbelohde capillary viscometer method in chloroform at a concentration of 0.1 g/dl. Poly(ε-caprolactone) is considered to have an approximate number average molecular weight of 107,000 when the inherent viscosity is 1.33 dl/g at 25°C when determined by Ubbelohde capillary viscometer method in chloroform at a concentration of 0.1 g/dl. Poly(ε-caprolactone) is considered to have an approximate number average molecular weight of 154,000 when the inherent viscosity is 1.80 dl/g at 25°C when determined by Ubbelohde capillary viscometer method in chloroform at a concentration of 0.1 g/dl.

The term "polyethylene oxide" may for the purpose of the invention be abbreviated by PEO and refers to a PEO-homopolymer. The molecular weight of polyethylene oxide for the purpose of the present invention relates to a weight average molecular weight. For the purpose of this invention the approximate molecular weight is based on rheological measurements. Polyethylene oxide is considered to have an approximate weight average molecular weight of 100,000 when a 5 % (by wt) aqueous solution of said polyethylene oxide using a Brookfield viscometer Model RVT, spindle No. 1, at 50 rpm, at 25°C shows a viscosity range of 30-50 mPa s (cP). Polyethylene oxide is considered to have an approximate weight average molecular weight of 900,000 when a 5 % (by wt) aqueous solution of said polyethylene oxide using a Brookfield viscometer Model RVF, spindle No. 2, at 2 rpm, at 25°C shows a viscosity range of 8,800-17,600 mPa s (cP). Polyethylene oxide is considered to have an approximate molecular weight of 1,000,000 when a 2% (by wt) aqueous solution of said polyethylene oxide using a Brookfield viscometer Model RVF, spindle No. 1, at 10 rpm, at 25°C shows a viscosity range of 400 to 800 mPa s (cP). Polyethylene oxide is considered to have an approximate molecular weight of 2,000,000 when a 2% (by wt) aqueous solution of said polyethylene oxide using a Brookfield viscometer Model RVF, spindle No. 3, at 10 rpm, at 25°C shows a viscosity range of 2000 to 4000 mPa s (cP). Polyethylene oxide is considered to have an approximate molecular weight of 4,000,000 when a 1% (by wt) aqueous solution of said polyethylene oxide using a Brookfield viscometer Model RVF, spindle No. 2, at 2 rpm, at 25°C shows a viscosity range of 1650 to 5500 mPa s (cP). Polyethylene oxide is considered to have an approximate molecular weight of 5,000,000 when a 1% (by wt) aqueous solution of said polyethylene oxide using a Brookfield viscometer Model RVF, spindle No. 2, at 2 rpm, at 25°C shows a viscosity range of 5500 to 7500 mPa s (cP). Polyethylene oxide is considered to have an approximate molecular weight of 7,000,000 when a 1% (by wt) aqueous solution of said polyethylene oxide using a Brookfield viscometer Model RVF, spindle No. 2, at 2 rpm, at 25°C shows a viscosity range of 7500 to 10,000 mPa s (cP). Polyethylene oxide is considered to have an approximate molecular weight of 8,000,000 when a 1% (by wt) aqueous solution of said polyethylene oxide using a Brookfield viscometer Model RVF, spindle No. 2, at 2 rpm, at 25°C shows a viscosity range of 10,000 to 15,000 mPa s (cP). Regarding the lower molecular weight polyethylene oxides; Polyethylene oxide is considered to have an approximate molecular weight of 100,000 when a 5% (by wt) aqueous solution of said polyethylene oxide using a Brookfield viscometer Model RVT, spindle No. 1, at 50 rpm, at 25°C shows a viscosity range of 30 to 50 mPa s (cP) and polyethylene oxide is considered to have an approximate molecular weight of 900,000 when a 5% (by wt) aqueous solution of said polyethylene oxide using a Brookfield viscometer Model RVF, spindle No. 2, at 2 rpm, at 25°C shows a viscosity range of 8800 to 17,600 mPa s (cP).

Within the meaning of this invention, the term "multi-particulates" refers to a possible shape of the extended release matrix formulation which requires at least two individual units in the dosage form. In comparison to a tablet, which includes an undivided dose of active agent, multi-particulates include a divided dose of active agent in the dosage form.

Within the meaning of this invention, the terms "thermo-treated", "thermo-treatment", and the like refer to a process which includes at least a step of subjecting poly(ε-caprolactone), or polyethylene oxide, or the mixture comprising at least one active agent and/or at least one poly(ε-caprolactone) and/or at least one polyethylene oxide, or the extended release matrix formulation to an elevated temperature.

Within the meaning of this invention, the term "cured" refers to a process by which firstly the mixture is shaped to form the extended release matrix formulation, and then the extended release matrix formulation is subjected to an elevated temperature.

Within the meaning of this invention, the term "elevated temperature" refers to a temperature which is at least the softening temperature of poly(ε-caprolactone) and/or polyethylene oxide. According to some embodiments, the elevated temperature is at least about 60 °C, or at least about 65 °C, or at least about 70 °C, or at least about 80 °C, or ranges from about 60 °C to about 105 °C, or from about 65 °C to about 105 °C, or from about 70 °C to about 105 °C, or from about 80 °C to about 105 °C, or from about 60 °C to about 100 °C, or from about 65 °C to about 100 °C, or from about 70 °C to about 100 °C, or from about 80 °C to about 100 °C.

Within the meaning of this invention, the term "melt formed" refers to a process wherein the mixture is shaped while simultaneously being subjected to elevated temperature. This includes that the mixture is subjected to elevated temperature before shaping and is shaped while still hot enough. It includes without being limited to shaped by melt extrusion, shaped by casting, shaped by injection molding and shaped by direct compression with simultaneous application of elevated temperature.

Within the meaning of this invention, the term "melt extrusion" refers to a process by which material is mixed, at least partially melted and then forced through a die under controlled conditions.

The term "casting" is defined for purposes of the present invention as referring to a process by which molten material is poured into a mold of a desired shape or onto a surface.

The term "injection molding" is defined for purposed of the present invention as referring to a process by which molten material is injected under pressure into a mold.

The term "direct compression" is defined for purposes of the present invention as referring to a tableting process wherein the tablet or any other compressed dosage form is made by a process comprising the steps of dry blending the components comprising the dosage form and compressing the dry blend to physically form the dosage form, e.g. by using a diffusion blend and/or convection mixing process (e.g. Guidance for Industry, SUPAC-IR/MR: Immediate Release and Modified Release Solid Oral Dosage Forms, Manufacturing Equipment Addendum).

The term "ppm" as used herein means "parts per million." Regarding 14-hydroxycodeinone, "ppm" means parts per million of 14-hydroxycodeinone in a particular sample product. The 14-hydroxycodeinone level can be determined by any method known in the art, preferably by HPLC analysis using UV detection.

Within the meaning of this invention, dosage forms are regarded as "tamper resistant" when the respective dosage form resists illicit use, e.g. when the dosage form resists crushing and/or resists alcohol extraction as defined herein.

Within the meaning of this invention, dosage forms are regarded as "resistant to alcohol extraction" when the respective dosage form at least fulfills the condition that an in-vitro dissolution rate, when measured in a USP Apparatus 1 (basket) at 100 rpm in 900 ml simulated gastric fluid comprising 40% ethanol at 37° C, is provided which is characterized by the percent amount of active released at 30 minutes of dissolution that deviates no more than 20 % points from the corresponding in-vitro dissolution rate measured in a USP Apparatus 1 (basket) at 100 rpm in 900 ml simulated gastric fluid at 37° C without ethanol.

Within the meaning of this invention the term "Simulated Gastric Fluid" (SGF) relates to Simulated Gastric Fluid without enzymes and without sodium lauryl sulfate. The term "Simulated Gastric Fluid comprising 40% Ethanol" relates to SGF with 40% Ethanol and without enzymes and without sodium lauryl sulfate.

Within the meaning of this invention, dosage forms are regarded as "resistant to crushing" when the respective dosage form at least fulfills the condition that at least about 85 % of the initial amount of the dosage form is retained by a mesh #30 after crushing for 10 seconds in a coffee mill, e.g. a Krups™ Coffee Mill Type 203.

### BRIEF DESCRIPTION OF THE DRAWINGS

Fig 1 to Fig 6 depict the dissolution profiles of Examples 1 to 6 as described below.
Fig 7a and Fig 8a depict the dissolution profiles of Examples 7 and 8, wherein the samples are collected after the first extruder passage (Pass 1).
Fig 7b and Fig 8b depict the dissolution profiles of the Examples 7 and 8, wherein the samples are collected after the second extruder passage (Pass 2).
Fig 7c and Fig 8c depict dissolution profiles of Examples 7 and 8, Pass 1 and Pass 2 in comparison.
Fig 9a depicts the dissolution profiles of Example 9 melt-extruded multi-particulates (MEMs) of about 1 mm diameter.
Fig 9b depicts the dissolution profiles of Example 9 melt-extruded multi-particulates (MEMs) (Pass 2) with various pellet sizes.
Fig 10 to Fig 14 depict the dissolution profiles of Examples 10 to 14.
Fig 15a depicts the dissolution profiles of Example 15 melt-extruded multi-particulates (MEMs) with pellet sizes of about 1.3 mm diameter at different times of sampling during melt extrusion.
Fig 15b depicts the dissolution profiles of Example 15 melt-extruded multi-particulates (MEMs) with various pellet sizes.
Fig 16 to Fig 18 depict the dissolution profiles of Examples 16 to 18.
Fig 19 to Fig 25 depict the dissolution profiles Examples 19 to 41.

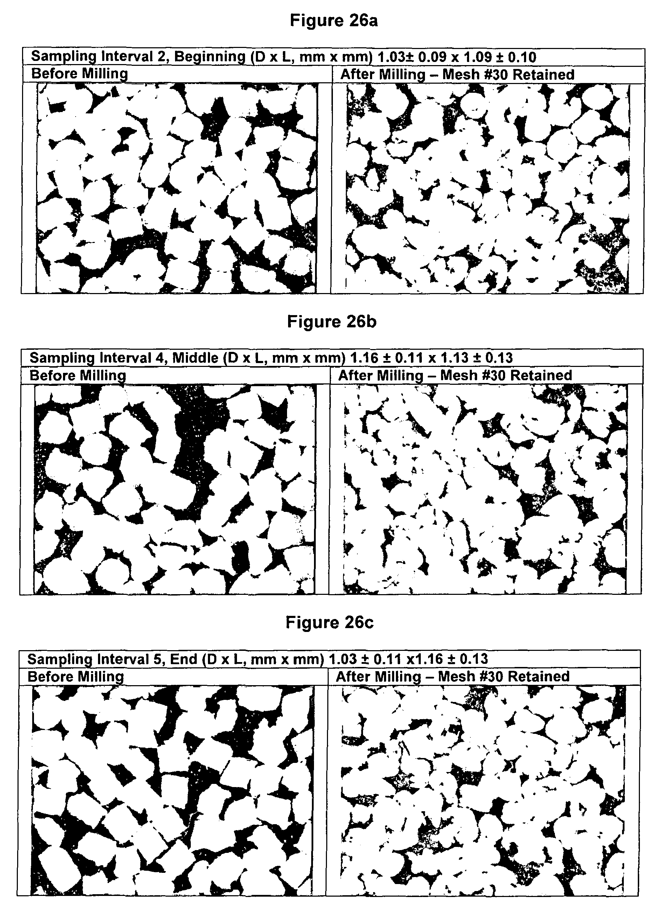

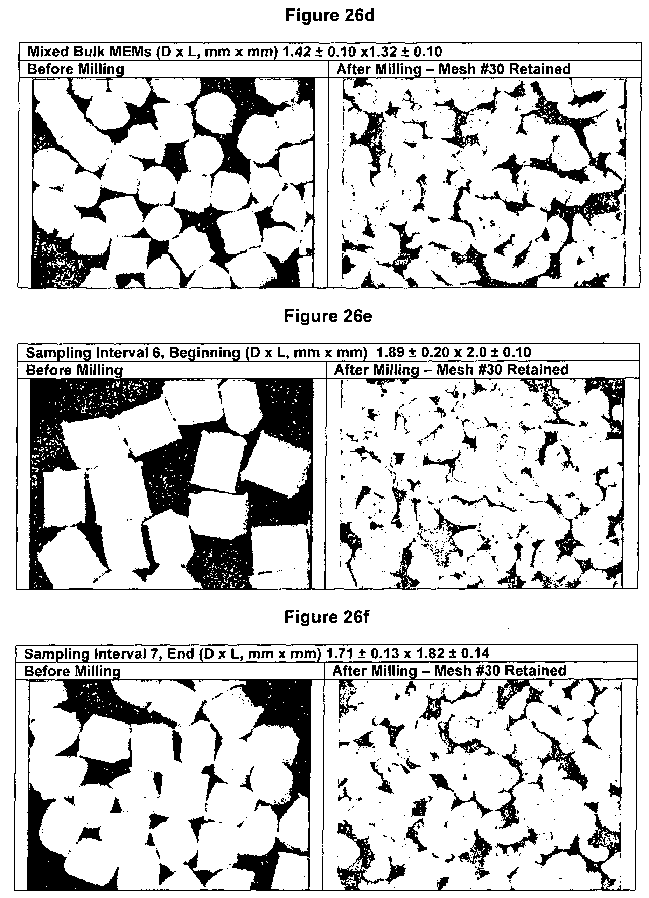

Fig 26a to Fig 26f show representative images of Example 16 melt-extruded multi-particulates (MEMs) before and after milling.

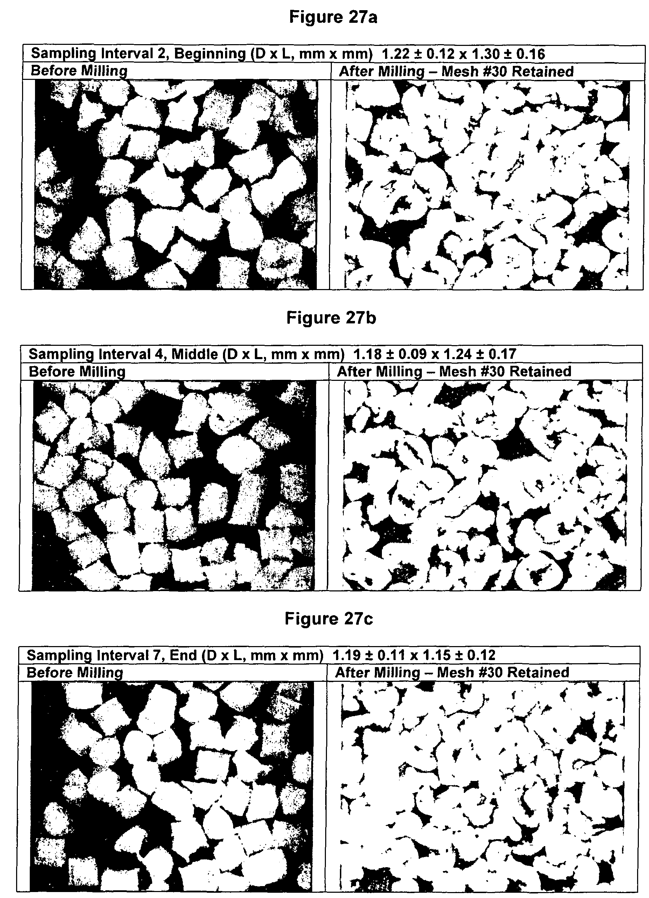

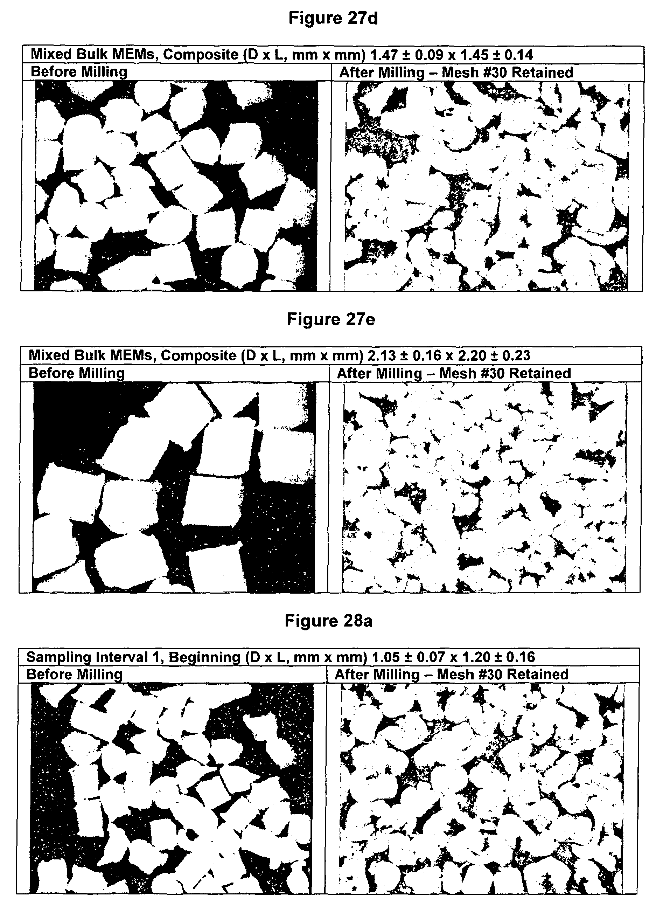

Fig 27a to Fig 27e show representative images of Example 17 melt-extruded multi-particulates (MEMs) before and after milling.

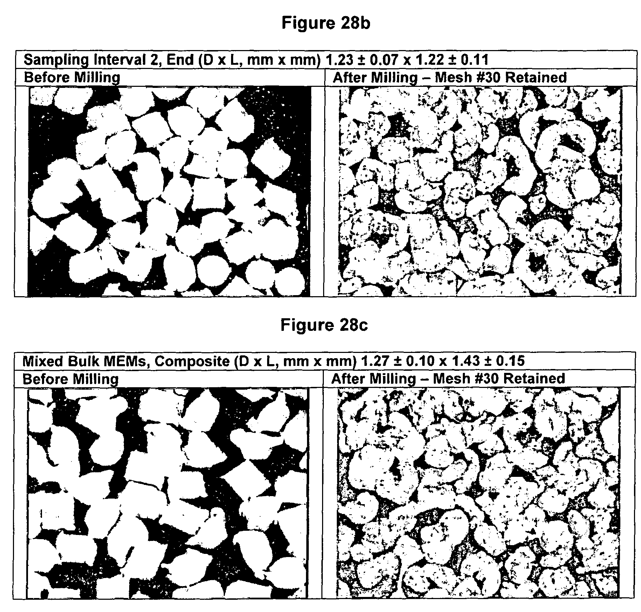

Fig 28a to Fig 28c show representative images of Example 18 melt-extruded multi-particulates (MEMs) before and after milling.

### DETAILED DESCRIPTION

### FORMULATION

According to certain embodiments of the invention the extended release matrix formulation comprises at least:
(1) at least one poly(ε-caprolactone)
(2) at least one polyethylene oxide, and
(3) at least one active agent.

According to certain embodiments of the invention, the extended release matrix formulation comprises at least one poly(ε-caprolactone) with an approximate number average molecular weight of from about 10,000 to about 200,000, or from about 30,000 to about 200,000, or from about 40,000 to about 200,000, or from about 43,000 to about 200,000, or more than 43,000, or from about 45,000 to about 200,000, or from about 60,000 to about 200,000, or from about 70,000 to about 200,000, or more than 75,000 to about 200,000, or from about 80,000 to about 200,000, or from about 85,000 to about 200,000, or from about 90,000 to about 200,000, or from about 100,000 to about 200,000, or from about 105,000 to about 200,000, or from about 110,000 to about 200,000, or from about 120,000 to about 200,000, or from about 130,000 to about 200,000, or from about 140,000 to about 200,000.

According to certain embodiments of the invention, in the extended release matrix formulation the overall content of poly(ε-caprolactone) is at least about 40 weight-%, or from about 40 weight-% to about 85 weight-%, or from about 40 weight-% to about 80 weight-%, or from about 40 weight-% to about 75 weight-%, or of from about 45 weight-% to about 75 weight-%, or from about 50 weight-% to about 75 weight-%, or from about 55 weight-% to about 75 weight-%, or from about 60 weight-% to about 75 weight-%, or from about 65 weight-% to about 75 weight-% of the extended release matrix formulation. According to certain embodiments of the invention, the overall content of poly(ε-caprolactone) is less than 50 weight-% of the extended release matrix formulation.

According to certain embodiments of the invention, in the extended release matrix formulation the overall content of the poly(ε-caprolactone) described in paragraph [0064] is at least about 40 weight-%, or from about 40 weight-% to about 85 weight-%, or from about 40 weight-% to about 80 weight-%, or from about 40 weight-% to about 75 weight-%, or of from about 45 weight-% to about 75 weight-%, or from about 50 weight-% to about 75 weight-%, or from about 55 weight-% to about 75 weight-%, or from about 60 weight-% to about 75 weight-%, or from about 65 weight-% to about 75 weight-% of the extended release matrix formulation. According to certain embodiments of the invention, the overall content of the poly(ε-caprolactone) described in paragraph [0064] is less than 50 weight-% of the extended release matrix formulation.

According to certain embodiments of the invention, the extended release matrix formulation comprises at least one polyethylene oxide with an approximate weight average molecular weight of from about 10,000 to less than 1,000,000, or from about 40,000 to less than 1,000,000, or from about 50,000 to less than 1,000,000, or from about 80,000 to less than 1,000,000, or from about 500,000 to about 950,000, or from about 600,000 to about 950,000, or from about 700,000 to about 950,000, or from about 50,000 to about 950,000, or from about 50,000 to about 400,000, or from about 50,000 to about 300,000, or from about 50,000 to about 200,000.

According to certain embodiments, wherein the poly(ε-caprolactone) has an approximate number average molecular weight of more than 43,000 or more than 80,000, the formulation comprises polyethylene oxide with an approximate weight average molecular weight of from about 1,000,000 to 10,000,000.

According to certain embodiments of the invention, in the extended release matrix formulation the overall content of polyethylene oxide is at least about 10 weight-%, or at least about 13 weight-%, or at least about 15 weight-%, or at least about 20 weight-%, or at least about 25 weight-%, or at least about 30 weight-%, or from about 10 weight-% to about 40 weight-%, or from about 13 weight-% to about 40 weight-%, or from about 15 weight-% to about 40 weight-%, or from about 20 weight-% to about 40 weight-%, or from about 25 weight-% to about 40 weight-%, or from about 30 weight-% to about 40 weight-%, or from about 15 weight-% to about 35 weight-% of the extended release matrix formulation.

According to certain embodiments of the invention, the active agent is present in an amount of at least about 10 weight-% of the extended release matrix formulation, or at least about 12.5 weight-%, or at least about 15 weight-%, or from about 10 weight-% to about 30 weight-%, or from about 10 weight-% to about 25 weight-%, or from about 12.5 weight-% to about 25 weight-% of the extended release matrix formulation.

### FURTHER RETARDANTS

According to certain embodiments of the invention, further retardants are present in the extended release matrix formulation, preferably in an amount of from about 0.1 weight-% to about 10 weight-%.

Further retardants useful in the present invention in addition to poly(ε-caprolactone) and polyethylene oxide include, but are not limited to, long chain (C₈-C₅₀) substituted or unsubstituted hydrocarbons, such as fatty acids, fatty alcohols, glyceryl esters of fatty acids, polyethylene glycol esters of fatty acids, mineral and vegetable oils and waxes. According to certain preferred embodiments, glyceryl behenate is used.

According to the invention, further retardants may be present in the extended release matrix formulation in an amount of from about 2 weight-% to about 7 weight-%, or from about 3 weight-% to about 6 weight-%, or from about 4 weight-% to about 6 weight-% of the extended release matrix formulation.

### DOSAGE FORM

According to the invention, the extended release matrix formulation of the solid extended release pharmaceutical dosage form is in the form of a single tablet, or is in the form of multi-particulates or in the form of a suppository. The diameter of the multi-particulates is preferably in the range of from about 0.1 mm to about 5 mm, or from about 0.1mm to about 2 mm, or from about 0.5 mm to about 2 mm. Multi-particulates may also be in the range of from about 2 mm to about 5 mm, and include dosage forms known in the art as minitabs. According to certain embodiments of the invention, the multi-particulates are placed in a capsule or formed into a tablet which disintegrates into the multi-particulates when placed in contact with gastric fluids. In accordance with the invention, the overall release rate can be adjusted by varying the final size of the extended release matrix formulation. e.g. the multi-particulates or the tablet subject to dissolution.

According to the invention the solid extended release pharmaceutical dosage form is preferably an oral dosage form. According to certain other embodiments of the invention the solid extended release pharmaceutical dosage form is a suppository.

### ACTIVE AGENT

The active agent used in accordance with the invention may be any active agent as known to the skilled person. In particular, the active agent is a substance that is subject to abuse, such as opioids, tranquillisers and other narcotics e.g. selected from the group consisting of N-{1-[2-(4-ethyl-5-oxo-2-tetrazolin-1-yl)ethyl]-4-methoxymethyl-4-piperidyl}propionanilide (alfentanil), 5,5-diallylbarbituric acid (allobarbital), allylprodine, alphaprodine, 8-chloro-1-methyl-6-phenyl-4H-[1,2,4]triazolo[4,3-a][1,4]-benzodiazepine (alprazolam), 2-diethylaminopropiophenone (amfepramone), (+-)-[alpha]-methyl-phenethylamine (amphetamine), 2-[alpha]-methylphenethylamino)-2-phenylacetonitrile (amphetaminil), 5-ethyl-5-isopentylbarbituric acid (amobarbital), anileridine, apocodeine, 5,5-diethylbarbituric acid (barbital), benzylmorphine, bezitramide, 7-bromo-5-(2-pyridyl)-1H-1,4-benzodiazepine-2(3H)-one (bromazepam), 2-bromo-4-(2-chlorophenyl)-9-methyl-6H-thieno[3,2-f][1,2,4]triazolo-[4,3-a][1,4]diazepine (brotizolam), 17-cyclopropylmethyl-4,5[alpha]-epoxy-7[alpha][(S)-1-hydroxy-1,2,2-trimethyl-propyl]-6-methoxy-6,14-endo-ethanomorphinane-3-ol (buprenorphine), 5-butyl-5-ethylbarbituric acid (butobarbital), butorphanol, (7-chloro-1,3-dihydro-1-methyl-2-oxo-5-phenyl-2H-1,4-benzodiazepine-3-yl)-dimethylcarbamate (camazepam), (1S,2S)-2-amino-1-phenyl-1-propanol (cathine/D-norpseudoephedrine), 7-chloro-N-methyl-5-phenyl-3H-1,4-benzodiazepine-2-ylamine-4-oxide (chlorodiazepoxide), 7-chloro-1-methyl-5-phenyl-1H-1,5-benzodiazepine-2,4(3H,5H)-dione (clobazam), 5-(2-chlorophenyl)-7-nitro-1H-1,4-benzodiazepine-2(3H)-one (clonazepam), clonitazene, 7-chloro-2,3-dihydro-2-oxo-5-phenyl-1H-1,4-benzodiazepine-3-carboxylic acid (clorazepate), 5-(2-chlorophenyl)-7-ethyl-1-methyl-1H-thieno[2,3-e][1,4]diazepine-2(3H)-one (clotiazepam), 10-chloro-11b-(2-chlorophenyl)-2,3,7,11b-tetrahydrooxazolo[3,2-d][1,4]benzodiazepine-6(5H)-one (cloxazolam), (-)-methyl-[3[beta]-benzoyloxy-2[beta](1[alpha](H,5[alpha]H)-tropancarboxylate](cocaine), 4,5[alpha]-epoxy-3-methoxy-17-methyl-7-morphinene-6[alpha]-ol(codeine), 5-(1-cyclohexenyl)-5-ethylbarbituric acid (cyclobarbital), cyclorphan, cyprenorphine, 7-chloro-5-(2-chlorophenyl)-1H-1,4-benzodiazepine-2(3H)-one (delorazepam), desomorphine, dextromoramide, (+)-(1-benzyl-3-dimethylamino-2-methyl-1-phenylpropyl)propionate (dextropropoxyphen), dezocine, diampromide, diamorphone, 7-chloro-1-methyl-5-phenyl-1H-1,4-benzodiazepine-2(3H)-one (diazepam), 4,5[alpha]-epoxy-3-methoxy-17-methyl-6[alpha]-morphinanol (dihydrocodeine), 4,5[alpha]-epoxy-17-methyl-3,6[alpha]-morphinandiol (dihydromorphine), dimenoxadol, dimepheptanol, dimethylthiambutene, dioxaphetyl butyrate, dipipanone, (6aR,10aR)-6,6,9-trimethyl-3-pentyl-6a,7,8,10a-tetrahydro-6H-benzo[c]chromene-1-ol (dronabinol), eptazocine, 8-chloro-6-phenyl-4H-[1,2,4]triazolo[4,3-a][1,4]benzodiazepine (estazolam), ethoheptazine, ethylmethylthiambutene, ethyl [7-chloro-5-(2-fluorophenyl)-2,3-dihydro-2-oxo-1H-1,4-benzodiazepine-3-carboxylate](ethyl loflazepate), 4,5[alpha]-epoxy-3-ethoxy-17-methyl-7-morphinene-6[alpha]-ol (ethylmorphine), etonitazene, 4,5[alpha]-epoxy-7[alpha]-(1-hydroxy-1-methylbutyl)-6-methoxy-17-methyl-6,14-endo-etheno-morphinan-3-ol (etorphine), N-ethyl-3-phenyl-8,9,10-trinorbornan-2-ylamine(fencamfamine), 7-[2-(1-methyl-phenethylamino)ethyl]-theophylline) (fenethylline), 3-([alpha]-methylphenethylamino)propionitrile (fenproporex), N-(1-phenethyl-4-piperidyl)propionanilide (fentanyl), 7-chloro-5-(2-fluorophenyl)-1-methyl-1H-1,4-benzodiazepine-2(3H)-one (fludiazepam), 5-(2-fluorophenyl)-1-methyl-7-nitro-1H-1,4-benzodiazepine-2(3H)-one (flunitrazepam), 7-chloro-1-(2-diethylaminoethyl)-5-(2-fluorophenyl)-1H-1,4-benzodiazepine-2(3H)-one (flurazepam), 7-chloro-5-phenyl-1-(2,2,2-trifluoroethyl)-1H-1,4-benzodiazepine-2(3H)-one (halazepam), 10-bromo-11b-(2-fluorophenyl)-2,3,7,11b tetrahydro[1,3]oxazolyl[3,2-d][1,4]benzodiazepine-6(5H)-one (haloxazolam), heroin, 4,5[alpha]-epoxy-3-methoxy-17-methyl-6-morphinanone (hydrocodone), 4,5[alpha]-epoxy-3-hydroxy-17-methyl-6-morphinanone (hydromorphone), hydroxypethidine, isomethadone, hydroxymethyl morphinane, 11-chloro-8,12b-dihydro-2,8-dimethyl-12b-phenyl-4H-[1,3]oxazino[3,2-d][1,4]benzodiazepine-4,7(6H)-dione (ketazolam), 1-[4-(3-hydroxyphenyl)-1-methyl-4-piperidyl]-1-propanone (ketobemidone), (3S,6S)-6-dimethylamino-4,4-diphenylheptan-3-yl acetate(levacetylmethadol (LAAM)), (-)-6-dimethyl-amino-4,4-diphenol-3-heptanone (levomethadone), (-)-17-methyl-3-morphinanol (levorphanol), levophenacylmorphane, lofentanil, 6-(2-chlorophenyl)-2-(4-methyl-1-piperazinylmethylene)-8-nitro-2H-imidazo[1,2-a] [1,4]-benzodiazepine-1(4H)-one (loprazolam), 7-chloro-5-(2-chlorophenyl)-3-hydroxy-1H-1,4-benzodiazepine-2(3H)-one (lorazepam), 7-chloro-5-(2-chlorophenyl)-3-hydroxy-1-methyl-1H-1,4-benzodiazepine-2(3H)-one (lormetazepam), 5-(4-chlorophenyl)-2,5-dihydro-3H-imidazo[2,1-a]isoindol-5-ol (mazindol), 7-chloro-2,3-dihydro-l-methyl-5-phenyl-lH-1,4-benzodiazepine (medazepam), N-(3-chloropropyl)-[alpha]-methylphenethylamine (mefenorex), meperidine, 2-methyl-2-propyltrimethylene dicarbamate (meprobamate), meptazinol, metazocine, methylmorphine, N,[alpha]-dimethylphenethylamine (methamphetamine), (O)-6-dimethylamino-4,4-diphenyl-3-heptanone (methadone), 2-methyl-3-o-tolyl-4(3H)-quinazolinone (methaqualone), methyl [2-phenyl-2-(2-piperidyl)acetate] (methylphenidate), 5-ethyl-1-methyl-5-phenylbarbituric acid (methylphenobarbital), 3,3-diethyl-5-methyl-2,4-piperidinedione (methyprylon), metopon, 8-chloro-6-(2-fluorophenyl)-1-methyl-4H-imidazo[1,5-a][1,4]benzodiazepine (midazolam), 2-(benzhydrylsulfinyl)-acetamide (modafinil), 4,5[alpha]-epoxy-17-methyl-7-morphinen-3,6[alpha]-diol (morphine), myrophine, (+-)-trans-3-(1,1-dimethylheptyl)-7,8,10,10[alpha]-tetrahydro-1-hydroxy-6,6-dimethyl-6H-dibenzo[b,d]pyrane-9 (6[alpha]H)-one (nabilone), nalbuphine, nalorphine, narceine, nicomorphine, 1-methyl-7-nitro-5-phenyl-1H-1,4-benzodiazepine-2(3H)-one (nimetazepam), 7-nitro-5-phenyl-1H-1,4-benzodiazepine-2(3H)-one (nitrazepam), 7-chloro-5-phenyl-1H-1,4-benzodiazepine-2(3H)-one (nordazepam), norlevorphanol, 6-dimethylamino-4,4-diphenyl-3-hexanone (normethadone), normorphine, norpipanone, the exudation of plants belonging to the species Papaver somniferum (opium), 7-chloro-3-hydroxy-5-phenyl-1H-1,4-benzodiazepine-2(3H)-one (oxazepam), (cis-trans)-10-chloro-2,3,7,11b-tetrahydro-2-methyl-11b-phenyloxazolo[3,2-d][1,4]benzodiazepine-6-(5H)-one (oxazolam), 4,5[alpha]-epoxy-14-hydroxy-3-methoxy-17-methyl-6-morphinanone (oxycodone), oxymorphone, plants and parts of plants belonging to the species Papaver somniferum (including the subspecies setigerum), papaveretum, 2-imino-5-phenyl-4-oxazolidinone (pernoline), 1,2,3,4,5,6-hexahydro-6,11-dimethyl-3-(3-methyl-2-butenyl)-2,6-methano-3-benzazocin-8-ol (pentazocine), 5-ethyl-5-(1-methylbutyl)-barbituric acid (pentobarbital), ethyl-(1-methyl-4-phenyl-4-piperidine carboxylate) (pethidine), phenadoxone, phenomorphan, phenazocine, phenoperidine, piminodine, pholcodine, 3-methyl-2-phenylmorpholine (phenmetrazine), 5-ethyl-5-phenylbarbituric acid (phenobarbital), [alpha],[alpha]-dimethylphenethylamine(phentermine), 7-chloro-5-phenyl-1-(2-propynyl)-1H-1,4-benzodiazepine-2(3H)-one (pinazepam), [alpha]-(2-piperidyl)benzhydryl alcohol (pipradrol), 1'-(3-cyano-3,3-diphenylpropyl)[1,4'-bipiperidine]-4'-carboxamide(piritramide), 7-chloro-1-(cyclopropylmethyl)-5-phenyl-1H-1,4-benzodiazepine-2(3H)-one (prazepam), profadol, proheptazine, promedol, properidine, propoxyphene, N-(1-methyl-2-piperidinoethyl)-N-(2-pyridyl)propionamide, methyl {3-[4-methoxycarbonyl-4-(N-phenylpropanamido)piperidino]propanoate} (remifentanil), 5-sec-butyl-5-ethylbarbituric acid (secbutabarbital), 5-allyl-5-(1-methylbutyl)-barbituric acid (secobarbital), N-{4-methoxymethyl-1-[2-(2-thienyl)ethyl]-4-piperidyl}-propionanilide (sufentanil), 7-chloro-2-hydroxy-methyl-5-phenyl-1H-1,4-benzodiazepin-2(3H)-one (temazepam), 7-chloro-5-(1-cyclohexenyl)-1-methyl-1H-1,4-benzodiazepine-2(3H)-one (tetrazepam), ethyl(2-dimethylamino-1-phenyl-3-cyclohexene-1-carboxylate) (tilidine (cis and trans)), tramadol, 8-chloro-6-(2-chlorophenyl)-1-methyl-4H-[1,2,4]triazolo[4,3-a][1,4]benzodiazepine (triazolam), 5-(1-methylbutyl)-5-vinylbarbituric acid (vinylbital), (1R*,2R*)-3-(3-dimethylamino-1-ethyl-2-methyl-propyl)-phenol, (1R,2R,4S)-2-(dimethylamino)methyl-4-(p-fluoro-benzyloxy)-1-(m-methoxyphenyl)cyclohexanol, (1R,2R)-3-(2-dimethylaminomethyl-cyclohexyl)phenol, (1S,2S)-3-(3-dimethylamino-1-ethyl-2-methyl-propyl)phenol, (2R,3R)-1-dimethylamino-3(3-methoxyphenyl)-2-methyl-pentan-3-ol, (1RS,3RS,6RS)-6-dimethylaminomethyl-1-(3-methoxyphenyl)-cyclohexane-1,3-diol, 3-(2-dimethylaminomethyl-l-hydroxy-cyclohexyl)phenyl 2-(4-isobutoxy-phenyl)-propionate, 3-(2-dimethylaminomethyl-1-hydroxy-cyclohexyl)phenyl 2-(6-methoxy-naphthalen-2-yl)-propionate, 3-(2-dimethylamino-methyl-cyclohex-1-enyl)-phenyl 2-(4-isobutyl-phenyl)-propionate, 3-(2-dimethylaminomethyl-cyclohex-1-enyl)-phenyl 2-(6-methoxy-naphthalen-2-yl)-propionate, (RR-SS)-2-acetoxy-4-trifluoromethyl-benzoic acid 3-(2-dimethylaminomethyl-1-hydroxy-cyclohexyl)-phenyl ester, (RR-SS)-2-hydroxy-4-trifluoromethyl-benzoic acid 3-(2-dimethylaminomethyl-1-hydroxy-cyclohexyl)-phenyl ester, (RR-SS)-4-chloro-2-hydroxy-benzoic acid 3-(2-dimethylaminomethyl-1-hydroxy-cyclohexyl)-phenyl ester, (RR-SS)-2-hydroxy-4-methyl-benzoic acid 3-(2-dimethylamino-methyl-1-hydroxy-cyclohexyl)-phenyl ester, (RR-SS)-2-hydroxy-4-methoxy-benzoic acid 3-(2-dimethylaminomethyl-1-hydroxy-cyclohexyl)-phenyl ester, (RR-SS)-2-hydroxy-5-nitro-benzoic acid 3-(2-dimethylaminomethyl-1-hydroxy-cyclohexyl)-phenyl ester, (RR-SS)-21,4'-difluoro-3-hydroxy-biphenyl-4-carboxylic acid 3-(2-dimethylaminomethyl-1-hydroxy-cyclohexyl)-phenyl ester and for corresponding stereoisomeric compounds, the corresponding derivatives thereof in each case, in particular esters or ethers, and the physiologically acceptable compounds thereof in each case, in particular the salts and solvates thereof.

According to specific preferred embodiments, the active agent is an opioid, in particular an opioid analgesic.

Opioid analgesics useful in the present invention include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, and mixtures of any of the foregoing. Preferred opioid analgesics include codeine, morphine, oxycodone, hydrocodone, hydromorphone, oxymorphone, pharmaceutically acceptable salts, hydrates and solvates thereof, and mixtures of any of the foregoing.

In certain embodiments, the opioid analgesic is oxycodone, hydromorphone or oxymorphone, or a pharmaceutically acceptable salt thereof, such as, e.g., the hydrochloride salt. The dosage form may comprise from about 5 mg to about 500 mg oxycodone hydrochloride, or from about 1 mg to about 100 mg hydromorphone hydrochloride, or from about 5 mg to about 500 mg oxymorphone hydrochloride. If the free base, or other salts, solvates or hydrates are used, equimolar amounts may be used.

The dosage form may comprise, e.g., 5 mg, 7.5 mg, 10 mg, 15 mg, 20 mg, 30 mg, 40 mg, 45 mg, 50 mg, 60 mg, 80 mg, 90 mg, 100 mg, 120 mg or 160 mg oxycodone hydrochloride, or an equimolar amount ofany other pharmaceutically acceptable salt, derivative or form including but not limited to hydrates and solvates or of the free base.

The dosage form may comprise, e.g., 5 mg, 7.5 mg, 10 mg, 15 mg, 20 mg, 30, mg, 40 mg, 45 mg, 50 mg, 60 mg, 80 mg, 90 mg, 100 mg, 120 mg or 160 mg oxymorphone hydrochloride, or an equimolar amount of any other pharmaceutically acceptable salt, derivative or form including but not limited to hydrates and solvates or of the free base.

The dosage form may comprise, e.g., 2 mg, 4 mg, 5 mg, 8 mg, 12 mg, 15 mg, 16 mg, 24 mg, 25 mg, 32 mg, 48 mg, 50 mg, 64 mg or 75 mg hydromorphone hydrochloride or equimolar amounts of any other pharmaceutically acceptable salt, derivative or form including but not limited to hydrates and solvates or of the free base.

The present invention disclosed herein is specifically meant to encompass the use of an opioid analgesic in any pharmaceutically acceptable salt thereof. Pharmaceutically acceptable salts include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like, and metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like.

The present invention disclosed herein is specifically meant to encompass the use of oxycodone hydrochloride, preferably present in an amount of from about 5 mg to about 500 mg oxycodone hydrochloride, more preferably present in an amount of 5 mg, 7.5 mg, 10 mg, 15 mg, 20 mg, 30, mg, 40 mg, 45 mg, 50 mg, 60 mg, 80 mg, 90 mg, 100 mg, 120 mg or 160 mg oxycodone hydrochloride, or at an amount of more than 15 weight-% of the extended release matrix formulation, and preferably with a 14-hydroxycodeinone level of less than about 25 ppm, preferably less than about 15 ppm, less than about 10 ppm, less than about 5 ppm, or less than about 1 ppm.

The following patent documents, PCT Published Patent Application WO 2005/097801 A1, US Patent No. 7,129,248 B2 and US Published Patent Application 2006/0173029 A1 describe processes for preparing oxycodone hydrochloride having a 14-hydroxycodeinone level of less than about 25 ppm, preferably less than about 15 ppm, less than about 10 ppm, less than about 5 ppm, more preferably less than about 2 ppm, less than about 1 ppm, less than about 0.5 ppm or about 0.25 ppm.

The invention disclosed herein is specifically meant to encompass the use of oxymorphone hydrochloride, preferably present in an amount of from about 1 mg to about 500 mg oxymorphone hydrochloride, more preferably present in an amount of 5 mg, 7.5 mg, 10 mg, 15 mg, 20 mg, 30 mg, 40 mg, 45 mg, 50 mg 60 mg, 80 mg, 90 mg, 100 mg, 120 mg or 160 mg oxymorphone hydrochloride.

The present invention disclosed herein is specifically meant to encompass the use of hydromorphone hydrochloride, preferably present in an amount of from about mg to about 100 mg hydromorphone hydrochloride, more preferably present in an amount of 2 mg, 4 mg, 5 mg, 8 mg, 12 mg, 15 mg, 16 mg, 24 mg, 25 mg, 32 mg, 48 mg, 50 mg, 64 mg or 75 mg hydromorphone hydrochloride.

Opioid antagonists useful in the invention, either alone or in combination with an opioid agonist, include, but are not limited to, naloxone, naltrexone and nalmephene, the pharmaceutically acceptable salts, hydrates and solvates thereof, and mixtures of any of the foregoing.

According to certain embodiments, naltrexone hydrochloride may be present in an amount of from about 1 mg to about 100 mg naltrexone hydrochloride, more preferably present in an amount of 2.5 mg, 5 mg, 10 mg, 15 mg, 20 mg, 30 mg, 40 mg, 50 mg or 60 mg of naltrexone hydrochloride or at an amount of at least about 10 weight-% of the extended release matrix formulation.

According to certain embodiments, opioid antagonists are useful in combination with opioid agonists, e.g. a combination of oxycodone HCl and naloxone HCl in a weight ratio of about 2:1 is used. Examples of actual weights of oxycodone HCl : naloxone HCl in milligrams in each unit dose are 5: 2.5, 10:5, 20:10, 30:15, 40:20, 60:30, 80:40, 100:50, 120:60, and 160:80.

In certain other embodiments, further therapeutically active agents may be used in accordance with the invention, either in combination with opioids or instead of opioids. Examples of such therapeutically active agents include antihistamines (e.g., dimenhydrinate, diphenhydramine, chlorpheniramine and dexchlorpheniramine maleate), non -steroidal anti-inflammatory agents (e.g., naproxen, diclofenac, indomethacin, ibuprofen, sulindac, Cox-2 inhibitors) and acetaminophen, anti-emetics (e.g., metoclopramide, methylnaltrexone), antiepileptics (e.g., phenytoin, meprobmate and nitrazepam), vasodilators (e.g., nifedipine, papaverine, diltiazem and nicardipine), anti-tussive agents and expectorants (e.g. codeine phosphate), anti-asthmatics (e.g. theophylline), antacids, anti-spasmodics (e.g. atropine, scopolamine), antidiabetics (e.g., insulin), diuretics (e.g., ethacrynic acid, bendrofluthiazide), anti-hypotensives (e.g., propranolol, clonidine), antihypertensives (e.g., clonidine, methyldopa), bronchodilatiors (e.g., albuterol), steroids (e.g., hydrocortisone, triamcinolone, prednisone), antibiotics (e.g., tetracycline), antihemorrhoidals, hypnotics, psychotropics, antidiarrheals, mucolytics, sedatives, decongestants (e.g. pseudoephedrine), laxatives, vitamins, stimulants (including appetite suppressants such as phenylpropanolamine) and cannabinoids, including all pharmaceutically acceptable salts, hydrates, and solvates of the same.

In certain embodiments, the invention is directed to the use of Cox-2 inhibitors as active agents, in combination with opioid analgesics or instead of opioid analgesics; for example, the use of a Cox-2 inhibitor such as meloxicam (4-hydroxy-2-methyl-*N*-(5-methyl-2-thiazolyl)-2*H*-1,2-benzothiazine-3-carboxamide-1,1-dioxide), as disclosed in U.S. Patent Application Serial Nos. 10/056,347 and 11/825,938; nabumetone (4-(6-methoxy-2-naphthyl)-2-butanone), as disclosed in U.S. Patent Application Serial No. 10/056,348; celecoxib (4-[5-(4-methylphenyl)-3-(trifluoromethyl)-1*H*-pyrazol-1-yl]benzenesulfonamide), as disclosed in U.S. Patent Application Serial No. 11/698,394; nimesulide (N-(4-Nitro-2-phenoxyphenyl) methanesulfonamide), as disclosed in U.S. Patent Application Serial No. 10/057,630, and N-[3-(formylamino)-4-oxo-6-phenoxy-4H-1-benzopyran-7-yl] methanesulfonamide (T-614), as disclosed in U.S. Patent Application Serial No. 10/057,632.

The present invention is also directed to dosage forms utilizing active agents such as benzodiazepines, barbiturates or stimulants such as amphetamines. These may be formulated as single active agents, or combined with their respective antagonists.

### METHOD OF MANUFACTURE

According to the invention, the dosage form is thermo-treated. Thermo-treatment in accordance with the invention includes a step comprising the application of elevated temperature as defined above.

According to certain embodiments, the dosage form is shaped without the application of an elevated temperature and then cured at an elevated temperature. In certain such embodiments, the extended release matrix formulation may be shaped by direct compression. The dosage form may also be melt formed. Melt formed dosage forms include dosage forms wherein the extended release matrix formulation is shaped by a melt extrusion method, or by a casting method, or by an injection molding method, or by direct compression with simultaneous application of elevated temperature.

According to one aspect, the invention relates to a process of preparing a solid extended release pharmaceutical dosage form in accordance with the invention and as described above in detail comprising the steps of:
1. combining at least one poly(ε-caprolactone), at least one polyethylene oxide, at least one active agent, and optionally one or more other ingredients to form a blend;
2. feeding the blend from step 1 into a single-screw volumetric dispenser;
3. metering the blend from the dispenser into a twin screw extruder and processing the blend at elevated temperature into strands;
4. drawing the strands from step 3 from the extruder and cooling the strands;
5. pelletizing the cooled strands from step 4 by cutting into pellets; or providing slices by cutting the cooled strands from step 4 into tablet slices with a blade;
   and optionally
6. metering the pellets from step 5 into a twin screw extruder and processing them at elevated temperature into strands;
7. Drawing and cooling the strands;
8. Pelletizing the cooled strands by cutting into pellets.

According to a certain preferred aspect of the invention, poly(ε-caprolactone) in the form of flakes or milled material ≤ 840 µm is used in step 1.

According to a further aspect, the invention relates to a process of preparing a solid extended release pharmaceutical dosage form in accordance with the invention and as described above in detail comprising the steps of:
1. blending at least one polyethylene oxide, at least one active agent and optionally other ingredients, except the at least one poly(ε-caprolactone), to form a first composition;
2. feeding the first composition of step 1 to a first hopper of a first volumetric dispenser fitted with a first single-screw assembly;
3. feeding poly(ε-caprolactone) as a second composition to a second hopper of a second volumetric dispenser fitted with a second screw assembly larger than the first screw assembly;
4. calibrating the feed rate of the two dispensers according to the relative proportion of the first and second compositions to obtain a total feed rate of e.g., 25 g/min;
5. metering the first and second compositions into a twin screw extruder and processing the resulting extrudate at elevated temperature into strands;
6. drawing and cooling the strands from step 5; and
7. pelletizing the cooled strands from step 6 by cutting them into pellets.

### RELEASE CHARACTERISTICS

According to the invention, the dosage form provides release rates of the active agent in-vitro when measured by the USP Basket Method at 100 rpm at 900 ml simulated gastric fluid at 37 °C, of from about 12.5 % to about 55 % (by wt) active agent released after 60 minutes, from about 25 % to about 65 % (by wt) active agent released after 120 minutes, from about 45 % to about 85 % (by wt) active agent released after 240 minutes, and from about 55 % to about 95 % (by wt) active agent released after 360 minutes.

According to the invention, the dosage form provides an in-vitro dissolution rate of the active agent, when measured by the USP Basket Method at 100 rpm at 900 ml simulated gastric fluid at 37 °C, of from about 10 % to about 30 % (by wt) active agent released after 30 minutes, from about 20 % to about 50 % (by wt) active agent released after 60 minutes, from about 30 % to about 65 % (by wt) active agent released after 120 minutes, from about 45 % to about 85 % (by wt) active agent released after 240 minutes, and from about 60 % to about 95 % (by wt) active agent released after 360 minutes.

### ALCOHOL RESISTANCE CHARACTERISTICS

According to the invention, the dosage form provides an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 30 minutes of dissolution that deviates no more than 20 % points from the corresponding in-vitro dissolution rate measured in a USP Apparatus 1 (basket) at 100 rpm in 900 ml simulated gastric fluid (SGF) at 37° C without ethanol, preferably no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

According to the invention, the dosage form provides an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 60 minutes of dissolution that deviates no more than 20 % points from the corresponding in-vitro dissolution rate measured in a USP Apparatus 1 (basket) at 100 rpm in 900 ml simulated gastric fluid (SGF) at 37° C without ethanol, preferably no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

According to the invention, the dosage form provides an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 120 minutes of dissolution that deviates no more than 20 % points from the corresponding in-vitro dissolution rate measured in a USP Apparatus 1 (basket) at 100 rpm in 900 ml simulated gastric fluid (SGF) at 37° C without ethanol, preferably no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

According to the invention, the dosage form provides an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 240 minutes of dissolution that deviates no more than 20 % points from the corresponding in-vitro dissolution rate measured in a USP Apparatus 1 (basket) at 100 rpm in 900 ml simulated gastric fluid (SGF) at 37° C without ethanol, preferably no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

According to the invention, the dosage form provides an in-vitro dissolution rate, when measured in a USP Apparatus 1 (basket) at 100 rpm in 900 ml simulated gastric fluid (SGF) comprising 40% ethanol at 37° C, characterized by the percent amount of active agent released at 360 minutes of dissolution that deviates no more than 20 % points from the corresponding in-vitro dissolution rate measured in a USP Apparatus 1 (basket) at 100 rpm in 900 ml simulated gastric fluid (SGF) at 37° C without ethanol, preferably no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

### CRUSHING CHARACTERISTICS

The resistance to crushing is measured in accordance with the following procedure:
1. A tablet or melt-extruded multi-particulates (MEMs) equivalent to one dose was added to the stainless steel milling chamber of a Krups™ mill (e.g. Krups™ Coffee Mill Type 203).
2. The material was milled in 10 second intervals up to a total of 60 seconds while monitoring the time lapsed using a stop watch.
3. The material that was retained, and which passed through mesh # 30 (600 µm) after each round of milling, was weighed and the weight recorded. The material was returned to the mill for the next round of milling. The mesh-retained and mesh-passed materials were also evaluated visually using a stereomicroscope.

According to the invention, the dosage form is further characterized by providing, after crushing for 10 seconds in a coffee mill, an amount of material retained by a mesh #30 of at least about 85 %, preferably at least about 90 %, or at least about 95 % of the initial amount of the dosage form.

According to the invention, the dosage form is further characterized by providing, after crushing for 20 seconds in a coffee mill, an amount of material retained by a mesh #30 of at least about 75 %, preferably at least about 80 %, or at least about 85 %, or at least about 90 % of the initial amount of the dosage form.

According to the invention, the dosage form is further characterized by providing, after crushing for 30 seconds in a coffee mill, an amount of material retained by a mesh #30 of at least about 65 %, preferably at least about 70 %, or at least about 80 %, or at least about 85 % of the initial amount of the dosage form.

According to the invention, the dosage form is further characterized by providing, after crushing for 40 seconds in a coffee mill, an amount of material retained by a mesh #30 of at least about 60 % of the initial amount of the dosage form, preferably at least about 65 %, or at least about 70 %, or at least about 75 %, or at least about 80 % of the initial amount of the dosage form.

According to the invention, the dosage form is further characterized by providing, after crushing for 50 seconds in a coffee mill, an amount of material retained by a mesh #30 of at least about 55 %, preferably at least about 60 %, or at least about 70 %, or at least about 75 % of the initial amount of the dosage form.

According to the invention, the dosage form is further characterized by providing, after crushing for 60 seconds in a coffee mill, an amount of material retained by a mesh #30 of at least about 45 %, preferably at least about 55 %, or at least about 65 %, or at least about 70 %, or at least about 75 %, or at least about 80 %, or at least about 85 % of the initial amount of the dosage form.

### METHOD OF TREATMENT

According to one aspect, the invention relates to a method of treatment wherein the solid extended release pharmaceutical dosage form, in particular the solid oral extended release pharmaceutical dosage form in accordance with the invention, and as described above in detail, is administered for treatment of pain to a patient in need thereof, wherein the dosage form comprises an opioid analgesic.

Examples of pain that can be treated include e.g. acute or chronic pain, such as cancer pain, neuropathic pain, labor pain, myocardial infarction pain, pancreatic pain, colic pain, post-operative pain, headache pain, muscle pain, arthritic pain, and pain associated with a periodontal disease, including gingivitis and periodontitis, pain associated with inflammatory diseases including, but not limited to, organ transplant rejection; reoxygenation injury resulting from organ transplantation (see Grupp et al., "Protection against Hypoxia-reoxygenation in the Absence of Poly (ADP-ribose) Synthetase in Isolated Working Hearts," J. Mol. Cell Cardiol. 31:297-303 (1999)) including, but not limited to, transplantation of the heart, lung, liver, or kidney; chronic inflammatory diseases of the joints, including arthritis, rheumatoid arthritis, osteoarthritis and bone diseases associated with increased bone resorption; inflammatory bowel diseases, such as ileitis, ulcerative colitis, Barrett's syndrome, and Crohn's disease; inflammatory lung diseases, such as asthma, adult respiratory distress syndrome, and chronic obstructive airway disease; inflammatory diseases of the eye, including corneal dystrophy, trachoma, onchocerciasis, uveitis, sympathetic ophthalmitis and endophthalmitis; chronic inflammatory disease of the gum, including gingivitis and periodontitis; tuberculosis; leprosy; inflammatory diseases of the kidney, including uremic complications, glomerulonephritis and nephrosis; inflammatory disease of the skin, including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; autoimmune diseases, including Type I and Type II diabetes mellitus; diabetic complications, including, but not limited to, diabetic cataract, glaucoma, retinopathy, nephropathy (such as microalbuminuria and progressive diabetic nephropathy), gangrene of the feet, atherosclerotic coronary arterial disease, peripheral arterial disease, nonketotic hyperglycemic-hyperosmolar coma, foot ulcers, joint problems, and a skin or mucous membrane complication (such as an infection, a shin spot, a candidal infection or necrobiosis lipoidica diabeticorum), immune-complex vasculitis, and systemic lupus erythematosus (SLE); inflammatory disease of the heart, such as cardiomyopathy, ischemic heart disease hypercholesterolemia, and artherosclerosis; as well as various other diseases that can have significant inflammatory components, including preeclampsia, chronic liver failure, brain and spinal cord trauma, and cancer. Pain associated with inflammatory disease that can, for example, be a systemic inflammation of the body, exemplified by gram-positive or gram negative shock, hemorrhagic or anaphylactic shock, or shock induced by cancer chemotherapy in response to pro-inflammatory cytokines, *e.g.*, shock associated with pro-inflammatory cytokines. Such shock can be induced, *e.g.,* by a chemotherapeutic agent that is administered as a treatment for cancer. pain associated with nerve injury *(i.e.,* neuropathic pain). Chronic neuropathic pain is a heterogenous disease state with an unclear etiology. In chronic neuropathic pain, the pain can be mediated by multiple mechanisms. This type of pain generally arises from injury to the peripheral or central nervous tissue. The syndromes include pain associated with spinal cord injury, multiple sclerosis, post-herpetic neuralgia, trigeminal neuralgia, phantom pain, causalgia, and reflex sympathetic dystrophy and lower back pain. The chronic pain is different from acute pain in that chronic neuropathic pain patients suffer the abnormal pain sensations that can be described as spontaneous pain, continuous superficial burning and/or deep aching pain. The pain can be evoked by heat-, cold-, and mechano-hyperalgesia, or by heat-, cold-, or mechano-allodynia.Chronic neuropathic pain can be caused by injury or infection of peripheral sensory nerves. It includes, but is not limited to, pain from peripheral nerve trauma, herpes virus infection, diabetes mellitus, causalgia, plexus avulsion, neuroma, limb amputation, and vasculitis. Neuropathic pain can also be caused by nerve damage from chronic alcoholism, human immunodeficiency virus infection, hypothyroidism, uremia, or vitamin deficiencies. Stroke (spinal or brain) and spinal cord injury can also induce neuropathic pain. Cancer-related neuropathic pain results from tumor growth compression of adjacent nerves, brain, or spinal cord. In addition, cancer treatments, including chemotherapy and radiation therapy, can cause nerve injury. Neuropathic pain includes but is not limited to pain caused by nerve injury such as, for example, the pain from which diabetics suffer.

### USE

According to one aspect, the invention relates to a use of polyethylene oxide having an approximate weight average molecular weight of from about 10,000 to less than 1,000,000 in the extended release matrix formulation in a solid extended release pharmaceutical dosage form, wherein the extended release matrix formulation comprises also an active agent and poly(ε-caprolactone) for imparting to the solid extended release dosage form resistance to alcohol extraction.

According to one further aspect, the invention relates to a use of poly(ε-caprolactone) having an approximate number average molecular weight of from about 10,000 to about 200,000 in the extended release matrix formulation in a solid extended release pharmaceutical dosage form, wherein the extended release matrix formulation comprises also an active agent and polyethylene oxide having an approximate weight average molecular weight of from about 10,000 to less than 1,000,000 for imparting to the solid extended release dosage form resistance to crushing.

### EXAMPLES

The present invention will now be more fully described with reference to the accompanying examples.

### GENERAL PROCEDURES

### Dissolution Method and Instrumentation

Apparatus - USP Type I (Baskets), 100 rpm at 37°C
Media - 900 ml Simulated Gastric Fluid, or 900 ml Simulated Gastric Fluid with 40% ethanol
Automated dissolution sampling device equipped with or without in-residence sampling probes, and in-line 25 mm glass fiber 1 µm filters (Waters P/N WAT200818) or 10 µm cannula filters (Hanson Research P/N 27-101-074)
HPLC System - Waters Alliance 2690/2695 HPLC system with 2487 UV-Vis absorbance detector or 996 photodiode array (PDA) detector
Ultrasonic equipment - Bransonic 8510
HPLC Vials - 12 x 32 mm screw neck vial and screw cap
Mobile phase filtration system -
   HPLC filtration assembly, 47mm Ultra-Ware all glass, Kimble
   Nylon-66 membrane filter 45 µm
HPLC Column and Conditions -
   Waters Atlantis dC18 (3.0 x 250 mm, 5 µm)
   Column heater - Column temperature 60 C
   HPLC pump - Flow rate 1.0 ml/min
   UV detector - Wavelength 230 nm
   Autoinjector - Injection volume 10 µl
   Run time - 10 minutes
   Quantitation Parameter - Peak Area

### Crush testing

### Equipment

Mill - Krups™ Coffee Mill Type 203, F2037051/86C-3108
Balance - Mettler Toledo
Stop Watch - Extech Instruments
Light Source - Schott EKE ACE 1, Serial No. 145862,
Stereomicroscope - Zeiss Stemi™ SV11 Apo, Diagnostic Instruments Inc.
Transmitted Light Base - TLB6000 series, Model # TLB 6.1
Camera - Spot Insight Firewire, 2 Megasample, Serial # - 235324, Model # 11.2
ColorMosaic™, Diagnostic Instruments Inc.
Operating Software - Spot Advanced, Version 4.6.4.3, 1997-2006, Spot Software, Diagnostic Instruments Inc
Calibration Standard - NIST Traceable Magnification Standards for Light
Microscopes, Reference - Duke Scientific, Slide # 23, Lot # 17855, 2022 µm ± 40 µm
Photo Image Calibration - SM 1.0 S1.0x at magnification S1.0x, 286 Sensor Pixels = 2.022 mm

### Photography Parameters

Lamp Setting - 90
Magnification - 1.0x
Software Controls - Auto
Brightfield-Transmitted Light, Brightness - 1.0, Gain Limit - 1.0, Auto Brightness - ON
Post Processing - Neutral, Gamma - 0.70

### Crush Testing Procedure

1. Oxycodone HCl or naltrexone HCl melt-extruded multi-particulates (MEMs) equivalent to one dose of oxycodone or naltrexone were weighed and added to the stainless steel milling chamber of the Krups™ mill.
2. The material was milled in 10 second intervals up to a total of 60 seconds while monitoring the elapsed time using a stop watch.
3. The material that was retained and which passed through mesh # 30 (600 µm) after each round of milling was weighed and the weight recorded. The material was returned to the mill for the next round of milling.
4. The mesh-retained and passed material were also evaluated visually using the stereomicroscope.

### Melt Extrusion Manufacturing Equipment

### Examples 1-18:

Micro-27 GGC Extruder (Co-Rotation)
Neslab Chiller (Temperature Setting 5°C)
Die Plate Hole diameter (mm): 1.0 (8-hole die plate)
AccuRate™ Volumetric Feeder
Co-rotating Screw Assembly
8-ft Dorner Conveyor Belt (2100 Series)
ExAir Air Knives
Balances
Randcastle Pelletizer
Laser Mike

### Examples 19-41:

Nano-16 25D Extruder with OD/ID ratio 1.18/1
4 heating zones/barrels
Screw Diameter - 16 mm
Drive Power - 1.12 kW
Co-rotating Screw Assembly
12-ft Conveyor Belt
ExAir Air Knives
Balances
Feeder Micro Plunger
Screw Standard
Die Rod 1.5 mm
Nozzle 2.0 mm
Downstream Pelletizer

### EXAMPLEs 1 - 6

### Composition

The compositions of the poly(ε-caprolactone) melt-extruded multi-particulates (MEMs) for examples 1-6 are summarized in Tables I to III below:

**Table I**

| **Example Number** | **1** | | | **2** | | |
|---|---|---|---|---|---|---|
| **Ingredient (Trade Name)** | **Amount** | | | **Amount** | | |
| | **(% w/w)** | **unit (mg)** | **batch (g)** | **(% w/w)** | **unit (mg)** | **batch (g)** |
| Oxycodone HCl* | **20.0** | 40.0 | 100.0 | **20.0** | 40.0 | 100.0 |
| Poly(ε-caprolactone), **Mn** ~ **98,000** (PC-12) | **47.4** | 94.8 | 237.0 | **63.2** | 126.4 | 316.0 |
| Polyethylene oxide, Mw ~ 100,000 (PEO WSR N10) | **31.6** | 63.2 | 158.0 | **15.8** | 31.6 | 79.0 |
| Butylated Hydroxy Toluene (BHT) | **1.0** | 2.0 | 5.0 | **1.0** | 2.0 | 5.0 |
| Glyceryl behenate (Compritol 888) | - | - | - | - | - | - |
| Total | **100** | 200 | 500 | **100** | 200 | 500 |

| | | | | | | |
|---|---|---|---|---|---|---|
| * Amount not corrected for water or impurities. | | | | | | |

**Table II**

| **Example Number** | **3** | | | **4** | | |
|---|---|---|---|---|---|---|
| **Ingredient (Trade Name)** | **Amount** | | | **Amount** | | |
| | **(% w/w)** | **unit (mg)** | **batch (g)** | **(% w/w)** | **unit (mg)** | **batch (g)** |
| Oxycodone HCl* | **15.0** | 30.0 | 75.0 | **15.0** | 30.0 | 75.0 |
| Poly(ε-caprolactone), **Mn** ~ **98,000** (PC-12) | **67.2** | 134.4 | 336.0 | **50.4** | 100.8 | 252.0 |
| Polyethylene oxide, Mw ~ | **16.8** | 33.6 | 84.0 | **33.6** | 67.2 | 168.0 |
| 100,000 (PEO WSR N10) | | | | | | |
| Butylated Hydroxy Toluene (BHT) | **1.0** | 2.0 | 5.0 | **1.0** | 2.0 | 5.0 |
| Glyceryl behenate (Compritol 888) | - | - | - | - | - | - |
| Total | **100** | 200 | 500 | **100** | 200 | 500 |

| | | | | | | |
|---|---|---|---|---|---|---|
| * Amount not corrected for water or impurities. | | | | | | |

**Table III**

| **Example Number** | **5** | | | **6** | | |
|---|---|---|---|---|---|---|
| **Ingredient (Trade Name)** | **Amount** | | | **Amount** | | |
| | **(% w/w)** | **unit (mg)** | **batch (g)** | **(% w/w)** | **unit (mg)** | **batch (g)** |
| Oxycodone HCl* | **20.0** | 40.0 | 100.0 | **20.0** | 40.0 | 75.0 |
| Poly(ε-caprolactone), **Mn** ~ **98,000** (PC-12) | **53.0** | 106.0 | 225.0 | **45.0** | 90.0 | 252.0 |
| Polyethylene oxide, Mw ~ 100,000 (PEO WSR N10) | **21.0** | 42.0 | 145.0 | **29.0** | 58.0 | 168.0 |
| Butylated Hydroxy Toluene (BHT) | **1.0** | 2.0 | 5.0 | **1.0** | 2.0 | 5.0 |
| Glyceryl behenate (Compritol 888) | **5.0** | 10.0 | 25.0 | **5.0** | 10.0 | 25.0 |
| Total | **100** | 200 | 500 | **100** | 200 | 500 |

| | | | | | | |
|---|---|---|---|---|---|---|
| * Amount not corrected for water or impurities. | | | | | | |

### Manufacturing Procedure

1. Blending: Oxycodone HCl, poly(ε-caprolactone) (used as obtained from the manufacturer in the form of 0.5 to 4 mm flakes without further processing), polyethylene oxide and milled BHT were loaded into a LDPE bag (12" x 20") and blended for 30 s to 1 minute, or until visually homogenous, at ambient temperature.
2. Feeding into extruder: Materials blended in Step 1 were added to a single-screw volumetric dispenser (AccuRate™) and its feed rate was calibrated to 25 ± 0.5 g/min.
3. Melt Extrusion: The blend was metered into 27-Micro GGC twin screw extruder with 10 heating zones, fitted with a main gated adapter and a multi-orifice coat-hanger type die and processed into strands.
4. Cooling: The strands from step 3 were drawn on an 8-ft conveyer belt fitted with 2 air knives and cooled at ambient temperature.
5. Pelletizing: The cooled strands from step 4 were cut into pellets of dimensions 1 mm x 1mm (Pelletizer Settings: Nip Roll (Hz) - 7.0; Cutter Roll (Hz) - 13.4).
6. Providing slices: Alternatively, the cooled strands from step 4 were cut into tablet slices with a diameter of 10 mm and a thickness of 1-2 mm manually with a blade.

The co-rotating screw configuration for Examples 1-6 is given in Table IV.

**Table IV**

| **Quantity** | **Screw Element Type** |
|---|---|
| FEED END | |
| 1 | GFA 2-40-90 |
| 1 | GFA 2-30-90 |
| 1 | GFA 2-20-90 |
| 2 | KB5 2-30-30 |
| 1 | GFA 2-30-60 |
| 2 | KB5 2-30-30 |
| 2 | KB5 2-30-60 |
| 1 | GFA 2-30-30 |
| 1 | KB5 2-30-60 |
| 1 | KB5 2-30-90 |
| 1 | KS1 2-10A |
| 1 | KS1 2-10E (90°) |
| 1 | GFA 2-30-90 |
| 1 | KB5 2-30-60 |
| 2 | KB5 2-30-90 |
| 1 | GFA 2-40-90 |
| 1 | GFA 2-30-90 |
| 1 | GFA 2-30-30 |
| 1 | GFA 2-20-90 |
| | HEXPLUG |

### Example 1

The processing conditions for Example 1 at the time of sampling are summarized in Table 1 below.

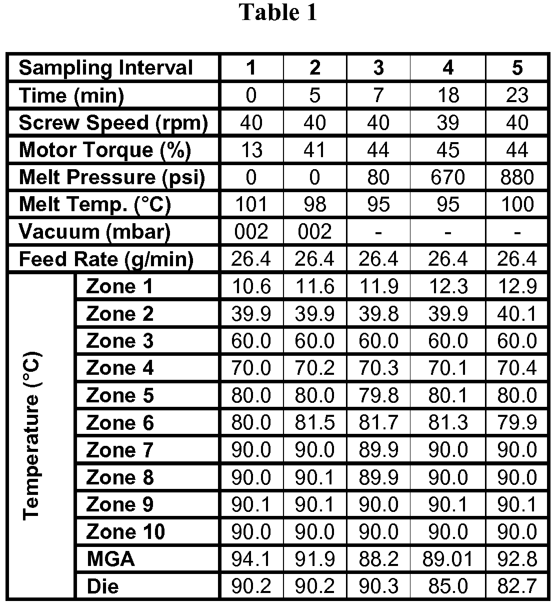

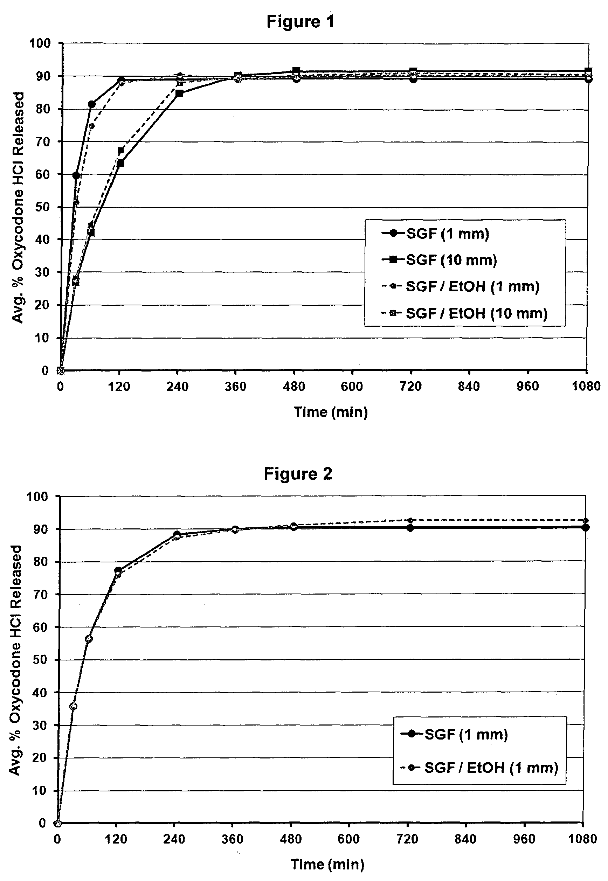

The dissolution results for Example 1 MEMs and tablet slices with a diameter of 10 mm and a thickness of 1-2 mm are summarized in Figure 1 and Table 1a and 1b.

**Table 1a**

| | **10 mm slices; composite of sampling intervals 1 and 2** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 26.9 (29.4) | 42.0 (45.9) | 63.6 (69.4) | 84.8 (92.6) | 90.2 (98.5) | 91.6 (100.0) | 91.7 (100.0) | 91.7 (100.0) |
| **SGF/ EtOH** (normalized) | 28.0 (30.9) | 44.4 (49.0) | 67.5 (74.6) | 88.1 (97.3) | 89.6 (98.9) | 90.3 (99.6) | 91.1 (100.6) | 90.6 (100.0) |

**Table 1b**

| | **1 mm x 1 mm MEMs; composite of sampling intervals 3 to 5** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 59.8 (67.0) | 81.5 (91.3) | 88.9 (99.5) | 89.1 (99.7) | 89.3 (100.0) | 89.4 (100.1) | 89.4 (100.1) | 89.3 (100.0) |
| **SGF/ EtOH** (normalized) | 51.5 (57.2) | 74.9 (83.2) | 87.9 (97.5) | 90.5 (100.5) | 89.1 (99.0) | 90.0 (99.9) | 90.4 (100.4) | 90.1 (100.0) |

The crush testing results of Example 1 are summarized in Table 1c.

**Table 1c**

| **Mean Pellet Dimension D (mm) x L (mm) (n = 37-40)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.84 ± 0.21 x 1.22 ± 0.26 | **Wt (mg)** | 205.2 | 199.4 | 192.6 | 182.6 | 168.4 | 164.3 | 157.2 |
| | **% Rtnd** | 100.0 | 97.2 | 93.9 | 89.0 | 82.1 | 80.1 | 76.6 |
| 1.14 ± 0.08 x 1.27 ± 0.28 | **Wt (mg)** | 201.1 | 170.5 | 143.0 | 114.7 | 91.9 | 76.6 | 64.0 |
| | **% Rtnd** | 100.0 | 84.8 | 71.1 | 57.1 | 45.7 | 38.1 | 31.8 |

### Example 2

The processing conditions for Example 2 at the time of sampling are summarized in Table 2 below.

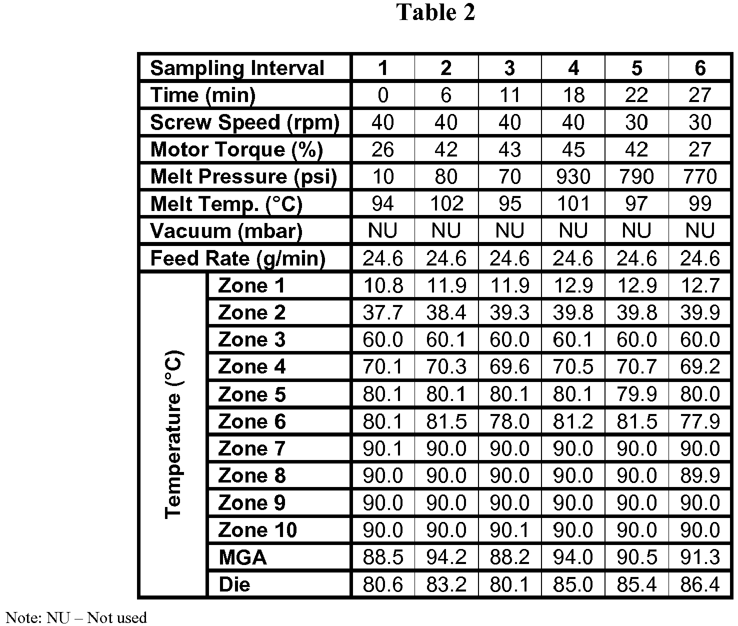

The dissolution results for Example 2 MEMs are summarized in Figure 2 and Table 2a.

**Table 2a**

| | **1 mm x 1 mm MEMs; Mixed Bulk Pellets** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 35.9 (39.8) | 56.5 (62.5) | 77.3 (85.6) | 88.4 (97.8) | 90.0 (99.6) | 90.6 (100.3) | 90.4 (100.1) | 90.3 (100.0) |
| **SGF/ EtOH** (normalized) | 35.9 (38.8) | 56.2 (60.8) | 76.1 (82.2) | 87.5 (94.6) | 89.6 (96.9) | 91.2 (98.6) | 92.6 (100.1) | 92.5 (100.0) |

The crush testing results of Example 2 are summarized in Table 2b .

**Table 2b**

| **Mean Pellet Dimension D (mm) x L (mm) (n = 34-42)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.85 ± 0.14 x 1.15 ± 0.26, | **Wt (mg)** | 200.2 | 193.0 | 181.6 | 177.8 | 168.6 | 158.9 | 148.8 |
| | **% Rtnd** | 100.0 | 96.4 | 90.7 | 88.8 | 84.2 | 79.4 | 74.3 |
| 1.19 ± 0.18 x 1.43 ± 0.34, | **Wt (mg)** | 203.0 | 190.9 | 181.7 | 174.7 | 164.6 | 162.1 | 154.7 |
| | **% Rtnd** | 100.0 | 94.1 | 89.5 | 86.1 | 81.1 | 79.9 | 76.2 |

### Example 3

The processing conditions for Example 3 at the time of sampling are summarized in Table 3 below.

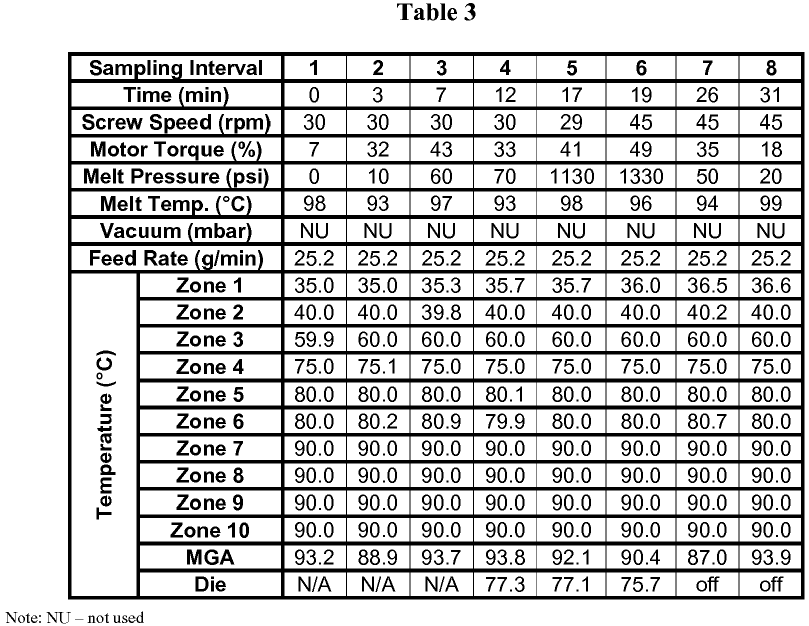

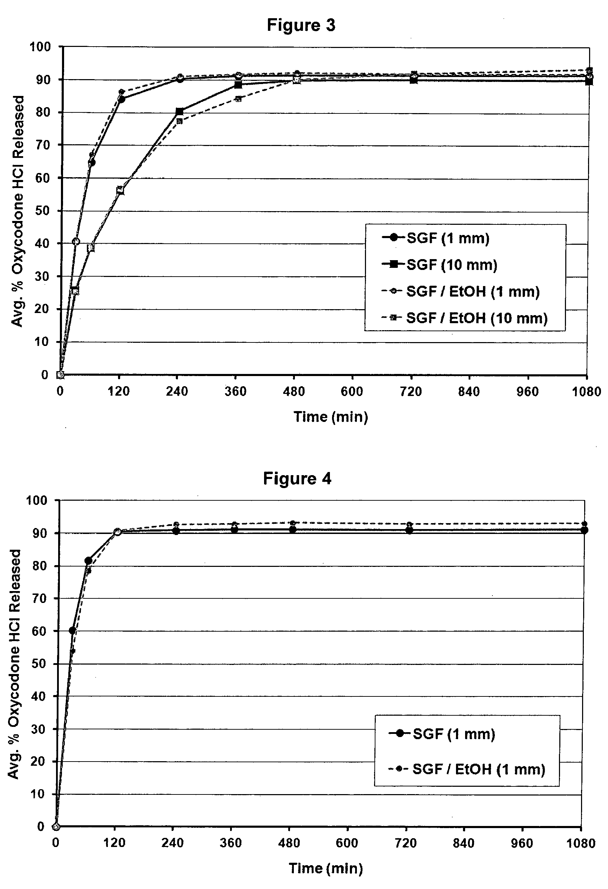

The dissolution results for Example 3 MEMs and tablet slices with a diameter of 10 mm and a thickness of 1-2 mm are summarized in Figure 3 and Tables 3a and 3b.

**Table 3a**

| | **1 mm x 1 mm MEMs; Mixed Bulk Pellets** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 40.8 (44.6) | 64.8 (70.8) | 84.3 (92.1) | 90.4 (98.8) | 91.3 (99.8) | 91.5 (100.0) | 91.5 (100.0) | 91.5 (100.0) |
| **SGF/ EtOH** (normalized) | 41.0 (44.6) | 67.1 (73.0) | 86.5 (94.1) | 91.2 (99.3) | 91.8 (99.9) | 92.4 (100.5) | 92.0 (100.1) | 91.9 (100.0) |

**Table 3b**

| | **10 mm slices; composite of sampling intervals 7 and 8** | | | | | | | |
|---|---|---|---|---|---|---|---|---|
| **Dissolution Media** | **Mean Oxycodone HCl % Released (n=2) *** | | | | | | | |
| | **30 min** | **60 min** | **120 min** | **240 min** | **360 min** | **480 min** | **720 min** | **1080 min** |
| **SGF** (normalized) | 25.8 (28.7) | 38.7 (43.0) | 56.1 (62.3) | 80.5 (89.4) | 88.6 (98.5) | 90.1 (100.2) | 90.2 (100.3) | 90.0 (100.0) |
| **SGF**/ **EtOH** (normalized) | 25.1 (26.9) | 39.1 (41.8) | 56.8 (60.7) | 77.5 (82.9) | 84.5 (90.4) | 90.2 (96.6) | 92.2 (98.6) | 93.4 (100.0) |

| | | | | | | | | |
|---|---|---|---|---|---|---|---|---|
| * n=1 for values measured in SGF. | | | | | | | | |

The crush testing results of Example 3 are summarized in Table 3c .

**Table 3c**

| **Mean Pellet Dimension D (mm) x L (mm) (n = 38)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.13 ± 0.11 x 1.15 ± 0.20 | **Wt (mg)** | 202.9 | 202.4 | 195.4 | 187.9 | 177.8 | 176.6 | 172.2 |
| | **% Rtnd** | 100.0 | 99.8 | 96.3 | 92.6 | 87.6 | 87.0 | 84.9 |
| 0.86 ± 0.13 x 1.23 ± 0.33 | **Wt (mg)** | 201.7 | 200.3 | 197.9 | 183.7 | 176.7 | 168.6 | 164.1 |
| | **% Rtnd** | 100.0 | 99.3 | 98.1 | 91.1 | 87.6 | 83.6 | 81.4 |

### Example 4

The processing conditions for Example 4 at the time of sampling are summarized in Table 4 below.

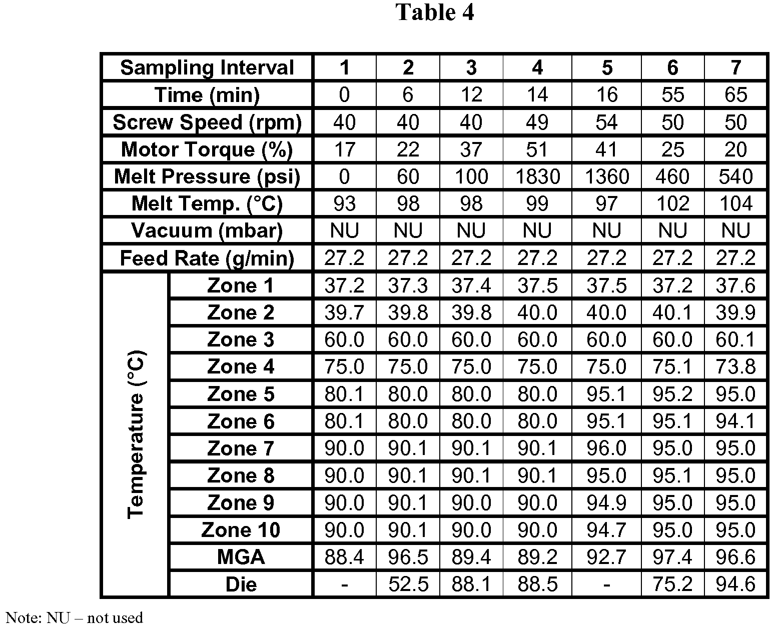

The dissolution results for Example 4 MEMs are summarized in Figure 4 and Table 4a.

**Table 4a**

| | **1 mm x 1 mm MEMs; Mixed Bulk Pellets** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 60.3 (66.1) | 81.7 (89.7) | 90.6 (99.4) | 90.9 (99.8) | 91.2 (100.0) | 91.2 (100.1) | 91.0 (99.9) | 91.1 (100.0) |
| **SGF/ EtOH** (normalized) | 54.1 (58.2) | 78.6 (84.5) | 90.7 (97.5) | 92.8 (99.7) | 92.8 (99.8) | 93.2 (100.2) | 92.8 (99.8) | 93.0 (100.0) |

The crush testing results of Example 4 are summarized in Table 4b .

**Table 4b**

| **Mean Pellet Dimension D (mm) x L (mm) (n = 36-38)** | | **Milling Number / Time** | | | | | | |
|---|---|---|---|---|---|---|---|---|
| | **Amount Retained** | **Initial / 0s** | **1 / 10s** | **2 / 20s** | **3 / 30s** | **4 / 40s** | **5 / 50s** | **6 / 60s** |
| 1.10 ± 0.12 x 1.24 ± 0.26 | **Wt (mg)** | 191.3 | 188.1 | 180.1 | 174.6 | 169.5 | 167.5 | 191.3 |
| | **% Rtnd** | 100.0 | 96.1 | 94.5 | 90.5 | 87.8 | 85.2 | 84.2 |
| 0.89 ± 0.14 x 1.21 ± 0.31 | **Wt (mg)** | 198.3 | 195.5 | 195.1 | 186.9 | 181.0 | 177.0 | 198.3 |
| | **% Rtnd** | 100.0 | 98.8 | 97.4 | 97.2 | 93.1 | 90.2 | 88.2 |

### Example 5

The processing conditions for Example 5 at the time of sampling are summarized in Table 5 below.

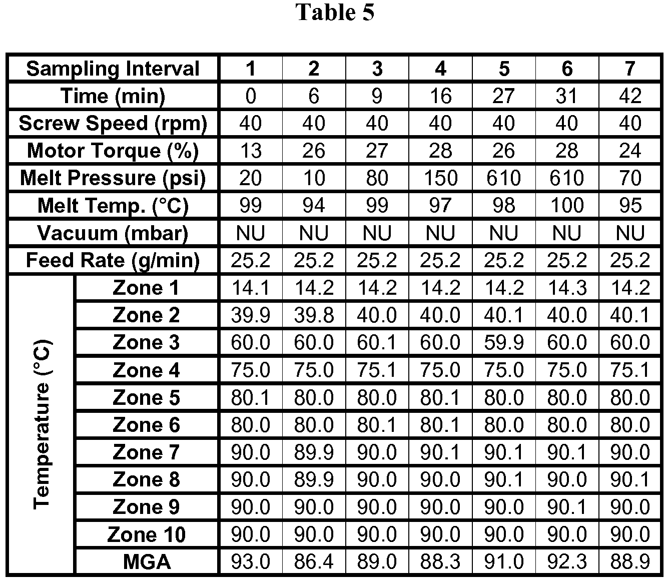

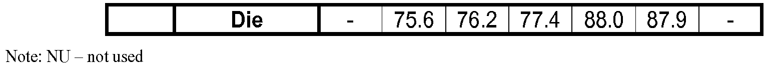

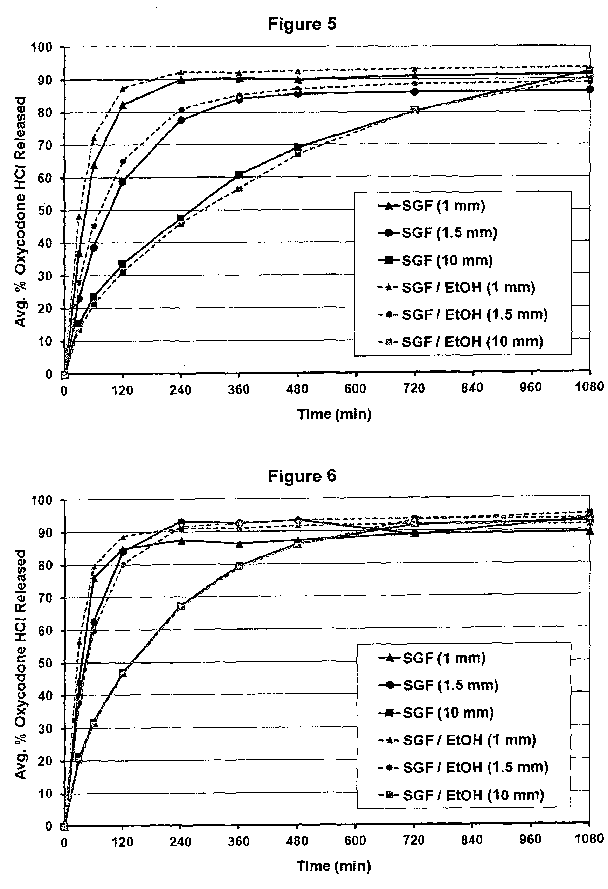

The dissolution results for Example 5 MEMs and slices with a diameter of 10 mm and a thickness of 1-2 mm are summarized in Figure 5 and Tables 5a to 5c.

**Table 5a**

| | **1 mm x 1 mm MEMs; Mixed Bulk Pellets** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 37.1 (40.5) | 63.9 (69.7) | 82.5 (90.1) | 90.1 (98.4) | 90.5 (98.8) | 90.1 (98.4) | 91.1 (99.4) | 91.6 (100.0) |
| **SGF/ EtOH** (normalized) | 48.4 (51.7) | 72.3 (77.3) | 87.5 (93.4) | 92.3 (98.6) | 92.0 (98.3) | 92.6 (99.0) | 93.2 (99.6) | 93.6 (100.0) |

**Table 5b**

| | **1.5 mm x 1.5 mm MEMs; Mixed Bulk Pellets** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 23.1 (26.8) | 38.6 (44.7) | 58.9 (68.1) | 77.7 (89.9) | 84.0 (97.2) | 85.7 (99.1) | 86.2 (99.6) | 86.5 (100.0) |
| **SGF/ EtOH** (normalized) | 28.1 (31.5) | 45.4 (50.9) | 65.1 (73.1) | 81.1 (91.0) | 85.2 (95.7) | 87.2 (97.9) | 88.6 (99.4) | 89.1 (100.0) |

**Table 5c**

| | **10 mm slices; sampling interval 7** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 15.4 (16.7) | 23.4 (25.3) | 33.7 (36.3) | 47.6 (51.3) | 60.9 (65.5) | 69.2 (74.5) | 80.4 (86.7) | 92.5 (100.0) |
| **SGF/EtOH** (normalized) | 13.6 (15.0) | 21.1 (23.3) | 31.2 (34.3) | 46.0 (50.6) | 56.6 (62.2) | 67.1 (73.9) | 80.5 (88.8) | 90.5 (100.0) |

The crush testing results of Example 5 are summarized in Table 5d.

**Table 5d**

| **Mean Pellet Dimension D (mm) x L (mm) (n = 25-31)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.99 ± 0.04 x 1.17 ± 0.51, | **Wt (mg)** | 200.6 | 193.7 | 187.4 | 184.6 | 178.9 | 174.2 | 169.8 |
| | **% Rtnd** | 100.0 | 96.6 | 93.4 | 92.0 | 89.2 | 86.8 | 84.6 |
| 1.56 ± 0.11 x 1.31 ± 0.16, | **Wt (mg)** | 203.9 | 185.6 | 181.4 | 176.1 | 172.5 | 164.7 | 162.6 |
| | **% Rtnd** | 100.0 | 91.0 | 88.99 | 86.4 | 84.6 | 80.8 | 79.8 |

### Example 6

The processing conditions for Example 6 at the time of sampling are summarized in Table 6 below.

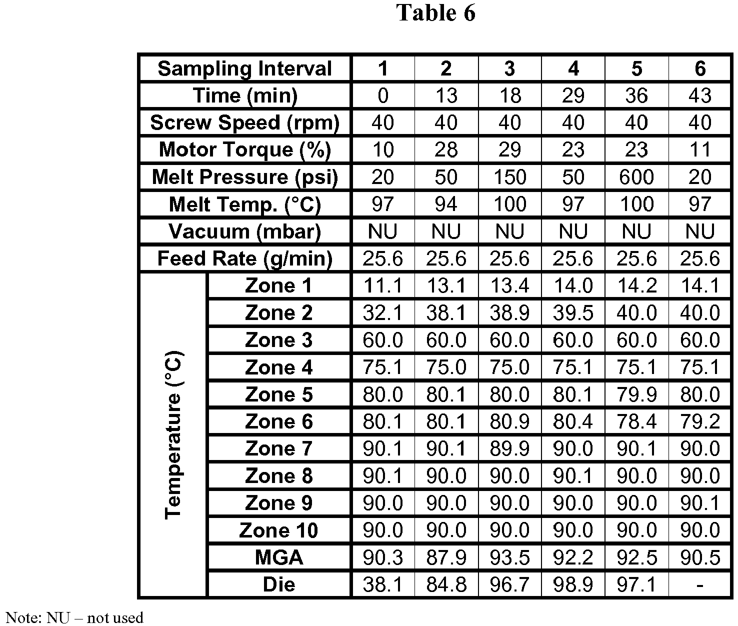

The dissolution results for Example 6 MEMs and slices with a diameter of 10 mm and a thickness of 1-2 mm are summarized in Figure 6 and Table 6a to 6c.

**Table 6a**

| | **1 mm x 1 mm MEMs; composite of sampling intervals 1 to 5** | | | | | | | |
|---|---|---|---|---|---|---|---|---|
| **Dissolution Media** | **Mean Oxvcodone HCl % Released (n=2)** | | | | | | | |
| | **30 min** | **60 min** | **120 min** | **240 min** | **360 min** | **480 min** | **720 min** | **1080 min** |
| **SGF** (normalized) | 44.4 (49.7) | 76.0 (85.0) | 84.8 (94.9) | 87.5 (97.9) | 86.3 (96.6) | 87.3 (97.7) | 89.1 (99.6) | 89.4 (100.0) |
| **SGF/ EtOH** (normalized) | 56.8 (61.9) | 79.6 (86.7) | 88.8 (96.8) | 90.9 (99.0) | 91.0 (99.2) | 91.8 (100.0) | 92.1 (100.3) | 91.8 (100.0) |

**Table 6b**

| | **1.5 mm x 1.5 mm MEMs; Mixed Bulk Pellets** | | | | | | | |
|---|---|---|---|---|---|---|---|---|
| **Dissolution Media** | **Mean Oxycodone HCI % Released (n=2)** | | | | | | | |
| | **30 min** | **60 min** | **120 min** | **240 min** | **360 min** | **480 min** | **720 min** | **1080 min** |
| **SGF** (normalized) | 39.9 (42.6) | 62.7 (66.8) | 84.2 (89.8) | 93.2 (99.5) | 92.6 (98.8) | 93.4 (99.7) | 88.9 (94.9) | 93.7 (100.0) |
| **SGF/ EtOH** (normalized) | 38.0 (40.5) | 59.8 (63.9) | 80.3 (85.7) | 91.6 (97.8) | 92.7 (99.0) | 93.5 (99.8) | 93.8 (100.1) | 93.7 (100.0) |

**Table 6c**

| | **10 mm slices; sampling interval 6** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 21.3 (22.9) | 31.9 (34.3) | 46.9 (50.5) | 67.4 (72.5) | 79.6 (85.6) | 86.2 (92.7) | 92.0 (99.0) | 92.9 (100.0) |
| **SGF/ EtOH** (normalized) | 20.3 (21.4) | 31.3 (32.9) | 46.7 (49.1) | 66.9 (70.4) | 79.1 (83.2) | 85.8 (90.2) | 93.5 (98.3) | 95.1 (100.0) |

The crush testing results of Example 6 are summarized in Table 6d.

**Table 6d**

| **Mean Pellet Dimension D (mm) x L (mm) (n = 29-37)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.76 ± 0.22 x 0.99 ± 0.22, (∼1 x 1) | **Wt (mg)** | 203.9 | 195.9 | 184.8 | 180,2 | 174.1 | 172.7 | 167.6 |
| | **% Rtnd** | 100.0 | 96.1 | 90.61 | 88.4 | 85.4 | 84.7 | 82.2 |
| 1.42 ± 0.17 x 1.33 ± 0.14, (∼ 1.5 x 1.5) | **Wt (mg)** | 204.5 | 186.0 | 180.3 | 175.1 | 170.3 | 165.1 | 161.4 |
| | **% Rtnd** | 100.0 | 91.0 | 88.18 | 85.6 | 83.3 | 80.7 | 78.9 |
| 0.71 ± 0.08 x 1.05 ± 0.23, (≤ 1.0 x 1.0) | **Wt (mg)** | 201.4 | 196.9 | 190.2 | 187.2 | 180.6 | 177.8 | 174.0 |
| | **% Rtnd** | 100.0 | 97.8 | 94.4 | 92.9 | 89.7 | 88.3 | 86.4 |

### EXAMPLEs 7 - 9

### Composition

The compositions of the poly(ε-caprolactone) melt-extruded multi-particulates (MEMs) for examples 7-9 are summarized in Table V below:

**Table V**

| **Example** | **7** | | | **8** | | | **9** | | |
|---|---|---|---|---|---|---|---|---|---|
| **Ingredient (Trade Name)** | **Amount** | | | **Amount** | | | **Amount** | | |
| | **(% w/w)** | **unit (mg)** | **batch (% (g)** | **(% w/w)** | **unit (mg)** | **batch (% (g)** | **(% w/w)** | **unit (mg)** | **batch (g)** |
| Oxycodone HCl* | **15.0** | 30.0 | 112.5 | **15.0** | 30.0 | 112.5 | **15.0** | 30 | 112.5 |
| Poly(ε-caprolactone), **Mn ∼ 98,000** (PC-12) | **69.0** | 138.0 | 517.5 | **71.0** | 142.0 | 532.5 | **73.0** | 146.0 | 547.5 |
| Polyethylene oxide, Mw ∼ 100,000 (PEO WSR N10) | **15.0** | 30.0 | 112.5 | **13.0** | 26.0 | 97.5 | **11.0** | 22.0 | 82.5 |
| Butylated Hydroxy Toluene (BHT) | **1.0** | 2.0 | 7.5 | **1.0** | 2.0 | 7.5 | **1.0** | 2.0 | 7.5 |
| Total | **100** | 200 | 750 | **100** | 200 | 750 | **100** | 200 | 750 |

| | | | | | | | | | |
|---|---|---|---|---|---|---|---|---|---|
| * Amount not corrected for water or impurities. | | | | | | | | | |

### Manufacturing Procedure

1. Blending: Oxycodone HCl, poly(ε-caprolactone) (used as obtained from the manufacturer in the form of 0.5 to 4 mm flakes without further processing), polyethylene oxide and milled BHT were loaded into a LDPE bag (12" x 20") and blended for 30 s to 1 minute, or until visually homogenous, at ambient temperature.
2. Feeding into Extruder: Materials blended in Step 1 were added to a single-screw volumetric dispenser (AccuRate™) and its feed rate was calibrated to 25 ± 0.5 g/min.
3. Melt Extrusion: The blend was metered into a 27-Micro GGC twin screw extruder with 10 heating zones, fitted with a main gated adapter and a multi-orifice coat-hanger type die and processed into strands.
4. Cooling: The strands from step 3 were drawn on an 8-ft conveyer belt fitted with 2 air knives and cooled at ambient temperature.
5. Pelletizing: The cooled strands from step 4 were cut into pellets of dimensions 1 mm x 1mm (Pelletizer Settings: Nip Roll (Hz) - 8.0; Cutter Roll (Hz) - 15.3), and 2 mm x 2mm (Pelletizer Settings: Nip Roll (Hz) - 8.0; Cutter Roll (Hz) - 9.05).
6. The material from step 5 was analyzed for drug release as Pass 1 material.
7. The step 5 material was again extruded at melt extrusion processing conditions similar to Pass 1 melt extrusion.
8. Step 7 material was cooled and pelletized similar to Pass 1 material.
9. For evaluation of content uniformity, MEMs samples were collected at various times during the extrusion. During the first pass of material through the extruder, beginning MEMs sample was collected during sampling interval 2, middle sample during interval 3 and end sample during interval 4. During the second pass of material through the extruder, beginning sample was collected during sampling interval 6, middle sample during interval 7 and end sample during intervals 8/9. 4.

The co-rotating screw configuration for Examples 7-9 is given in Table VI.

**Table VI**

| **Quantity** | **Screw Element Type** |
|---|---|
| FEED END | |
| 1 | GFA 2-40-90 |
| 1 | GFA 2-30-90 |
| 1 | GFA 2-20-90 |
| 2 | KB5 2-30-30 |
| 1 | GFA 2-30-60 |
| 2 | KB5 2-30-30 |
| 2 | KB5 2-30-60 |
| 1 | GFA 2-30-30 |
| 1 | KB5 2-30-60 |
| 1 | KB5 2-30-90 |
| 1 | KS1 2-10A |
| 1 | KS1 2-10E (90°) |
| 1 | GFA 2-30-90 |
| 1 | KB5 2-30-60 |
| 2 | KB5 2-30-90 |
| 1 | GFA 2-40-90 |
| 1 | GFA 2-30-90 |
| 1 | GFA 2-30-30 |
| 1 | GFA 2-20-90 |
| | HEXPLUG |

### Example 7

The processing conditions for Example 7 at the time of sampling are summarized in Table 7 below. Pass 1 and Pass 2 indicate the first and second passage thru extruder.

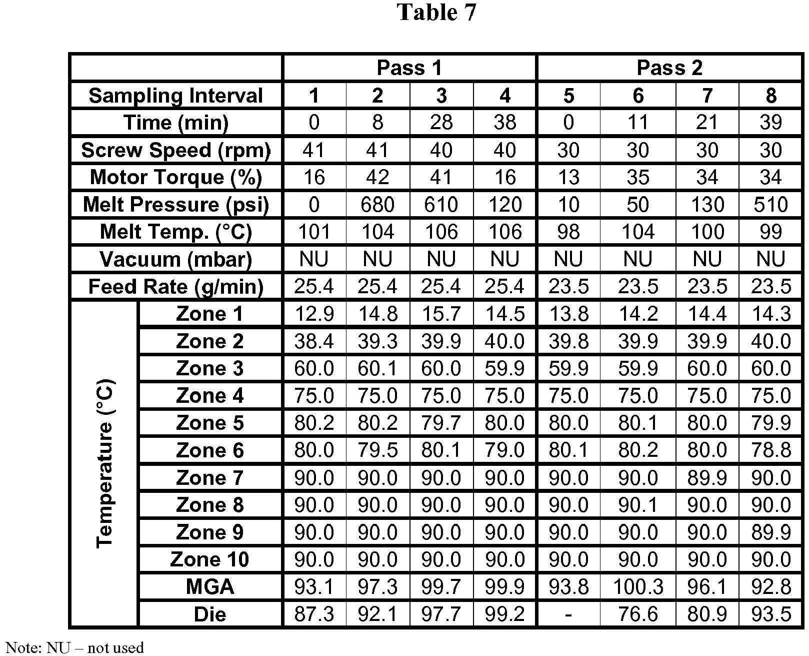

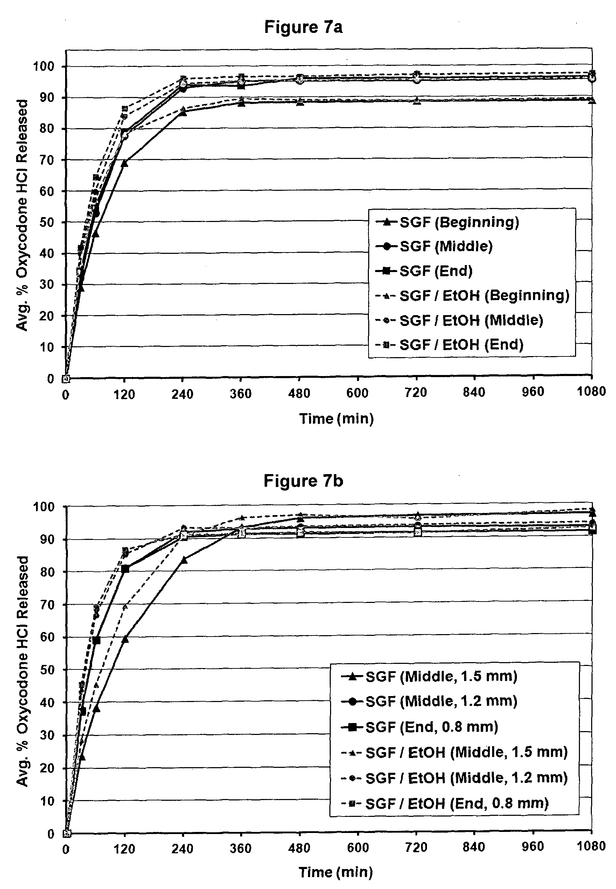

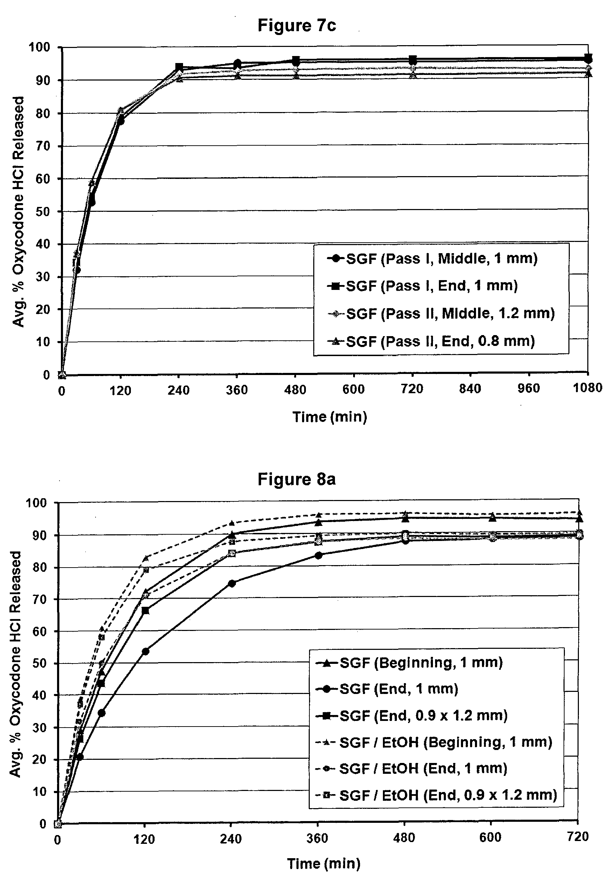

The dissolution results for Example 7 MEMs are summarized in Figures 7a to 7c and Tables 7a to 7d.

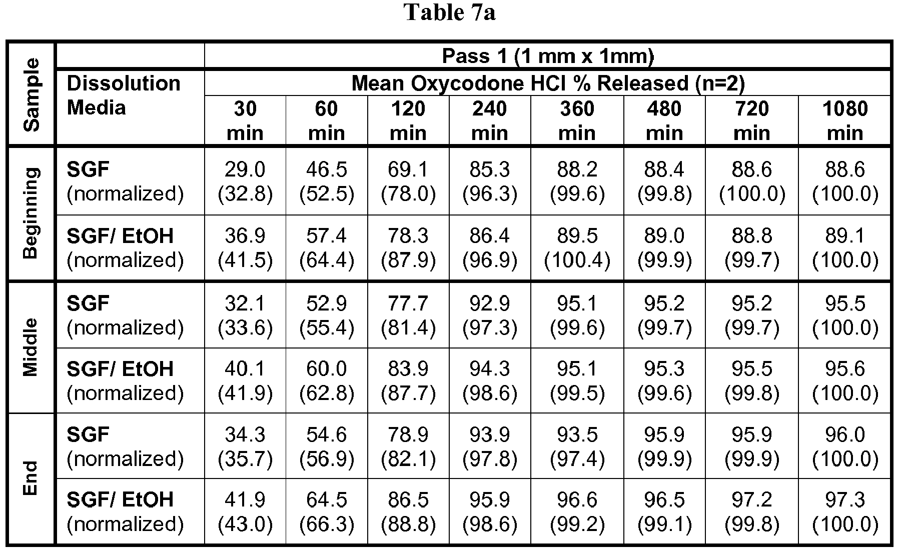

**Table 7b**

| | **Pass 2, Middle (MEMs - 1 mm x 1.5 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 23.6 (24.4) | 38.4 (39.6) | 59.5 (61.4) | 83.7 (86.4) | 93.2 (96.2) | 96.0 (99.0) | 96.6 (99.7) | 96.9 (100.0) |
| **SGF/ EtOH** (normalized) | 28.3 (28.8) | 45.4 (46.3) | 69.5 (70.8) | 91.0 (92.7) | 96.2 (98.1) | 97.0 (98.9) | 95.7 (97.5) | 98.1 (100.0) |

**Table 7c**

| | **Pass 2, Middle (MEMs - 1.2 mm x 1 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 37.6 (40.4) | 59.1 (63.5) | 81.1 (87.2) | 91.8 (98.7) | 92.7 (99.7) | 93.0 (100.0) | 93.2 (100.3) | 93.0 (100.0) |
| **SGF/ EtOH** (normalized) | 43.1 (45.8) | 66.7 (70.9) | 85.4 (90.7) | 93.2 (99.1) | 92.9 (98.7) | 93.4 (99.2) | 93.8 (99.6) | 94.1 (100.0) |

**Table 7d**

| | **Pass 2, End (MEMs - ≤ 1 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **720 min** | **1080 min** |
| **SGF** (normalized) | 37.3 (40.7) | 59.0 (64.5) | 81.0 (88.5) | 90.6 (99.0) | 91.2 (99.6) | 91.3 (99.7) | 91.4 (99.9) | 91.6 (100.0) |
| **SGF/ EtOH** (normalized) | 45.5 (49.1) | 69.0 (74.5) | 86.7 (93.6) | 91.3 (98.5) | 91.2 (98.4) | 91.6 (98.9) | 91.3 (98.6) | 92.6 (100.0) |

The crush testing results of Example 7 are summarized in Table 7e.

**Table 7e**

| **Mean Pellet Dimension D (mm) x L (mm) (n = 12-35)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.05 ± 0.09 x 0.95 ± 0.16, (Pass 1 -Middle - 1 mm) | **Wt (mg)** | 204.2 | 193.0 | 190.3 | 182.3 | 174.1 | 169.4 | 154.1 |
| | **% Rtnd** | 100.0 | 94.5 | 93.2 | 89.3 | 85.2 | 82.9 | 75.5 |
| 1.19 ± 0.08 x 1.51 ± 0.16, (Pass 2 -2 mm) | **Wt (mg)** | 204.4 | 190.2 | 181.9 | 172.7 | 167.2 | 157.5 | 152.7 |
| | **% Rtnd** | 100.0 | 93.1 | 89.0 | 84.5 | 81.8 | 77.1 | 74.7 |
| 0.74 ± 0.94 x 0.87 ± 0.22, (Pass 2 - Middle 1 mm) | **Wt (mg)** | 202.8 | 194.3 | 185.4 | 180.4 | 167.4 | 158.2 | 148.7 |
| | **% Rtnd** | 100.0 | 95.8 | 91.4 | 88.9 | 82.5 | 78.0 | 73.3 |
| 0.86 ± 0.09 x 0.99 ± 0.11, (Pass 2 - ≤ 1 mm) | **Wt (mg)** | 200.8 | 191.4 | 184.9 | 173.3 | 162.5 | 157.4 | 156.1 |
| | **% Rtnd** | 100.0 | 95.3 | 92.1 | 86.3 | 80.9 | 78.4 | 77.7 |

### Example 8

The processing conditions for Example 8 at the time of sampling are summarized in Table 8 below.

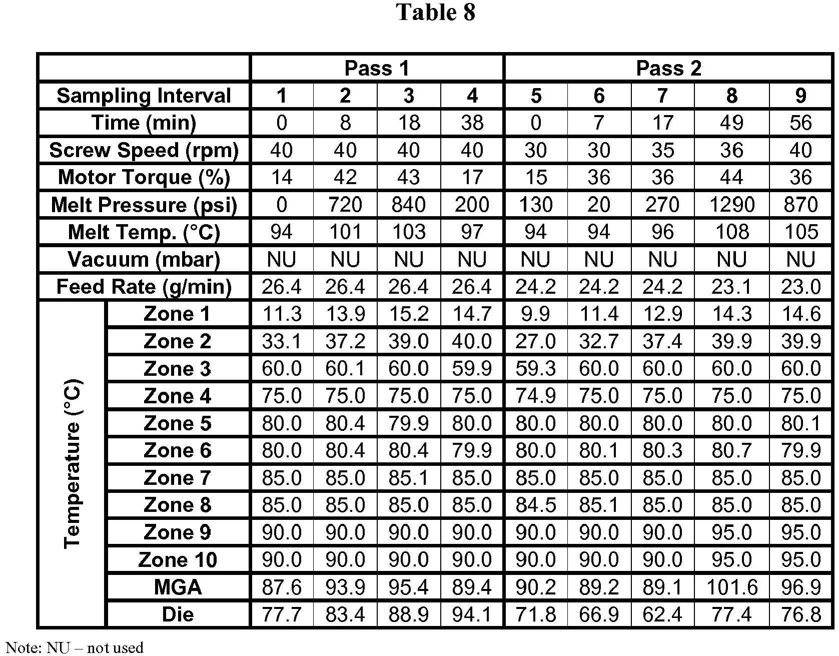

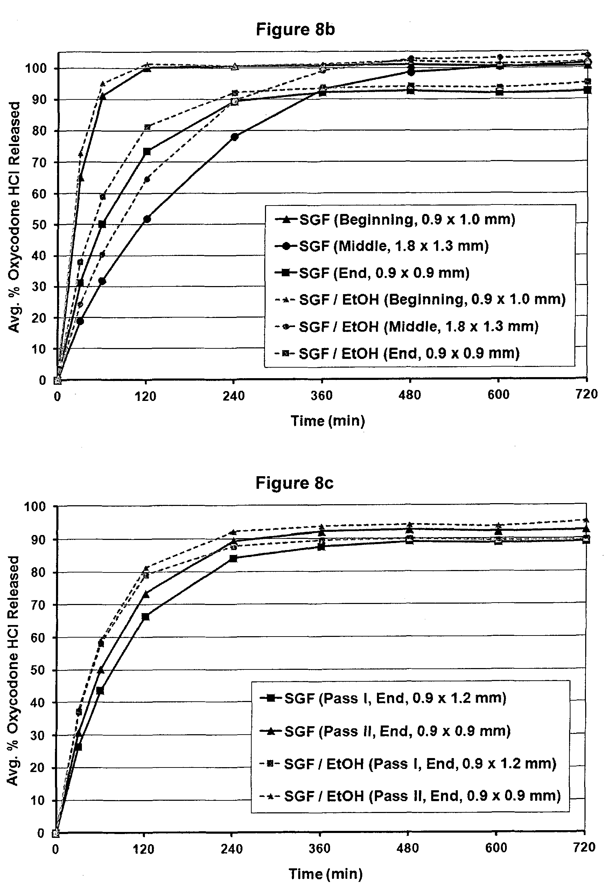

The dissolution results for Example 8 MEMs are summarized in Figures 8a to 8c and Tables 8a to 8e.

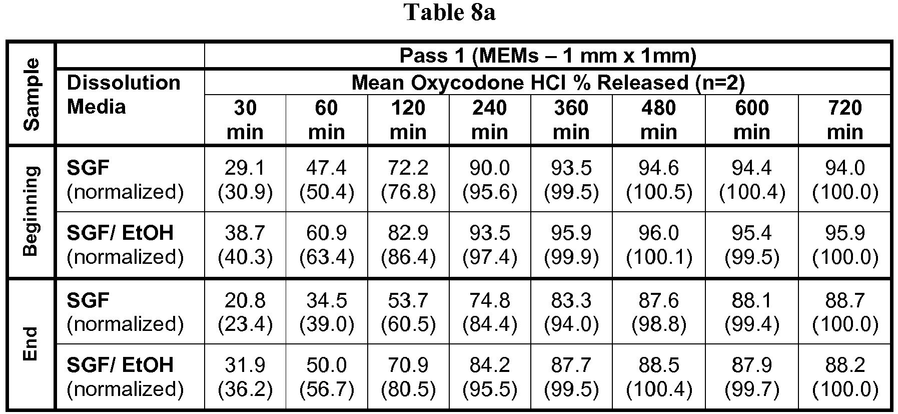

**Table 8b**

| | **Pass 1, End (MEMs - 0.86 mm x 1.21 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 26.3 (29.6) | 43.5 (48.9) | 66.5 (74.7) | 84.1 (94.5) | 87.5 (98.4) | 89.1 (100.2) | 88.8 (99.7) | 89.0 (100.0) |
| **SGF / EtOH** (normalized) | 37.1 (41.3) | 58.1 (64.8) | 79.1 (88.2) | 87.7 (97.8) | 89.4 (99.7) | 90.0 (100.3) | 89.4 (99.7) | 89.7 (100.0) |

**Table 8c**

| | **Pass 2, Beginning (MEMs - 0.94 x 0.99 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 65.3 (64.9) | 91.2 (90.7) | 100.2 (99.6) | 100.5 (99.9) | 100.7 (100.1) | 101.0 (100.4) | 100.5 (100.0) | 100.6 (100.0) |
| **SGF/EtOH** (normalized) | 72.9 (71.6) | 95.2 (93.4) | 101.3 (99.4) | 100.5 (98.6) | 101.1 (99.3) | 102.3 (100.4) | 101.4 (99.5) | 101.8 (100.0) |

**Table 8d**

| | **Pass 2, Middle (MEMs - 1.76 x 1.33 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 18.9 (18.6) | 31.7 (31.3) | 52.0 (51.2) | 78.1 (76.9) | 93.2 (91.9) | 98.6 (97.2) | 100.4 (98.9) | 101.4 (100) |
| **SGF/EtOH** (normalized) | 24.3 (23.4) | 40.4 (38.9) | 64.8 (62.4) | 89.5 (86.2) | 99.1 (95.4) | 102.9 (99.1) | 103.1 (99.3) | 103.8 (100) |

**Table 8e**

| | **Pass 2, End (MEMs -0.90 x 0.92 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 31.0 (33.5) | 50.2 (54.3) | 73.5 (79.4) | 89.3 (96.6) | 92.1 (99.5) | 92.7 (100.2) | 92.0 (99.5) | 92.5 (100.0) |
| **SGF/ EtOH** (normalized) | 37.7 (39.6) | 59.1 (62.1) | 81.3 (85.4) | 92.1 (96.7) | 93.6 (98.3) | 94.2 (98.9) | 93.7 (98.4) | 95.2 (100.0) |

The crush testing results of Example 8 are summarized in Table 8f.

**Table 8f**

| **Mean Pellet Dimension D (mm) x L (mm) (n = 19-37)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.99± 0.13 x 1.04 ± 0.12, Pass 1 - Middle | **Wt (mg)** | 203.4 | 187.4 | 175.9 | 168.8 | 164.4 | 158.6 | 154.5 |
| | **% Rtnd** | 100.0 | 92.1 | 86.5 | 83.0 | 80.8 | 78.0 | 76.0 |
| 0.86 ± 0.10 x 1.21 ± 0.44, Pass 1 - End | **Wt (mg)** | 203.0 | 192.6 | 183.1 | 169.3 | 159.3 | 158.5 | 152.1 |
| | **% Rtnd** | 100.0 | 94.9 | 90.2 | 83.4 | 78.5 | 78.1 | 74.9 |
| 1.76 ± 0.09 x 1.33 ± 0.14, Pass 2 - Middle 2mm | **Wt (mg)** | 204.1 | 186.9 | 182.8 | 176.3 | 171.1 | 165.6 | 161.3 |
| | **% Rtnd** | 100.0 | 91.6 | 89.6 | 86.4 | 83.8 | 81.1 | 79.0 |
| 0.74 ± 0.15 x 0.84 ± 0.19, Pass 2 - Middle | **Wt (mg)** | 202.4 | 193.9 | 185.1 | 178.6 | 170.5 | 163.6 | 153.5 |
| | **% Rtnd** | 100.0 | 95.8 | 91.5 | 88.3 | 84.3 | 80.9 | 75.9 |

### Example 9

The processing conditions for Example 9 at the time of sampling are summarized in Table 9 below.

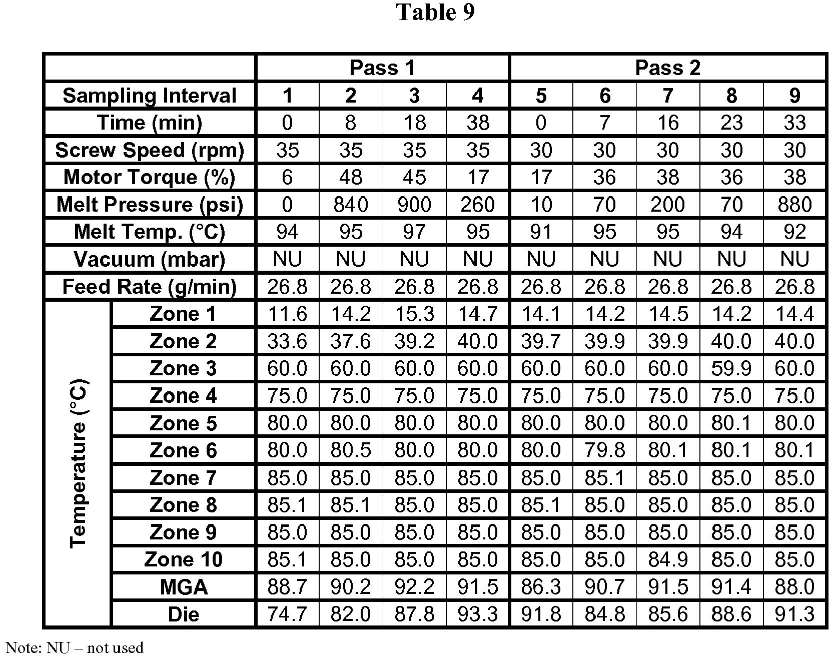

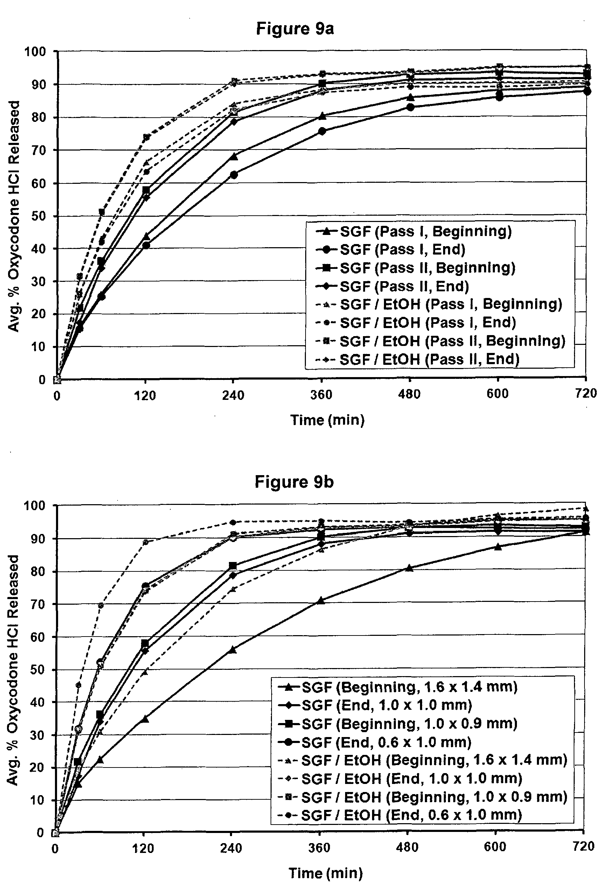

The dissolution results for Example 9 MEMs are summarized in Figure 9a and 9b and Tables 9a to 9d.

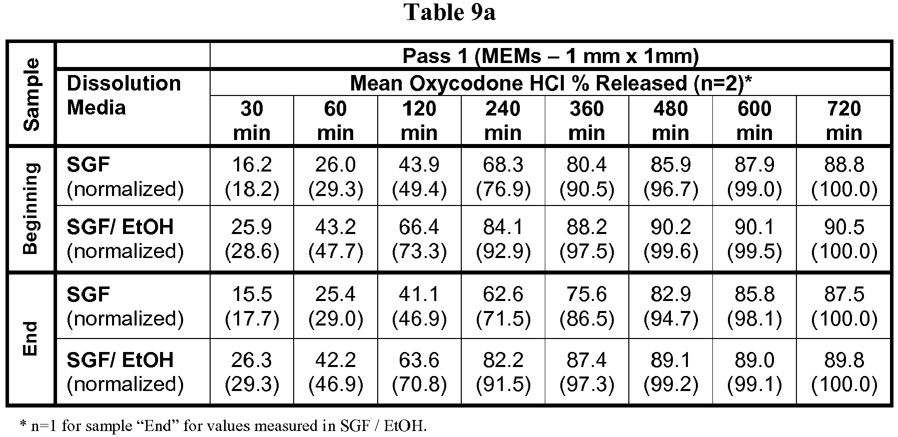

**Table 9b**

| | **Pass 2, Beginning (MEMs - 1.62 mm x 1.38 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 15.0 (16.5) | 22.5 (24.6) | 35.1 (38.5) | 56.1 (61.4) | 70.9 (77.7) | 80.6 (88.2) | 86.9 (95.1) | 91.4 (100.0) |
| **SGF/ EtOH** (normalized) | 19.2 (19.5) | 31.1 (31.6) | 49.4 (50.2) | 74.5 (75.8) | 86.3 (87.8) | 93.2 (94.9) | 96.5 (98.2) | 98.3 (100.0) |

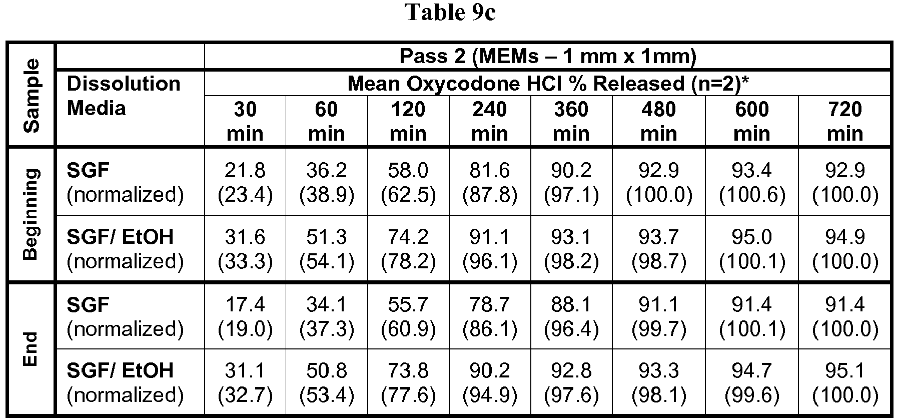

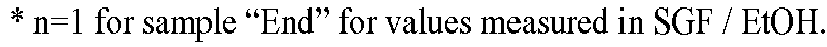

**Table 9d**

| | **Pass 2, End (MEMs - 0.62 mm x 0.97 mm)** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 32.1 (34.8) | 52.2 (56.6) | 75.6 (81.9) | 90.1 (97.7) | 92.2 (100.0) | 93.0 (100.8) | 92.5 (100.3) | 92.2 (100.0) |
| **SGF/ EtOH** (normalized) | 45.3 (47.4) | 69.7 (72.8) | 88.9 (92.9) | 94.7 (99.0) | 94.9 (99.2) | 94.4 (98.7) | 95.3 (99.6) | 95.7 (100.0) |

The crush testing results of Example 9 are summarized in Table 9e.

**Table 9e**

| **Mean Pellet Dimension D (mm) x L (mm) (n = 25-42)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.12± 0.15 x 1.04 ± 0.19, Pass 1 - Middle | **Wt (mg)** | 202.5 | 188.5 | 180.2 | 170.8 | 169.8 | 162.7 | 158.7 |
| | **% Rtnd** | 100.0 | 93.1 | 89.0 | 84.3 | 83.9 | 80.3 | 78.4 |
| 1.62 ± 0.13 x 1.38 ± 0.14, Pass 2 - Middle 2mm | **Wt (mg)** | 203.9 | 184.5 | 176.3 | 171.7 | 161.1 | 158.5 | 151.6 |
| | **% Rtnd** | 100.0 | 90.5 | 86.5 | 84.2 | 79.0 | 77.7 | 74.4 |
| 1.01 ± 0.10 x 0.97 ± 0.12, Pass 2 - Middle | **Wt (mg)** | 201.7 | 195.1 | 184.3 | 177.1 | 168.5 | 164.4 | 152.7 |
| | **% Rtnd** | 100.0 | 96.7 | 91.4 | 87.8 | 83.5 | 81.5 | 75.7 |
| 0.62 ± 0.11 x 0.97 ± 0.22, Pass 2 - End ≤ 1 mm | **Wt (mg)** | 201.3 | 192.3 | 191.0 | 178.1 | 172.1 | 165.1 | 161.2 |
| | **% Rtnd** | 100.0 | 95.5 | 94.9 | 88.5 | 85.5 | 82.0 | 80.1 |

### EXAMPLEs 10 - 12

### Composition

The compositions of the poly(ε-caprolactone) melt-extruded multi-particulates (MEMs) for Examples 10 - 12 are summarized in Table VII below:

**Table VII**

| **Example** | **10** | | | **11** | | | **12** | | |
|---|---|---|---|---|---|---|---|---|---|
| **Ingredient (Trade Name)** | **Amount** | | | **Amount** | | | **Amount** | | |
| | **(% w/w)** | **unit (mg)** | **batch (g)** | **w/w)** | **unit (mg)** | **batch (g)** | **(% w/w)** | **unit (mg)** | **batch (g)** |
| Oxycodone HCl* | **15.0** | 30.0 | 90.0 | **15.0** | 30.0 | 90.0 | **15.0** | 30.0 | 90.0 |
| Poly(ε-caprolactone), **Mn ∼ 98,000** (PC-12) | **69.0** | 138.0 | 414.0 | - | - | - | - | - | - |
| Poly(ε-caprolactone), **Mn ∼** 70,000 - **90,000^{‡}** | - | - | - | **69.0** | 138.0 | 414.0 | - | - | - |
| Poly(ε-caprolactone), **Mn ∼ 45,000^{‡}** | - | - | - | - | - | - | **69.0** | 138.0 | 414.0 |
| Polyethylene oxide, Mw ∼ 100,000 (PEO WSR N10) | **15.0** | 30.0 | 90.0 | **15.0** | 30.0 | 90.0 | **15.0** | 30.0 | 90.0 |
| Butylated Hydroxy Toluene (BHT) | **1.0** | | 6.0 | **1.0** | 2.0 | 6.0 | **1.0** | 2.0 | 6.0 |
| Total | **100** | 200 | 600 | **100** | 200 | 600 | **100** | 200 | 600 |

| | | | | | | | | | |
|---|---|---|---|---|---|---|---|---|---|
| * Amount not corrected for water or impurities. ‡no trade name available; purchased from Sigma Aldrich. | | | | | | | | | |

### Manufacturing Procedure

1. Blending: Oxycodone HCl, poly(ε-caprolactone) (material obtained from the manufacturer was cryo-milled, and milled material ≤ 840 µm used), polyethylene oxide and milled BHT were loaded into an HDPE bottle of appropriate size and blended in Turbula™ mixer for 5 minutes at medium speed at ambient temperature.
2. Feeding into Extruder: Materials blended in Step 1 were added to a single-screw volumetric dispenser (AccuRate™) and its feed rate was calibrated to 24 ± 0.5 g/min.
3. Melt Extrusion: The blend was metered into 27-Micro GGC twin screw extruder with 10 heating zones, fitted with a main gated adapter and a multi-orifice coat-hanger type die and processed into strands.
4. Cooling: The strands from step 3 were drawn on an 8-ft conveyer belt fitted with 2 air knives and cooled at ambient temperature.
5. Pelletizing: The cooled strands from step 4 were cut into pellets of dimensions 1 mm x 1mm (Pelletizer Settings: Nip Roll (Hz) - 8.0; Cutter Roll (Hz) - 15.3) and 2 mm x 2 mm (Pelletizer Settings: Nip Roll (Hz) - 8.0; Cutter Roll (Hz) - 9.05)

The co-rotating screw configuration for Examples 10 - 12 is given in Table VIII.

**Table VIII**

| Quantity | Screw Element Type |
|---|---|
| FEED END | |
| 2 | GFA 2-40-90 |
| 1 | GFA 2-30-90 |
| 2 | GFA 2-30-60 |
| 2 | GFA 2-30-90 |
| 1 | GFA 2-20-90 |
| 1 | KB5 2-30-30 |
| 1 | KB5 2-30-60 |
| 1 | KB5 2-30-90 |
| 1 | KS1 2-10A |
| 1 | KS1 2-10E (90°) |
| 1 | GFA 2-40-90 |
| 2 | GFA 2-30-30 |
| 1 | GFA 2-30-90 |
| 1 | GFA 2-20-90 |
| | HEXPLUG |

### Example 10

The processing conditions for Example 10 at the time of sampling are summarized in Table 10 below.

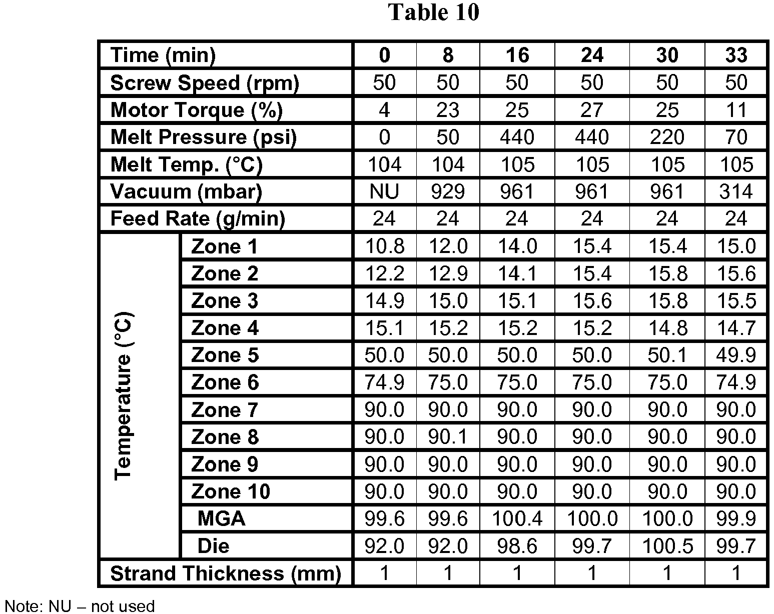

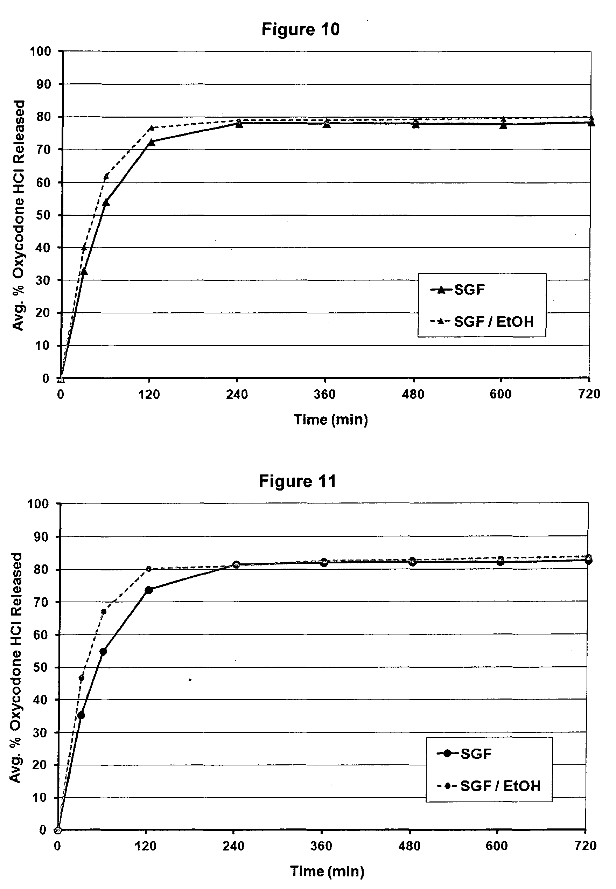

The dissolution results for Example 10 MEMs are summarized in Figure 10 and Table 10a.

**Table 10a**

| | **MEMs - 1 mm x 1 mm; Sampling time ∼ 10 minutes** | | | | | | | |
|---|---|---|---|---|---|---|---|---|
| **Dissolution Media** | **Mean Oxycodone HCL % Released (n=2)** | | | | | | | |
| | **30 min** | **60 min** | **120 min** | **240 min** | **360 min** | **480 min** | **600 min** | **720 min** |
| **SGF** (normalized) | 32.9 (42.0) | 54.2 (69.0) | 72.5 (92.4) | 78.0 (99.4) | 78.0 (99.5) | 78.0 (99.5) | 77.8 (99.2) | 78.5 (100.0) |
| **SGF/ EtOH** (normalized) | 40.3 (50.3) | 62.1 (77.5) | 76.8 (95.9) | 78.9 (98.6) | 79.0 (98.5) | 79.3 (98.5) | 79.7 (98.9) | 80.1 (100.0) |

The crush testing results of Example 10 are summarized in Table 10b.

**Table 10b**

| **Mean Pellet Dimension D (mm) x L (mm) (n = 17-42)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.95 ± 0.07 x 1.02 ± 0.09 | **Wt (mg)** | 210.5 | 206.3 | 206.1 | 200.6 | 201.1 | 196.2 | 199.0 |
| | **% Rtnd** | 100.0 | 98.0 | 97.9 | 95.3 | 95.5 | 93.2 | 94.5 |
| 1.13 ± 0.12 x 1.29 ± 0.18 | **Wt (mg)** | 208.6 | 200.4 | 191.7 | 187.5 | 177.2 | 166.1 | 158.2 |
| | **% Rtnd** | 100.0 | 96.1 | 91.9 | 89.9 | 85.0 | 79.6 | 75.8 |
| 0.97 ± 0.07 x 1.10 ± 0.08 | **Wt (mg)** | 205.1 | 196. | 190.3 | 182.0 | 174.6 | 166.1 | 158.9 |
| | **% Rtnd** | 100.0 | 95.9 | 92.8 | 88.7 | 85.1 | 81.0 | 77.5 |
| 1.34 ± 0.08 x 1.20 ± 0.23 | **Wt (mg)** | 203.7 | 200.1 | 190.5 | 184.1 | 177.7 | 168.6 | 166.1 |
| | **% Rtnd** | 100.0 | 98.2 | 93.5 | 90.4 | 87.2 | 82.8 | 81.5 |

### Example 11

The processing conditions for Example 11 at the time of sampling are summarized in Table 11 below.

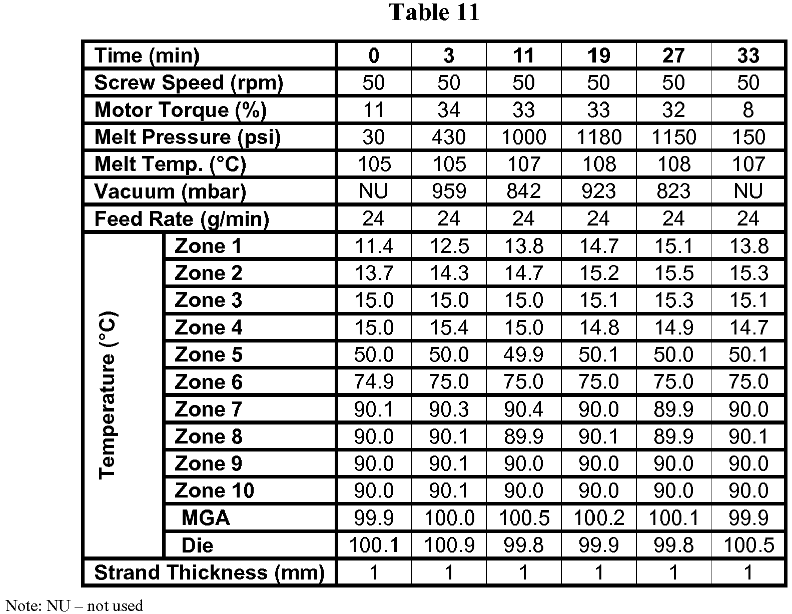

The dissolution results for Example 11 MEMs are summarized in Figure 11 and Table 11a.

**Table 11a**

| | **MEMs - 1 mm x 1 mm; Sampling time ∼ 20 minutes** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 35.5 (43.0) | 54.9 (66.5) | 73.8 (89.4) | 81.5 (98.7) | 82.0 (99.3) | 82.2 (99.5) | 82.1 (99.4) | 82.6 (100.0) |
| **SGF/ EtOH** (normalized) | 46.8 (55.9) | 67.2 (80.2) | 80.2 (95.8) | 81.2 (96.9) | 82.6 (98.7) | 82.9 (99.0) | 83.3 (99.5) | 83.8 (100.0) |

The crush testing results of Example 11 are summarized in Table 11b.

**Table 11b**

| **Mean Pellet Dimension D (mm) x L (mm) (n = 14-42)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 0.90 ± 0.21 x 0.95 ± 0.17 | **Wt (mg)** | 203.1 | 192.2 | 190.2 | 185.1 | 181.0 | 181.0 | 174.3 |
| | **% Rtnd** | 100.0 | 94.6 | 93.6 | 91.1 | 89.1 | 89.1 | 85.8 |
| 1.02 ± 0.10 x 1.13 ± 0.09 | **Wt (mg)** | 204.4 | 199.5 | 195.6 | 194.3 | 191.2 | 187.1 | 182.7 |
| | **% Rtnd** | 100.0 | 97.6 | 95.7 | 95.1 | 93.5 | 91.5 | 89.4 |
| 1.02 ± 0.08 x 0.98 ± 0.12 | **Wt (mg)** | 202.2 | 200.6 | 199.6 | 196.6 | 194.4 | 191.5 | 190.0 |
| | **% Rtnd** | 100.0 | 99.2 | 98.7 | 97.2 | 96.2 | 94.7 | 94.0 |
| 1.32 ± 0.11 x 1.12 ± 0.17 | **Wt (mg)** | 201.3 | 197.4 | 195.2 | 193.6 | 192.8 | 191.6 | 189.3 |
| | **% Rtnd** | 100.0 | 98.1 | 97.0 | 96.2 | 95.8 | 95.2 | 94.0 |
| 0.92 ± 0.09 x 1.12 ± 0.09 | **Wt (mg)** | 204.8 | 201.1 | 196.8 | 193.7 | 191.4 | 188.7 | 185.6 |
| | **% Rtnd** | 100.0 | 98.2 | 96.1 | 94.6 | 93.5 | 92.1 | 90.6 |
| 0.86 ± 0.10 x 1.08 ± 0.19 | **Wt (mg)** | 205.5 | 202.7 | 201.1 | 197.6 | 195.0 | 194.2 | 190.6 |
| | **% Rtnd** | 100.0 | 98.6 | 97.8 | 96.1 | 94.9 | 94.5 | 92.7 |

### Example 12

The processing conditions for Example 12 at the time of sampling are summarized in Table 12 below.

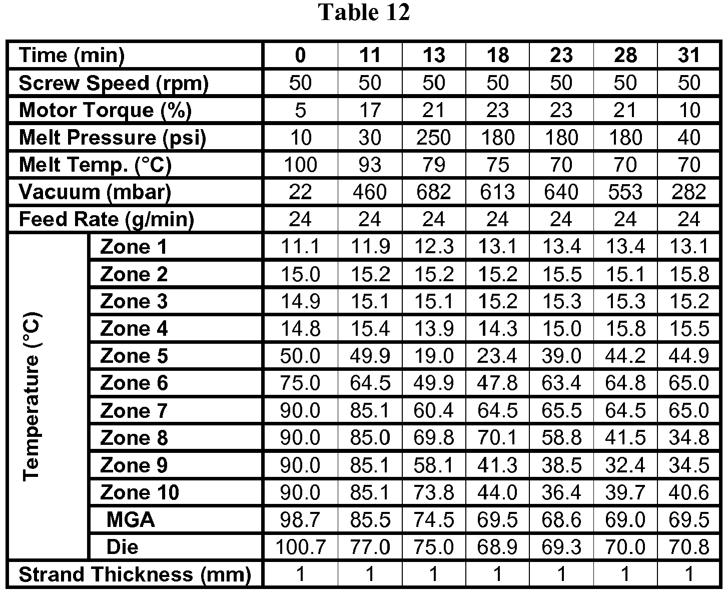

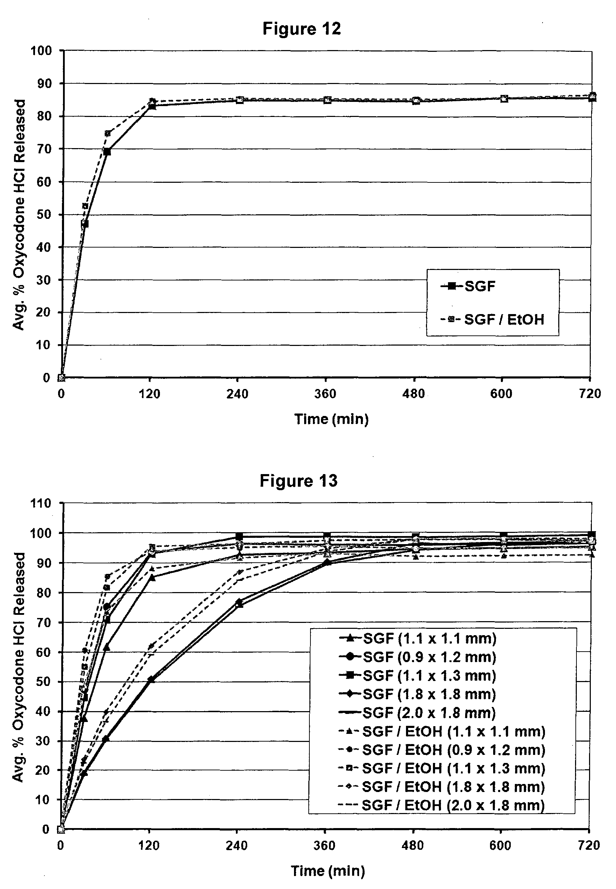

The dissolution results for Example 12 MEMs are summarized in Figure 12 and Table 12a.

**Table 12a**

| | **MEMs - 1 mm x 1 mm; Sampling time ∼ 27 minutes** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 47.3 (55.2) | 69.3 (80.9) | 83.3 (97.2) | 84.9 (99.1) | 85.0 (99.2) | 84.8 (99.0) | 85.6 (99.9) | 85.7 (100.0) |
| **SGF/ EtOH** (normalized) | 52.7 (60.9) | 74.7 (86.4) | 84.8 (97.8) | 85.4 (98.6) | 85.4 (98.5) | 85.3 (98.5) | 86.7 (98.9) | 86.6 (100.0) |

The crush testing results of Example 12 are summarized in Table 12b.

**Table 12b**

| **Mean Pellet Dimension D (mm) x L (mm) (n = 26-42)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.23 ± 0.16 x 1.15 ± 0.19 | **Wt (mg)** | 211.3 | 196.2 | 157.1 | 132.5 | 128.0 | 105.2 | 91.2 |
| | **% Rtnd** | 100.0 | 92.9 | 74.4 | 62.7 | 60.6 | 49.8 | 43.2 |
| 0.81 ± 0.07 x 1.04 ± 0.12 | **Wt (mg)** | 207.3 | 181.6 | 152.2 | 137.3 | 121.5 | 110.8 | 102.4 |
| | **% Rtnd** | 100.0 | 87.6 | 73.4 | 66.2 | 58.6 | 53.5 | 49.4 |
| 0.88 ± 0.06 x 1.02 ± 0.09 | **Wt (mg)** | 210.9 | 184.2 | 156.0 | 134.4 | 124.2 | 111.6 | 103.0 |
| | **% Rtnd** | 100.0 | 87.4 | 74.0 | 63.7 | 58.9 | 52.9 | 48.8 |
| 0.87 ± 0.07 x 1.08 ± 0.17 | **Wt (mg)** | 209.4 | 189.9 | 150.1 | 137.2 | 125.2 | 121.6 | 102.5 |
| | **% Rtnd** | 100.0 | 90.7 | 71.7 | 65.5 | 59.8 | 58.1 | 48.9 |
| 1.00 ± 0.09 x 1.01 ± 0.08 | **Wt (mg)** | 208.4 | 177.6 | 162.1 | 155.3 | 145.2 | 124.2 | 117.9 |
| | **% Rtnd** | 100.0 | 85.2 | 77.8 | 74.5 | 69.7 | 59.6 | 56.6 |
| 1.03 ± 0.07 x 1.05 ± 0.15 | **Wt (mg)** | 206.5 | 170.6 | 147.4 | 134.3 | 120.1 | 104.8 | 95.7 |
| | **% Rtnd** | 100.0 | 82.6 | 71.4 | 65.0 | 58.2 | 50.7 | 46.3 |
| 0.94 ± 0.2 x 1.06 ± 0.11 | **Wt (mg)** | 208.9 | - | 152.8 | - | 134.5 | - | 107.4 |
| | **% Rtnd** | 100.0 | - | 73.2 | - | 64.4 | - | 51.4 |
| 0.69 ± 0.09 x 1.10 ± 0.27 | **Wt (mg)** | 215.4 | - | 172.0 | - | 140.2 | - | 112.9 |
| | **% Rtnd** | 100.0 | - | 79.9 | - | 65.1 | - | 52.4 |
| 1.13 ± 0.14 x 1.06 ± 0.28 | **Wt (mg)** | 205.9 | - | 163.1 | - | 140.2 | - | 112.8 |
| | **% Rtnd** | 100.0 | - | 79.2 | - | 66.1 | | 54.8 |

### EXAMPLEs 13 - 18

### Composition

The compositions of the poly(ε-caprolactone) melt-extruded multi-particulates (MEMs) for Example 13 -18 are summarized in Tables XI and XII below:

**Table XI**

| **Example** | **13** | | | **14** | | | **15** | | |
|---|---|---|---|---|---|---|---|---|---|
| **Ingredient (Trade Name)** | **Amount** | | | **Amount** | | | **Amount** | | |
| | **(% w/w)** | **unit (mg)** | **batch (g)** | **(% w/w)** | **unit (mg)** | **batch (g)** | **(% w/w)** | **unit (mg)** | **batch (g)** |
| Oxycodone HCl* | **15.0** | 30.0 | 300.0 | **15.0** | 30.0 | 300.0 | **15.0** | 30.0 | 300.0 |
| Poly(ε-caprolactone), **Mn ∼ 78,000^{◊}** | **69.0** | 138.0 | 1380.0 | - | - | - | - | - | - |
| Poly(ε-caprolactone), **Mn ∼ 107,000**^{◊} | - | - | - | **69.0** | 138.0 | 1380.0 | - | - | - |
| Poly(ε-caprolactone), **Mn ∼ 70,000 - 90, 000^{‡}** | - | - | - | - | - | - | **69.0** | 138.0 | 1380.0 |
| Polyethylene oxide, Mw ∼ 100,000 (PEO WSR N10) | **15.0** | 30.0 | 300.0 | **15.0** | 30.0 | 90.0 | **15.0** | 30.0 | 300.0 |
| Butylated Hydroxy Toluene (BHT) | 1.0 | 2.0 | 20.0 | 1.0 | 2.0 | 20.0 | **1.0** | 2.0 | 20.0 |
| Total | **100** | 200 | 2000 | **100** | 200 | 2000 | **100** | 200 | 2000 |

| | | | | | | | | | |
|---|---|---|---|---|---|---|---|---|---|
| * Amount not corrected for water or impurities. ◊ no trade name available; purchased from Purac Biomaterials. ‡no trade name available; purchased from Sigma Aldrich. | | | | | | | | | |

**Table XII**

| **Example** | **16** | | | **17** | | | **18** | | |
|---|---|---|---|---|---|---|---|---|---|
| **Ingredient (Trade Name)** | **Amount** | | | **Amount** | | | **Amount** | | |
| | **(% w/w)** | **unit (mg)** | **batch (g)** | **(% w/w)** | **unit (mg)** | **batch (g)** | **(% w/w)** | **unit (mg)** | **batch (g)** |
| Oxycodone HCl* | **15.0** | 30.0 | 195.0 | **12.86** | 25.7 | 128.6 | **15.0** | 30.0 | 45.0 |
| Poly(ε-caprolactone), **Mn ∼ 154,000** | **70.0** | 140.0 | 910.0 | **70.00** | 140.0 | 700.0 | **65.0** | 130.0 | 195.0 |
| Polyethylene oxide, Mw ∼ 100,000 (PEO WSR N10) | **15.0** | 30.0 | 195.0 | **17.14** | 34.3 | 171.4 | **20.0** | 40.0 | 60.0 |
| Total | **100** | 200 | 1300 | **100** | 200 | 1000 | **100** | 200 | 300 |

| | | | | | | | | | |
|---|---|---|---|---|---|---|---|---|---|
| * Amount not corrected for water or impurities. | | | | | | | | | |

### Manufacturing Procedure

1. Blending: Oxycodone HCl and polyethylene oxide were weighed, screened and were loaded into the chamber of an 8-qt v Blender, and blended for 10 minutes with I-bar on.
2. Feeding into Extruder: The powder blend was transferred to the hopper of AccuRate™ volumetric dispenser fitted with narrow auger single-screw assembly and set atop Barrel 1. The required amount of PCL flakes (used as obtained from manufacturer) was transferred to the hopper of the other AccuRate™ volumetric dispenser fitted with larger screw assembly and set atop Barrel 1.
3. The feed rate of the two Accurate™ dispensers was calibrated according to the relative proportion of the 2 components in the formulation to obtain a total feed rate of 25 g/min. For Examples 13-15, the target feed rate for ex-PCL blend = 0.31 x 25 g/min = 7.75 g/min, where 0.31 is the proportion of OXY + PEO + BHT in the formulation which is 31 % w/w, target feed rate for PCL = 0.69 x 25 g/min = 17.25 g/min, where 0.69 is the proportion of PCL in the formulation which is 69% w/w, and 25 g/min is the total feed rate. For Examples 16 and 17, the target feed rate for ex-PCL blend = 0.30 x 25 g/min = 7.50 g/min, where 0.30 is the proportion of OXY + PEO in the formulation, and target feed rate for PCL = 0.70 x 25 g/min = 17.50 g/min, where 0.70 is the proportion of PCL in the formulation. For Example 18, the ex-PCL blend fraction was 0.35 (Oxy - 0.15 and PEO - 0.2), with target feed rate of 0.35 x 25 g/min = 8.75 g/min, and target feed rate for PCL = 0.65 x 25 g/min = 16.25 g/min.
4. Melt Extrusion: The materials were metered into 27-Micro GGC twin screw extruder with 10 heating zones, fitted with a main gated adapter and a multi-orifice coat-hanger type die and processed into strands.
5. Cooling: The strands from step 4 were drawn on an 8-ft conveyer belt fitted with 2 air knives and cooled at ambient temperature.
6. Pelletizing: The cooled strands were cut into pellets of dimensions 1 mm x 1mm (Pelletizer Settings: Nip Roll (Hz) - 8.0; Cutter Roll (Hz) - 15.3) and 2 mm x 2 mm (Pelletizer Settings: Nip Roll (Hz) - 8.0; Cutter Roll (Hz) - 9.05)

The co-rotating screw configuration for Examples 13-18 is given in Table XIII.

**Table XIII**

| **Quantity** | **Screw Element Type** |
|---|---|
| FEED END | |
| 1 | GFA 2-40-90 |
| 1 | GFA 2-30-90 |
| 1 | GFA 2-20-90 |
| 2 | KB5 2-30-30 |
| 1 | GFA 2-30-60 |
| 2 | KB5 2-30-30 |
| 2 | KB5 2-30-60 |
| 1 | GFA 2-30-30 |
| 1 | KB5 2-30-60 |
| 1 | KB5 2-30-90 |
| 1 | KS1 2-10A |
| 1 | KS1 2-10E (90°) |
| 1 | GFA 2-30-90 |
| 1 | KB5 2-30-60 |
| 2 | KB5 2-30-90 |
| 1 | GFA 2-40-90 |
| 1 | GFA 2-30-90 |
| 1 | GFA 2-30-30 |
| 1 | GFA 2-20-90 |
| | HEXPLUG |

### Example 13

The processing conditions for Example 13 at the time of sampling are summarized in Table 13 below.

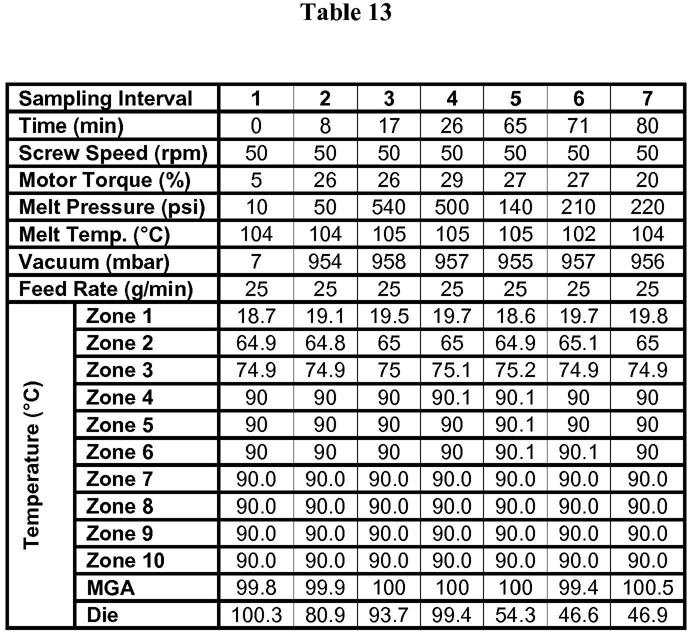

The dissolution results for Example 13 MEMs are summarized in Figure 13 and Tables 13a-13e.

**Table 13a**

| | **MEMs - 1.06 mm ± 0.09 x 1.10 mm ± 0.12; Sampling time - 10 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 37.9 (39.8) | 62.0 (65.1) | 85.3 (89.5) | 92.8 (97.4) | 93.3 (97.9) | 94.6 (99.3) | 94.9 (99.6) | 95.3 (100.0) |
| **SGF/ EtOH** (normalized) | 48.8 (52.7) | 73.7 (79.6) | 88.2 (95.3) | 91.8 (99.1) | 93.1 (100.6) | 92.1 (99.4) | 92.4 (99.8) | 92.6 (100.0) |

**Table 13b**

| | **MEMs: 0.87 mm ± 0.06 x 1.15 mm ± 0.13; Sampling time - 20 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 47.3 (48.8) | 75.3 (77.7) | 93.7 (96.7) | 96.5 (99.6) | 96.1 (99.2) | 96.0 (99.0) | 96.7 (99.8) | 96.9 (100.0) |
| **SGF/ EtOH** (normalized) | 60.7 (63.9) | 85.6 (90.1) | 93.9 (98.9) | 95.2 (100.3) | 95.6 (100.6) | 94.8 (99.8) | 94.9 (99.9) | 95.0 (100.0) |

**Table 13c**

| | **MEMs: 1.09 mm ± 0.14 x 1.27 mm ± 0.18; Sampling time - 35 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 44.6 (45.0) | 70.9 (71.4) | 93.1 (93.8) | 98.9 (99.7) | 98.9 (99.7) | 98.6 (99.3) | 98.7 (99.5) | 99.2 (100.0) |
| **SGF/ EtOH** (normalized) | 55.0 (56.4) | 81.7 (83.9) | 95.6 (98.2) | 96.3 (98.9) | 97.7 (100.3) | 97.7 (100.3) | 97.7 (100.3) | 97.4 (100.0) |

**Table 13d**

| | **MEMs: 1.83 mm ± 0.11 x 1.84 mm ± 0.15; Sampling time - 72 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 19.0 (19.7) | 30.9 (32.0) | 50.9 (52.7) | 77.0 (79.8) | 90.3 (93.5) | 96.4 (99.9) | 95.8 (99.3) | 96.5 (100.0) |
| **SGF/ EtOH** (normalized) | 23.8 (24.3) | 39.9 (40.8) | 62.1 (63.4) | 87.1 (88.9) | 94.8 (96.7) | 98.1 (100.1) | 98.1 (100.1) | 98.0 (100.0) |

**Table 13e**

| | **MEMs: 2.03 mm ± 0.16 x 1.82 mm ± 0.18; Sampling time - 80 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 18.5 (19.0) | 30.2 (31.0) | 50.0 (51.3) | 75.4 (77.3) | 89.6 (91.9) | 94.2 (96.6) | 96.2 (98.7) | 97.5 (100.0) |
| **SGF/ EtOH** (normalized) | 22.7 (23.5) | 36.7 (38.0) | 59.2 (61.3) | 84.2 (87.2) | 93.9 (97.2) | 98.0 (101.4) | 98.0 (101.4) | 96.6 (100.0) |

The crush testing results of Example 13 are summarized in Table 13f.

**Table 13f**

| **Mean Pellet Dimension D (mm) x L (mm) (n = 16-76)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.06 ± 0.09 x 1.10 ± 0.12 | **Wt (mg)** | 211.1 | 189.3 | 181.1 | 168.8 | 157.9 | 148.5 | 139.5 |
| | **% Rtnd** | 100.0 | 89.7 | 85.8 | 80.0 | 74.8 | 70.3 | 66.1 |
| 0.87 ± 0.06 x 1.15 ± 0.13 | **Wt (mg)** | 202.9 | 190.2 | 176.5 | 166.0 | 154.5 | 146.5 | 138.8 |
| | **% Rtnd** | 100.0 | 93.7 | 87.0 | 81.8 | 76.1 | 72.2 | 68.4 |
| 1.09 ± 0.14 x 1.27 ± 0.18 | **Wt (mg)** | 203.4 | 187.8 | 175.7 | 163.4 | 157.6 | 148.3 | 140.5 |
| | **% Rtnd** | 100.0 | 92.3 | 86.4 | 80.3 | 77.5 | 72.9 | 69.1 |
| 1.83 ± 0.11 x 1.84 ± 0.15 | **Wt (mg)** | 208.0 | 188.8 | 179.6 | 171.3 | 161.7 | 152.4 | 141.6 |
| | **% Rtnd** | 100.0 | 90.8 | 86.3 | 82.4 | 77.7 | 73.3 | 68.1 |
| 2.03 ± 0.16 x 1.82 ± 0.18 | **Wt (mg)** | 201.8 | 176.2 | 164.6 | 156.3 | 145.3 | 138.7 | 122.9 |
| | **% Rtnd** | 100.0 | 87.3 | 81.6 | 77.5 | 72.0 | 68.7 | 60.9 |

### Example 14

The processing conditions for Example 14 at the time of sampling are summarized in Table 14 below.

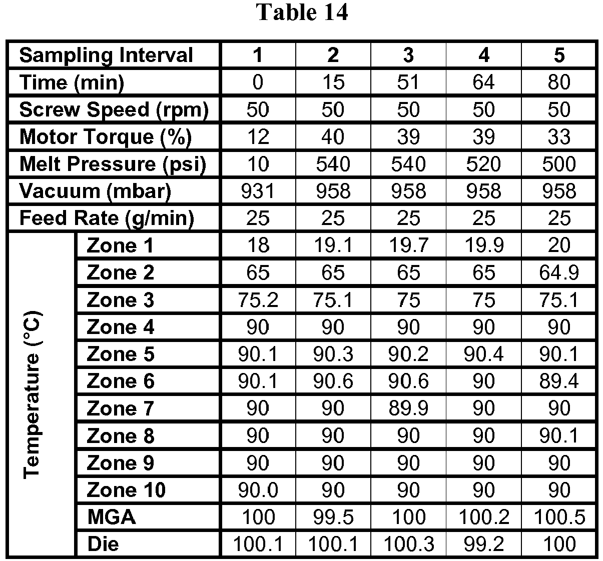

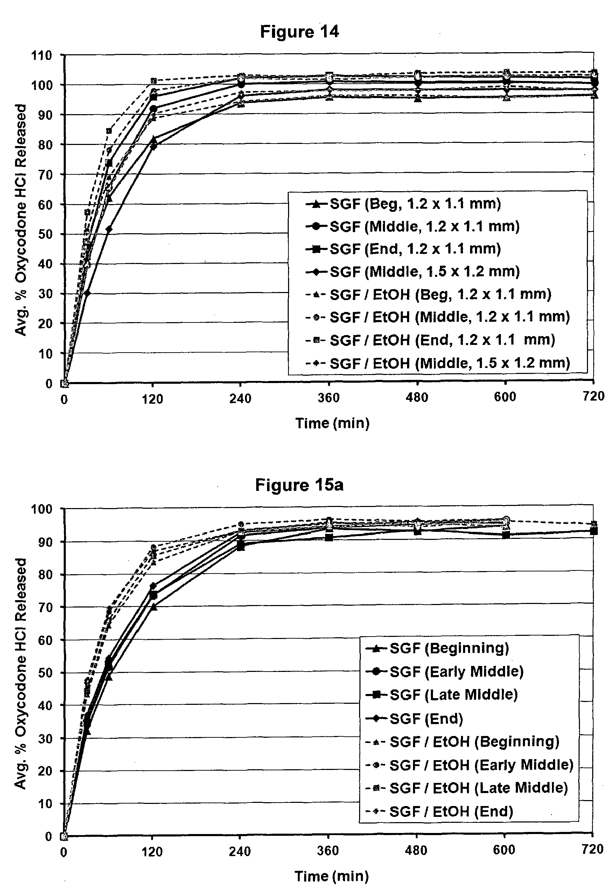

The dissolution results for Example 14 MEMs are summarized in Figure 14 and Tables 14a-14b.

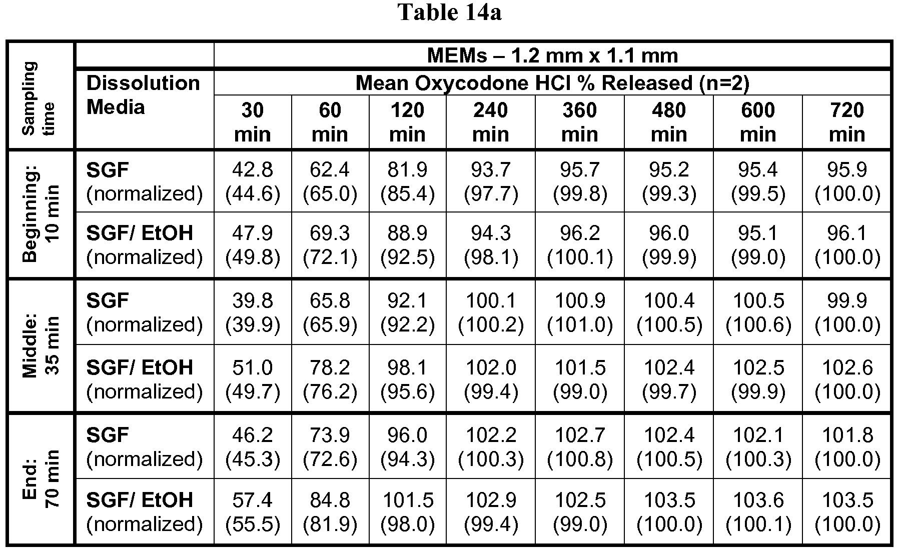

**Table 14b**

| | **MEMs - 1.46 mm ± 0.05 x 1.15 mm ± 0.17; Sampling time - Middle: 50 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 30.3 (31.0) | 51.6 (52.8) | 79.3 (81.0) | 96.2 (98.3) | 98.2 (100.4) | 98.0 (100.1) | 97.9 (100.1) | 97.8 (100.0) |
| **SGF/ EtOH** (normalized) | 39.4 (40.4) | 65.2 (66.8) | 90.2 (92.5) | 97.3 (99.7) | 97.9 (100.4) | 97.8 (100.2) | 98.9 (101.4) | 97.5 (100.0) |

The crush testing results of Example 14 are summarized in Table 14c.

**Table 14c**

| **Mean Pellet Dimension D (mm) x L (mm) (n = 24-45)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.22 ± 0.11 x 1.06 ± 0.13 | **Wt (mg)** | 206.3 | 190.9 | 183.0 | 179.4 | 173.0 | 166.0 | 162.8 |
| | **% Rtnd** | 100.0 | 92.5 | 88.7 | 87.0 | 83.9 | 80.5 | 78.9 |
| 1.14 ± 0.09 x 1.12 ± 0.14 | **Wt (mg)** | 207 | 189.4 | 181.1 | 178.0 | 173.8 | 165.3 | 162.0 |
| | **% Rtnd** | 100.0 | 91.5 | 87.5 | 86.0 | 84.0 | 79.9 | 78.3 |
| 1.04 ± 0.14 x 1.10 ± 0.10 | **Wt (mg)** | 200.8 | 193.5 | 183.0 | 178.5 | 175.9 | 173.0 | 166.7 |
| | **% Rtnd** | 100.0 | 96.4 | 91.1 | 88.9 | 87.6 | 86.2 | 83.0 |
| 1.46 ± 0.05 x 1.15 ± 0.17 | **Wt (mg)** | 204.1 | 194.4 | 193.3 | 182.4 | 175.8 | 175.6 | 168.9 |
| | **% Rtnd** | 100.0 | 95.2 | 94.7 | 89.4 | 86.1 | 86.0 | 82.8 |

### Example 15

The processing conditions for Example 15 at the time of sampling are summarized in Table 15 below.

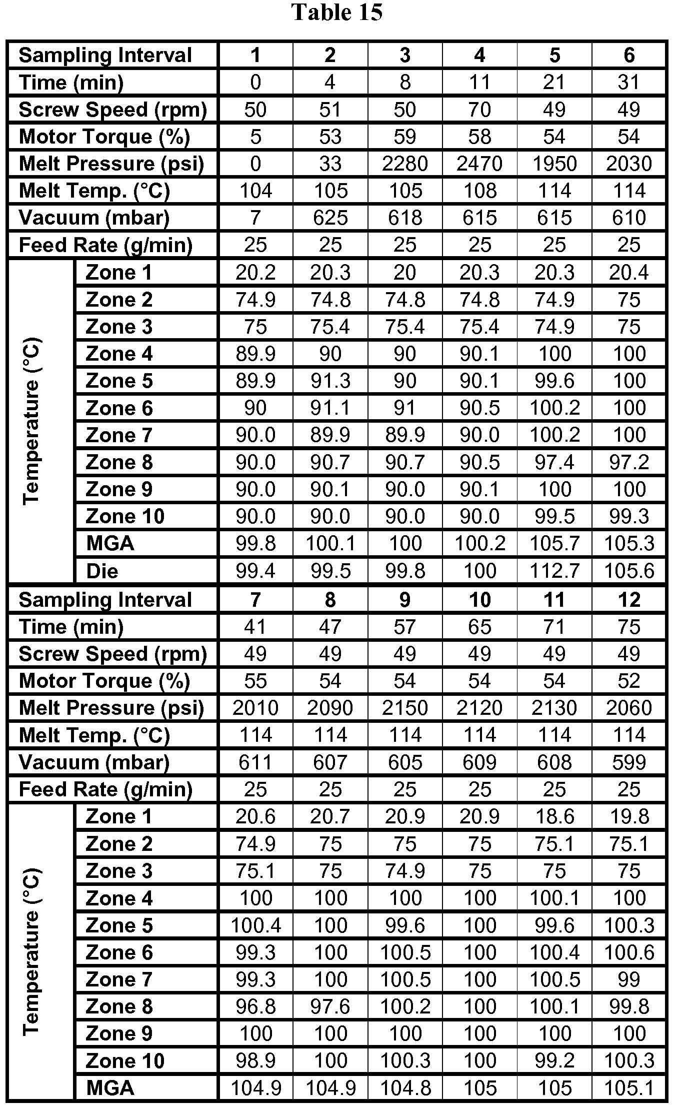

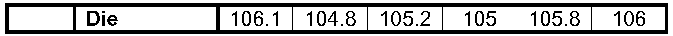

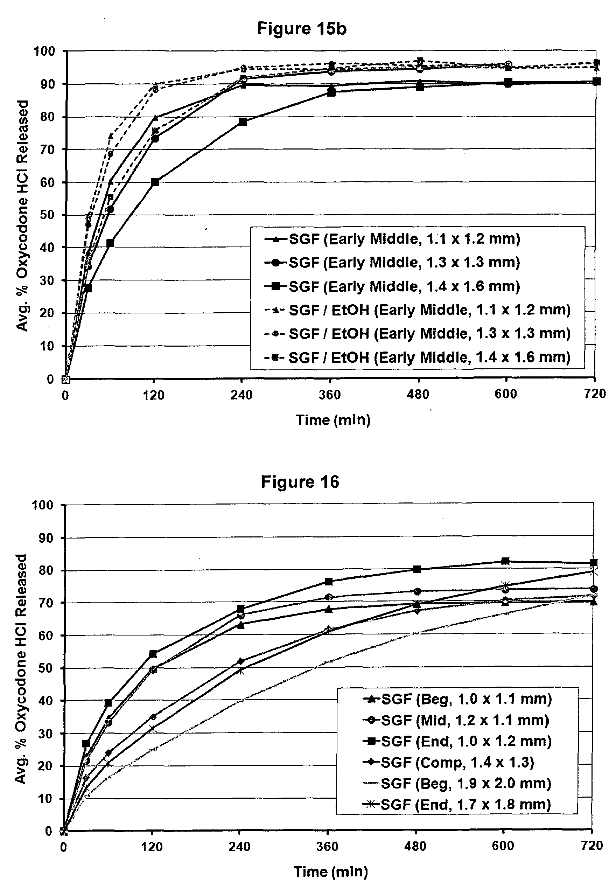

The dissolution results for Example 15 MEMs are summarized in Figures 15a and 15b and Tables 15a-15c.

**Table 15a**

| | **MEMs - 1.11 mm ± 0.14 x 1.15 mm ± 0.11); Sampling time - Early Middle: 15 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 38.5 (42.5) | 60.3 (66.7) | 79.9 (88.2) | 89.6 (99.0) | 89.5 (98.9) | 90.9 (100.4) | 89.7 (99.2) | 90.5 (100.0) |
| **SGF/ EtOH** (normalized) | 49.6 (52.2) | 74.1 (78.1) | 90.0 (94.8) | 94.5 (99.6) | 94.5 (99.5) | 95.2 (100.2) | 94.8 (99.8) | 95.0 (100.0) |

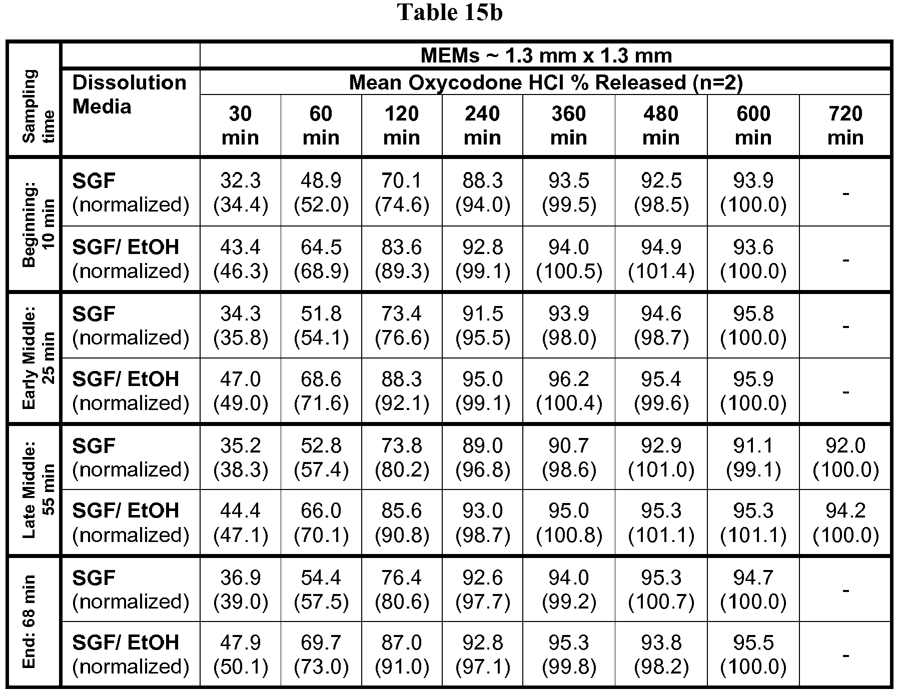

**Table 15c**

| | **MEMs: 1.44 mm ± 0.06 x 1.56 mm ± 0.23; Sampling time - Early Middle: 35 min** | | | | | | | |
|---|---|---|---|---|---|---|---|---|
| **Dissolution Media** | **Mean Oxycodone HCl % Released (n=2)** | | | | | | | |
| | **30 min** | **60 min** | **120 min** | **240 min** | **360 min** | **480 min** | **600 min** | **720 min** |
| **SGF** (normalized) | 27.7 (30.6) | 41.3 (45.5) | 60.1 (66.3) | 78.5 (86.6) | 87.5 (96.5) | 89.2 (98.3) | 90.4 (99.7) | 90.7 (100.0) |
| **SGF/ EtOH** (normalized) | 35.4 (36.8) | 55.6 (57.7) | 75.9 (78.7) | 91.8 (95.3) | 94.8 (98.4) | 97.1 (100.7) | 94.9 (98.5) | 96.4 (100.0) |

The crush testing results of Example 15 are summarized in Table 15d.

**Table 15d**

| **Mean Pellet Dimension D (mm) x L (mm) (n = 24-45)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.32 ± 0.10 x 1.40 ± 0.12 | **Wt (mg)** | 205.4 | 204.5 | 204.1 | 202.1 | 201.5 | 200.0 | 197.7 |
| | **% Rtnd** | 100.0 | 99.6 | 99.4 | 98.4 | 98.1 | 97.4 | 96.3 |
| 1.11 ± 0.14 x 1.15 ± 0.11 | **Wt (mg)** | 202.2 | 200.2 | 199.9 | 198.7 | 195.4 | 193.3 | 194.2 |
| | **% Rtnd** | 100.0 | 99.0 | 98.9 | 98.3 | 96.6 | 95.6 | 96.0 |
| 1.28 ± 0.09 x 1.33 ± 0.15 | **Wt (mg)** | 205.1 | 199.9 | 199.2 | 198.2 | 198.1 | 197.8 | 193.4 |
| | **% Rtnd** | 100.0 | 97.5 | 97.1 | 96.6 | 96.6 | 96.4 | 94.3 |
| 1.44 ± 0.06 x 1.56 ± 0.23 | **Wt (mg)** | 208.1 | 202.5 | 200.1 | 199.4 | 196.3 | 195.2 | 193.4 |
| | **% Rtnd** | 100.0 | 97.3 | 96.2 | 95.8 | 94.3 | 93.8 | 92.9 |
| 1.23 ± 0.12 x 1.36 ± 0.17 | **Wt (mg)** | 208.5 | 204.8 | 203.8 | 202.5 | 200.8 | 200.5 | 199.3 |
| | **% Rtnd** | 100.0 | 98.2 | 97.7 | 97.1 | 96.3 | 96.2 | 95.6 |
| 1.27 ± 0.09 x 1.38 ± 0.13 | **Wt (mg)** | 203.1 | 198.1 | 195.5 | 194.9 | 192.8 | 191.8 | 191.2 |
| | **% Rtnd** | 100.0 | 97.5 | 96.3 | 96.0 | 94.9 | 94.4 | 94.1 |

### Example 16

The processing conditions for Example 16 at the time of sampling are summarized in Table 16 below.

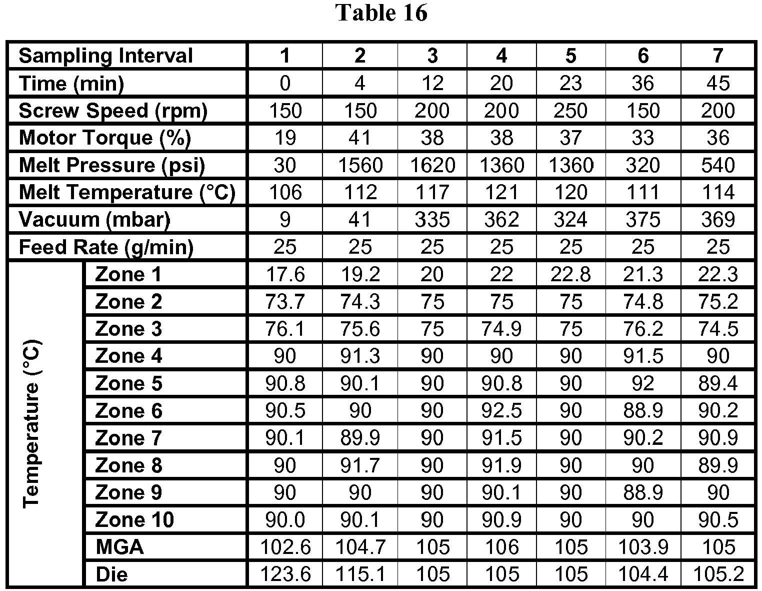

The dissolution results for Example 16 MEMs are summarized in Figure 16 and Table 16a.

**Table 16a**

| **Mean Pellet Dimension D (mm) x L (mm) (n = 22-50)** | **Mean Oxycodone HCl % Released (n=2) Dissolution Media SGF (normalized)** | | | | | | | |
|---|---|---|---|---|---|---|---|---|
| | **30 min** | **60 min** | **120 min** | **240 min** | **360 min** | **480 min** | **600 min** | **720 min** |
| 1.03 ± 0.09 x 1.09 ± 0.10, sampling interval 2 (Beq) | 22.8 (32.7) | 34.6 (49.6) | 49.7 (71.3) | 63.2 (90.7) | 67.8 (97.3) | 69.2 (99.3) | 69.6 (99.8) | 69.7 (100.0) |
| 1.16 ± 0.11 x 1.13 ± 0.13, sampling interval 4 (Mid) | 21.6 (29.3) | 33.4 (45.3) | 49.4 (67.1) | 66.1 (89.7) | 71.4 (97.0) | 73.1 (99.2) | 73.4 (99.7) | 73.7 (100.0) |
| 1.03 ± 0.11 x 1.16 ± 0.13, sampling interval 5 (End) | 26.9 (33.0) | 39.2 (48.1) | 54.3 (66.6) | 67.9 (83.3) | 76.3 (93.6) | 79.9 (98.0) | 82.1 (100.7) | 81.5 (100.0) |
| 1.42 ± 0.10 x 1.32 ± 0.10, Mixed Bulk MEMs (Composite) | 16.4 (23.0) | 24.1 (33.6) | 35.1 (49.0) | 51.9 (72.5) | 61.6 (86.1) | 67.2 (93.8) | 70.4 (98.3) | 71.6 (100.0) |
| 1.89 ± 0.20 x 2.00 ± 0.16, sampling interval 6 (Beq) | 10.8 (15.0) | 16.3 (22.7) | 25.0 (34.8) | 39.8 (55.4) | 51.7 (72.0) | 60.3 (84.0) | 66,0 (92.0) | 71.8 (100.0) |
| 1.71 ± 0.13 x 1.82 ± 0.14, sampling interval 7 (End) | 13.3 (16.9) | 20.9 (26.5) | 31.5 (39.9) | 49.2 (62.3) | 61.0 (77.3) | 69.2 (87.7) | 74.7 (94.6) | 79.0 (100.0) |

The crush testing results of Example 16 are summarized in Table 16b.

**Table 16b**

| **Mean Pellet Dimension D (mm) x L (mm) (n = 22-50)** | **Amount Retained** | **Milling Number/Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.03 ± 0.09 x 1.09 ± 0.10, sampling interval 2 (Beq) | **Wt (mg)** | 204.9 | 198.1 | 195.0 | 189.9 | 185.7 | 180.4 | 178.9 |
| | **% Rtnd** | 100.0 | 96.7 | 95.2 | 92.7 | 90.6 | 88.0 | 87.3 |
| 1.16 ± 0.11 x 1.13 ± 0.13, sampling interval 4 (Mid) | **Wt (mg)** | 208.7 | 193.7 | 193.0 | 191.1 | 190.7 | 187.2 | 184.0 |
| | **% Rtnd** | 100.0 | 92.8 | 92.5 | 91.6 | 91.4 | 89.7 | 88.2 |
| 1.03 ± 0.11 x 1.16 ± 0.13, sampling interval 5 (End) | **Wt (mg)** | 204.6 | 201.1 | 202.6 | 202.0 | 193.2 | 193.9 | 193.8 |
| | **% Rtnd** | 100.0 | 98.3 | 99.0 | 98.7 | 94.4 | 94.8 | 94.7 |
| 1.42 ± 0.10 x 1.32 ± 0.10, Mixed Bulk MEMs (Comp) | **Wt (mg)** | 212.0 | 206.5 | 202.5 | 198.4 | 198.2 | 196.4 | 193.1 |
| | **% Rtnd** | 100.0 | 97.4 | 95.5 | 93.6 | 93.5 | 92.6 | 91.1 |
| 1.89 ± 0.20 x 2.00 ± 0.16, sampling interval 6 (Beq) | **Wt (mg)** | 204.9 | 194.1 | 187.8 | 185.3 | 183.3 | 180.8 | 178.3 |
| | **% Rtnd** | 100.0 | 94.7 | 91.7 | 90.4 | 89.5 | 88.2 | 87.0 |
| 1.71 ± 0.13 x 1.82 ± 0.14, sampling interval 7 (End) | **Wt (mg)** | 209.9 | 202.6 | 199.1 | 194.5 | 192.2 | 189.6 | 187.4 |
| | **% Rtnd** | 100.0 | 96.5 | 94.9 | 92.7 | 91.6 | 90.3 | 89.3 |

### Example 17

The processing conditions for Example 17 at the time of sampling are summarized in Table 17 below.

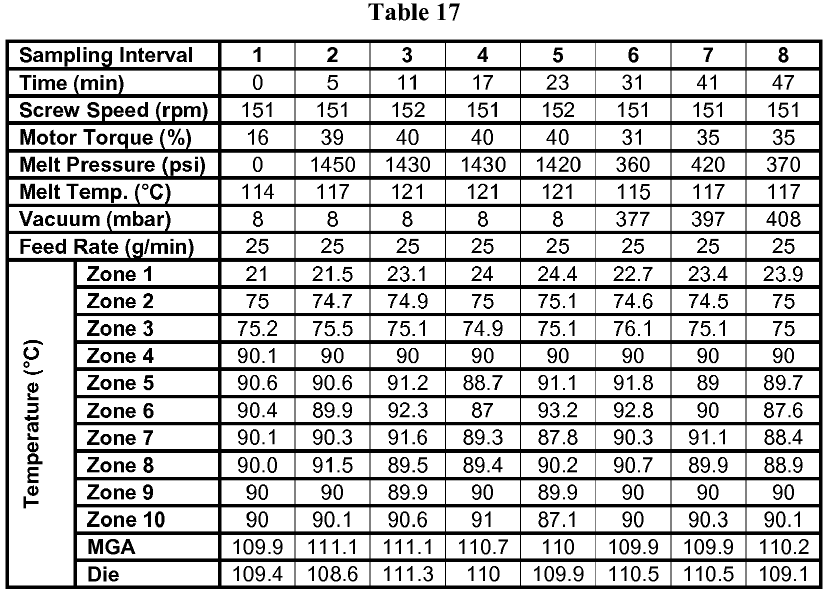

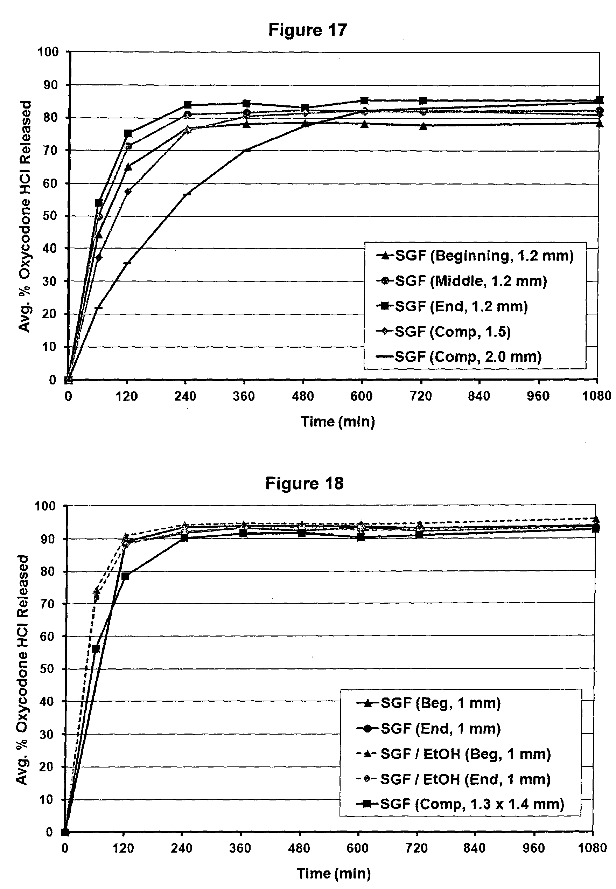

The dissolution results for Example 17 MEMs are summarized in Figure 17 and Tables 17a-17c.

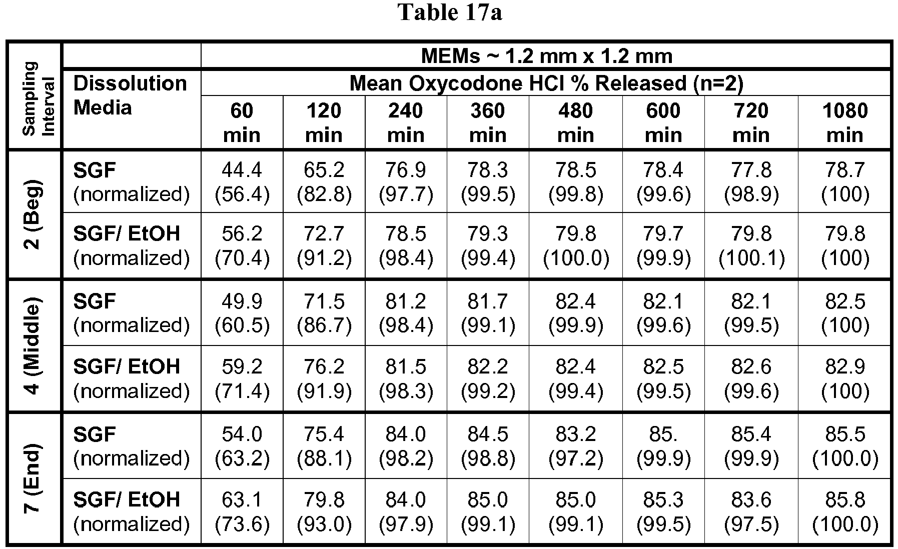

**Table 17b**

| | **MEMs ∼ 1.5 mm x 1.5 mm; Mixed Bulk MEMs (Composite)** | | | | | | | |
|---|---|---|---|---|---|---|---|---|
| **Dissolution Media** | **Mean Oxycodone HCl % Released (n=2)** | | | | | | | |
| | **60 min** | **120 min** | **240 min** | **360 min** | **480 min** | **600 min** | **720 min** | **1080 min** |
| **SGF** (normalized) | 37.3 (46.0) | 57.7 (71.1) | 76.2 (93.9) | 80. (99.3) | 81.5 (100.5) | 82. (101.6) | 82.4 (101.6) | 81.1 (100.0) |

**Table 17c**

| | **MEMs ∼ 2 mm x 2 mm; Mixed Bulk MEMs (Composite)** | | | | | | | |
|---|---|---|---|---|---|---|---|---|
| **Dissolution Media** | **Mean Oxycodone HCl % Released (n=2)** | | | | | | | |
| | **60 min** | **120 min** | **240 min** | **360 min** | **480 min** | **600 min** | **720 min** | **1080 min** |
| **SGF** (normalized) | 21.9 (25.8) | 35.6 (41.9) | 56.8 (66.9) | 70.2 (82.7) | 77.7 (91.5) | 82.3 (97.0) | 83.0 (97.8) | 84.9 (100.0) |

The crush testing results of Example 17 are summarized in Table 17d.

**Table 17d**

| **Mean Pellet Dimension D (mm) x L (mm) (n = 17-41)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.22 ± 0.12 x 1.30 ± 0.16, Beg | **Wt (mg)** | 210.3 | 202.3 | 197.1 | 194.6 | 191.9 | 189.5 | 186.9 |
| | **% Rtnd** | 100.0 | 96.2 | 93.7 | 92.5 | 91.3 | 90.1 | 88.9 |
| 1.18 ± 0.09 x 1.24 ± 0.17, Mid | **Wt (mg)** | 210.6 | 208.7 | 205.1 | 203.7 | 200.3 | 199.8 | 195.6 |
| | **% Rtnd** | 100.0 | 99.1 | 97.4 | 96.7 | 95.1 | 94.9 | 92.9 |
| 1.19 ± 0.11 x 1.15 ± 0.12, End | **Wt (mg)** | 207.0 | 205.6 | 203.6 | 199.9 | 198.2 | 197.0 | 192.9 |
| | **% Rtnd** | 100.0 | 99.3 | 98.4 | 96.6 | 95.7 | 95.2 | 93.2 |
| 1.47 ± 0.09 x 1.45 ± 0.14, Comp | **Wt (mg)** | 208.2 | 203.9 | 200.0 | 195.8 | 193.8 | 191.2 | 188.8 |
| | **% Rtnd** | 100.0 | 97.9 | 96.1 | 94.0 | 93.1 | 91.8 | 90.7 |
| 2.13 ± 0.16 x 2.20 ± 0.23, Comp | **Wt (mg)** | 209.0 | 202.5 | 193.2 | 190.5 | 181.5 | 171.4 | 171.6 |
| | **% Rtnd** | 100.0 | 96.9 | 92.4 | 91.1 | 86.8 | 82.0 | 82.1 |

### Example 18

The processing conditions for Example 18 at the time of sampling are summarized in Table 18 below.

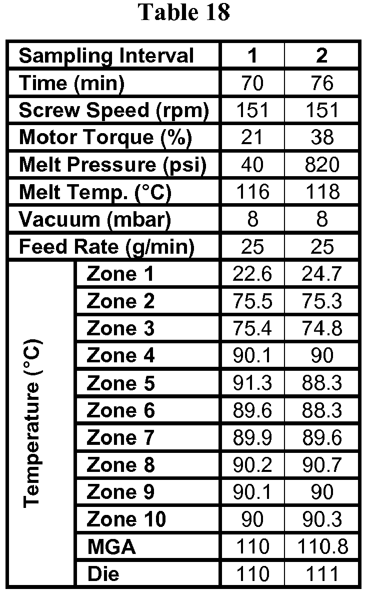

The dissolution results for Example 18 MEMs are summarized in Figure 18 and Tables 18a-18b.

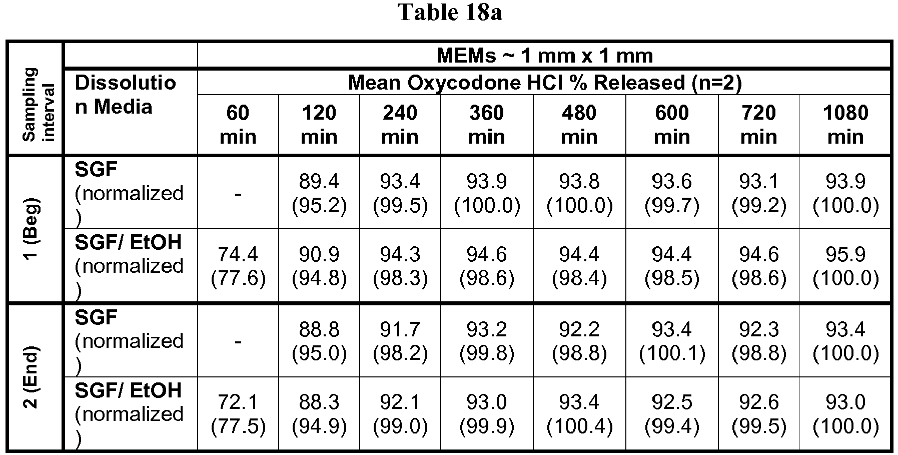

**Table 18b**

| | **MEMs: 1.27 mm ± 0.10 x 1.43 mm ± 0.15; Mixed Bulk MEMs (Comp)** | | | | | | | |
|---|---|---|---|---|---|---|---|---|
| **Dissolution Media** | **Mean Oxycodone HCl % Released (n=2)** | | | | | | | |
| | **60 min** | **120 min** | **240 min** | **360 min** | **480 min** | **600 min** | **720 min** | **1080 min** |
| **SGF** (normalized) | 56.2 (60.7) | 78.5 (84.8) | 90.2 (97.4) | 91.6 (98.9) | 91.7 (99.0) | 90.3 (97.5) | 91.0 (98.3) | 92.6 (100.0) |

The crush testing results of Example 18 are summarized in Table 18c.

**Table 18c**

| **Mean Pellet Dimension D (mm) x L (mm) (n = 31-43)** | **Amount Retained** | **Milling Number / Time** | | | | | | |
|---|---|---|---|---|---|---|---|---|
| | | **Initial / 0s** | **1 / 10s** | **2 / 20s** | **3 / 30s** | **4 / 40s** | **5 / 50s** | **6 / 60s** |
| 1.05 ± 0.07 x 1.20 ± 0.16, Beg | **Wt (mg)** | 205.0 | 204.7 | 202.4 | 197.2 | 193.7 | 191.5 | 188.2 |
| | **% Rtnd** | 100.0 | 99.9 | 98.7 | 96.2 | 94.5 | 93.4 | 91.8 |
| 1.23 ± 0.07 x 1.22 ± 0.11, End | **Wt (mg)** | 206.3 | 204.1 | 201.3 | 196.8 | 193.7 | 190.2 | 192.1 |
| | **% Rtnd** | 100.0 | 98.9 | 97.6 | 95.4 | 93.9 | 92.2 | 93.1 |
| 1.27 ± 0.10 x 1.43 ± 0.15, Comp | **Wt (mg)** | 202.0 | 197.9 | 194.1 | 191.8 | 190.2 | 188.7 | 187.3 |
| | **% Rtnd** | 100.0 | 98.0 | 96.1 | 95.0 | 94.2 | 93.4 | 92.7 |

### EXAMPLES 19 - 36

### Composition

The compositions of the poly(ε-caprolactone) melt-extruded multi-particulates (MEMs) for examples 19-36 are summarized in Tables XIV to XIX below:

**Table XIV**

| **Example Number** | **19** | | **20** | | **21** | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **15.0** | 7.5 | **15.0** | 7.5 | **15**.**0** | 7.5 |
| Poly(ε-caprolactone), **Mn ~ 42**,**500** | **67.2** | 33.6 | **58.8** | 29.4 | **50**.**4** | 25.2 |
| Polyethylene oxide, Mw ~ 100,000 (PEO WSR N10) | **16.8** | 8.4 | **25.2** | 12.6 | **33.6** | 16.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

**Table XV**

| **Example Number** | **22** | | **23** | | **24** | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **20**.**0** | 10.0 | **20**.**0** | 10.0 | **20**.**0** | 10.0 |
| Poly(ε-caprolactone), **Mn** ~ **42**,**500** | **63**.**2** | 31.6 | **55**.**3** | 27.7 | **47.4** | 23.7 |
| Polyethylene oxide, Mw ~ 100,000 (PEO WSR N10) | **15.8** | 7.9 | **23**.**7** | 11.9 | **31**.**6** | 15.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

**Table XVI**

| **Example Number** | **25** | | **26** | | **27** | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **25.0** | 12.5 | **25.0** | 12.5 | **25.0** | 12.5 |
| Poly(ε-caprolactone), **Mn ∼ 42,500** | **59.2** | 29.6 | **51.8** | 25.9 | **44.4** | 22.2 |
| Polyethylene oxide, Mw **∼** 100,000 (PEO WSR N10) | **14.8** | 7.4 | **22.2** | 11.1 | **29.6** | 14.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

**Table XVII**

| **Example Number** | **28** | | **29** | | **30** | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **15.0** | 7.5 | **15.0** | 7.5 | **15.0** | 7.5 |
| Poly(ε-caprolactone), **Mn ~ 42,500** | **67.2** | 33.6 | **58.8** | 29.4 | **50.4** | 25.2 |
| Polyethylene oxide, Mw ~ 900,000 (PEO WSR 1105) | **16.8** | 8.4 | **25.2** | 12.6 | **33.6** | 16.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

**Table XVIII**

| **Example Number** | **31** | | **32** | | **33** | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **20.0** | 10.0 | **20.0** | 10.0 | **20**.**0** | 10.0 |
| Poly(ε-caprolactone), **Mn ~ 42,500** | **63.2** | 31.6 | **55.3** | 27.7 | **47.4** | 23.7 |
| Polyethylene oxide, Mw ~ 900,000 (PEO WSR 1105) | **15.8** | 7.9 | **23.7** | 11.9 | **31.6** | 15.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

**Table XIX**

| **Example Number** | **34** | | **35** | | 36 | |
|---|---|---|---|---|---|---|
| **Ingredient (Trade Name)** | **Amount** | | | | | |
| | **(% w/w)** | **batch (g)** | **(% w/w)** | **batch (g)** | **(% w/w)** | **batch (g)** |
| Naltrexone HCl* | **25.0** | 12.5 | **25.0** | 12.5 | **25.0** | 12.5 |
| Poly(ε-caprolactone), **Mn ~ 42,500** | **59.2** | 29.6 | **51.8** | 25.9 | **44.4** | 22.2 |
| Polyethylene oxide, Mw ~ 900,000 (PEO WSR 1105) | **14.8** | 7.4 | **22.2** | 11.1 | **29.6** | 14.8 |
| Butylated Hydroxy Toluene (BHT) | **1** | 0.5 | **1** | 0.5 | **1** | 0.5 |
| Total | **100** | 50 | **100** | 50 | **100** | 50 |

| | | | | | | |
|---|---|---|---|---|---|---|
| * Amount not corrected for water or impurities. | | | | | | |

### Manufacturing Procedure

1. Blending: Naltrexone HCl, poly(ε-caprolactone) (milled form), polyethylene oxide and milled BHT were added to a glass mortar and triturated for 30 s to 1 minute, or until visually homogenous at ambient temperature.
2. Feeding into Extruder: Materials blended in Step 1 were added to the feeder "Micro-Plunger" of Nano-16™.
3. Melt Extrusion: The blend was metered into Nano-16™ extruder with 4 heating zones, fitted with a main gated adapter (MGA) with a 1.5 mm single-hole die to obtain the strands.
4.
   Cooling: The strands were drawn on a 12ft conveyer belt fitted with 4-fans and cooled at ambient temperature.
   Pelletizing: A downstream pelletizer was used to pelletize the strand into 1.5 mm x 1.5mm pellets; the speed of the conveyer belt was either increased or decreased to obtain thinner or thicker strands respectively.

The processing conditions for Examples 19 to 36 at the time of sampling are summarized in Table 19 below.

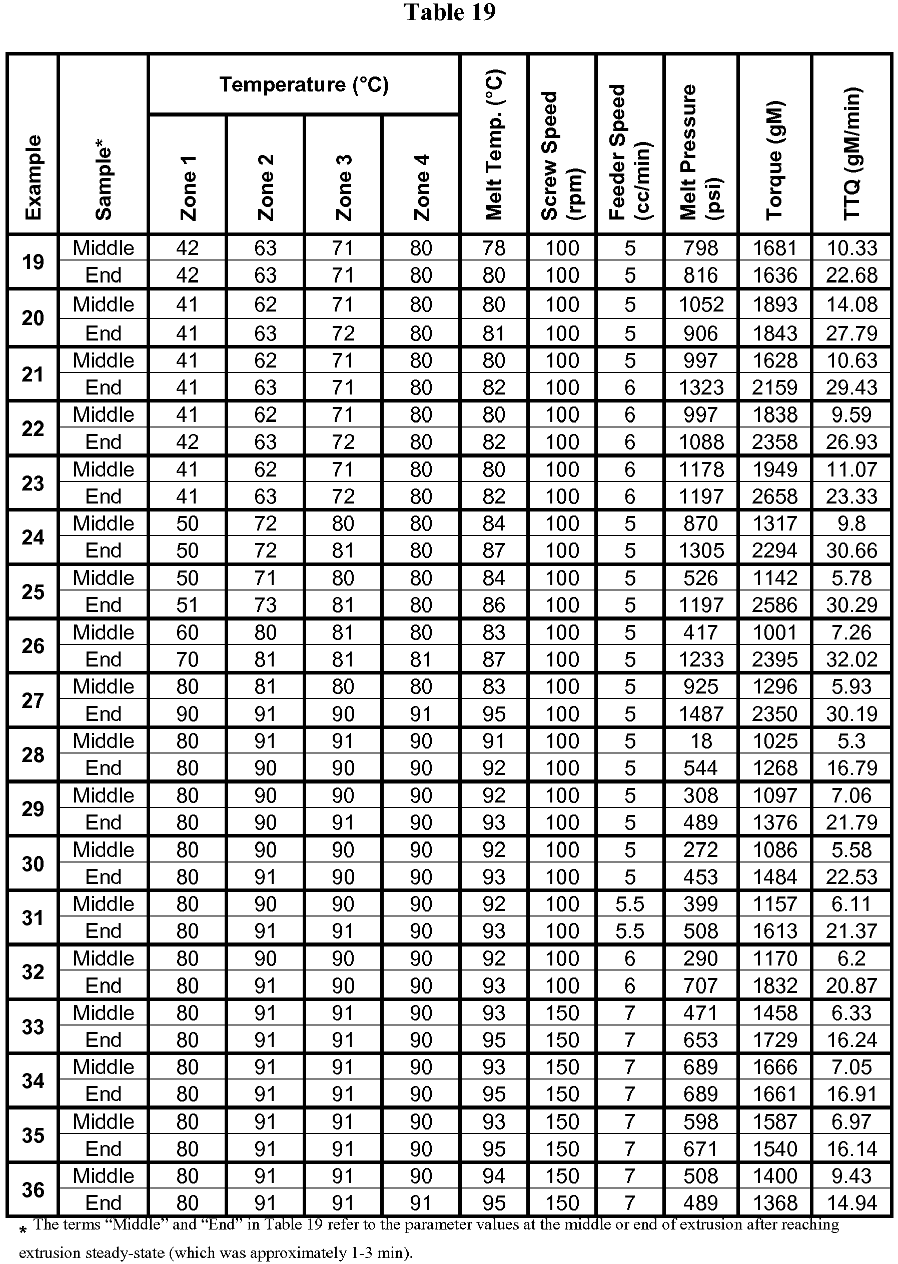

The total extrusion time for Examples 19-36 varies from 15 to 20 minutes. Samples of MEMs to perform dissolution and crush testing measurements for Examples 19 to 36 are removed from bulk pellets as composites.

The crush testing results of Examples 19-36 are summarized in Table 19a.

**Table 19a**

| **Ex.** | **Mean Pellet Dimension D (mm) x L (mm) (n = 10-22)*** | **Amount Retained** | **Milling Number/Time** | | | | | | |
|---|---|---|---|---|---|---|---|---|---|
| | | | **Initial /0s** | **1 / 10s** | **2 / 20s** | **3 / 30s** | **4 / 40s** | **5 / 50s** | **6 / 60s** |
| **19** | 1.76 ± 0.17 x 1.44 ± 0.19 | **Wt (mg)** | 201.2 | 189.6 | 168.2 | 156.2 | 154.4 | 139.8 | 136.2 |
| | | **% Rtnd** | 100.0 | 94.2 | 83.60 | 77.6 | 76.7 | 69.5 | 67.7 |
| **20** | 1.78 ± 0.23 x 1.34 ± 0.26 | **Wt (mg)** | 203.4 | 198.3 | 193.4 | 183.4 | 154.9 | 146.4 | 153.9 |
| | | **% Rtnd** | 100.0 | 97.5 | 95.08 | 90.2 | 76.2 | 72.0 | 75.7 |
| **21** | 1.93 ± 0.19 x 1.31 ± 0.25 | **Wt (mg)** | 205.4 | 187.8 | 181.8 | 176.4 | 159.9 | 150.2 | 146.2 |
| | | **% Rtnd** | 100.0 | 91.4 | 88.51 | 85.9 | 77.8 | 73.1 | 71.2 |
| **22** | 1.65 ± 0.15 x 1.34 ± 0.18 | **Wt (mg)** | 201.4 | 182.3 | 164.6 | 142.1 | 133.2 | 129.2 | 123.7 |
| | | **% Rtnd** | 100.0 | 90.5 | 81.73 | 70.6 | 66.1 | 64.2 | 61.4 |
| **23** | 1.63 ± 0.18 x 1.31 ± 0.24 | **Wt (mg)** | 206.7 | 179.8 | 174.4 | 149.6 | 135.2 | 129.8 | 117.4 |
| | | **% Rtnd** | 100,0 | 87,0 | 84.37 | 72.4 | 65.4 | 62.8 | 56.8 |
| **24** | 1.77 ± 0.24 x 1.43 ± 0.21 | **Wt (mg)** | 205.7 | 184.5 | 168.3 | 156.7 | 138.9 | 128.1 | 118.6 |
| | | **% Rtnd** | 100.0 | 89.7 | 81.8 | 76.2 | 67.5 | 62.3 | 57.7 |
| **25** | 1.63 ± 0.12 x 1.35 ± 0.09 | **Wt (mg)** | 201.8 | 174.5 | 158.8 | 145.8 | 131.2 | 120.1 | 113.0 |
| | | **% Rtnd** | 100.0 | 86.5 | 78.69 | 72.2 | 65.0 | 59.5 | 56.0 |
| **26** | 1.66 ± 0.14 x 1.25 ± 0.15 | **Wt (mg)** | 205.8 | 189.5 | 165.3 | 150.3 | 143.8 | 123.4 | 116.5 |
| | | **% Rtnd** | 100.0 | 92.1 | 80.32 | 73.0 | 69.9 | 60.0 | 56.6 |
| **27** | 1.68 ± 0.21 x 1.41 ± 0.17 | **Wt (mg)** | 204.3 | 182.2 | 160.2 | 140.6 | 132.2 | 119.1 | 106.1 |
| | | **% Rtnd** | 100.0 | 89.2 | 78.4 | 68.8 | 64.7 | 58.3 | 51.9 |
| **28** | ~ 1.5 x 1.5 | **Wt (mg)** | 206.7 | 194.8 | 173.9 | 155.1 | 138.5 | 114.8 | 106.2 |
| | | **% Rtnd** | 100.0 | 94.2 | 84.13 | 75.0 | 67.0 | 55.5 | 51.4 |
| **29** | 1.78 ± 0.15 x 0.22 ± 0.16 | **Wt (mg)** | 206.6 | 199.7 | 196.6 | 189.4 | 182.7 | 168.5 | 154.1 |
| | | **% Rtnd** | 100.0 | 96.7 | 95.16 | 91.7 | 88.4 | 81.6 | 74.6 |
| **30** | ~ 1.5 x 1.5 | **Wt (mg)** | 206.1 | 194.5 | 185.9 | 170.8 | 161.1 | 142.8 | 123.0 |
| | | **% Rtnd** | 100.0 | 94.4 | 90.20 | 82.9 | 78.2 | 69.3 | 59.7 |
| **31** | ~ 1.5 x 1.5 | **Wt (mg)** | 204.1 | 185.8 | 167.5 | 149.1 | 122.6 | 112.7 | 96.4 |
| | | **% Rtnd** | 100.0 | 91.0 | 82.1 | 73.1 | 60.1 | 55.2 | 47.2 |
| **32** | 1.38 ± 0.07 x 1.04 ± 0.27 | **Wt (mg)** | 205.6 | 193.1 | 174.1 | 161.5 | 144.3 | 128.1 | 110.6 |
| | | **% Rtnd** | 100.0 | 93.9 | 84.7 | 78.6 | 70.2 | 62.3 | 53.8 |
| **33** | ~ 1.5 x 1.5 | **Wt (mg)** | 204.2 | 187.0 | 171.3 | 155.7 | 132.7 | 123.7 | 100.8 |
| | | **% Rtnd** | 100.0 | 91.6 | 83.9 | 76.2 | 65.0 | 60.6 | 49.4 |
| **34** | ~ 1.5 x 1.5 | **Wt (mg)** | 203.6 | 192.0 | 184.7 | 162.4 | 143.3 | 127.2 | 123.6 |
| | | **% Rtnd** | 100.0 | 94.3 | 90.7 | 79.8 | 70.4 | 62.5 | 60.7 |
| **35** | 1.69 ± 0.19 x 1.37 ± 0.09 | **Wt (mg)** | 207.3 | 195.1 | 168.7 | 150.5 | 130.4 | 111.7 | 98.6 |
| | | **% Rtnd** | 100.0 | 94.1 | 81.4 | 72.6 | 62.9 | 53.9 | 47.6 |
| **36** | ~ 1.5 x 1.5 | **Wt (mg)** | 205.8 | 200.2 | 191.0 | 179.3 | 160.4 | 150.1 | 139.0 |
| | | **% Rtnd** | 100.0 | 97.3 | 92.8 | 87.1 | 77.9 | 72.9 | 67.5 |

| | | | | | | | | | |
|---|---|---|---|---|---|---|---|---|---|
| * Pellet dimension analysis was not performed for Examples 28, 30, 31, 33, 34 and 36. | | | | | | | | | |

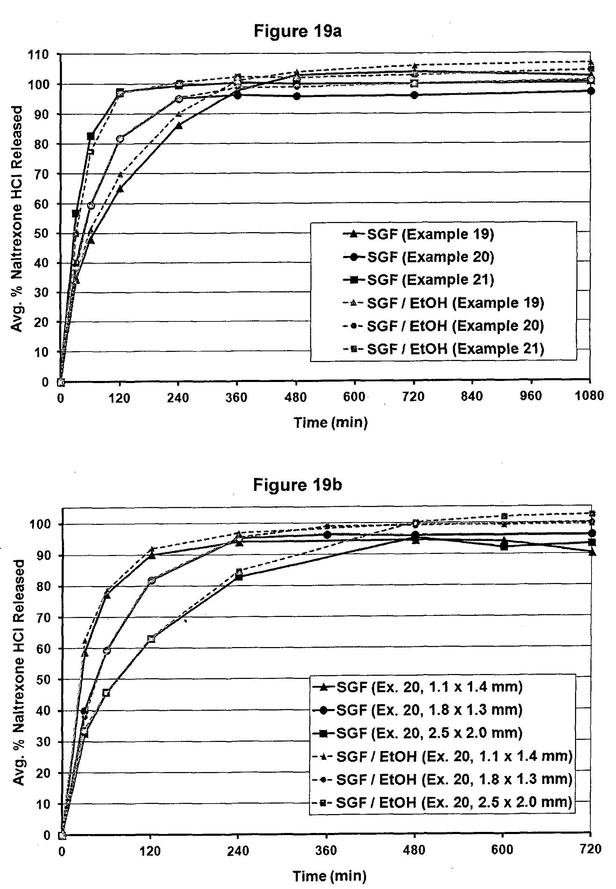

The dissolution results for Examples 19-21 MEMs in capsules are summarized in Figure 19a and Table 19b.

**Table 19b**

| **Ex.** | **Dissolution Media** | **Mean Naltrexone HCl % Released (n=2)** | | | | | | | |
|---|---|---|---|---|---|---|---|---|---|
| | | **30 min** | **60 min** | **120 min** | **240 min** | **360 min** | **480 min** | **720 min** | **1080 min** |
| **19** | **SGF** (normalized) | 34.4 (33.6) | 48.1 (46.9) | 65.3 (63.6) | 86.4 (84.2) | 97.7 (95.3) | 102.9 (100.4) | 104.0 (101.5) | 102.6 (100.0) |
| | **SGF/ EtOH** (normalized) | 35.0 (32.7) | 51.4 (48.0) | 69.9 (65.3) | 90.2 (84.3) | 101.1 (94.5) | 104.1 (97.3) | 106.1 (99.2) | 107.0 (100.0) |
| **20** | **SGF** (normalized) | 39.8 (41.0) | 59.4 (61.1) | 81.8 (84.2) | 95.3 (98.0) | 96.3 (99.1) | 96.0 (98.8) | 96.2 (99.0) | 97.2 (100.0) |
| | SGF/EtOH (normalized) | 38.0 (37.6) | 59.7 (59.0) | 82.1 (81.2) | 95.5 (94.4) | 98.9 (97.8) | 99.1 (97.9) | 100.1 (98.9) | 101.2 (100.0) |
| **21** | SGF (normalized) | 56.7 (56.3) | 82.6 (82.1) | 97.6 (96.9) | 99.5 (98.8) | 100.5 (99.9) | 100.1 (99.5) | 100.1 (99.4) | 100.7 (100.0) |
| | **SGF/ EtOH** (normalized) | 49.9 (47.8) | 77.4 (74.0) | 96.9 (92.7) | 100.6 (96.3) | 102.4 (98.0) | 102.3 (97.8) | 103.1 (98.6) | 104.5 (100.0) |

For Example 20, strands of thicker and thinner dimensions were also collected and pelletized. The dissolution results for Example 20 MEMs in capsules with a pellet size smaller than 1.5 mm x 1.5 mm are summarized in Table 19c, dissolution results for Example 20 MEMs in capsules with a pellet size larger than 1.5 mm x 1.5 mm are summarized in Table 19d. The dissolution results for all Example 20 MEMs in capsules are summarized in Figure 19b.

**Table 19c**

| | **MEMs: 1.06 mm ± 0.05 x 1.36 mm ± 0.30** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 58.8 (65.1) | 77.6 (85.9) | 90.0 (99.7) | 94.1 (104.2) | 94.5 (104.7) | 94.2 (104.3) | 90.3 (100.0) |
| **SGF/ EtOH** (normalized) | 62.6 (62.8) | 78.8 (79.1) | 92.0 (92.3) | 96.9 (97.3) | 99.6 (100.0) | 99.3 (99.7) | 99.6 (100.0) |

**Table 19d**

| | **MEMs: 2.46 mm ± 0.17 x 1.98 mm ± 0.34** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 32.4 (34.7) | 45.8 (49.0) | 63.0 (67.5) | 82.8 (88.7) | 95.3 (102.1) | 92.1 (98.7) | 93.3 (100.0) |
| **SGF/ EtOH** (normalized) | 33.9 (33.1) | 45.5 (44.4) | 63.3 (61.8) | 84.8 (82.7) | 100.1 (97.7) | 101.9 (99.4) | 102.5 (100.0) |

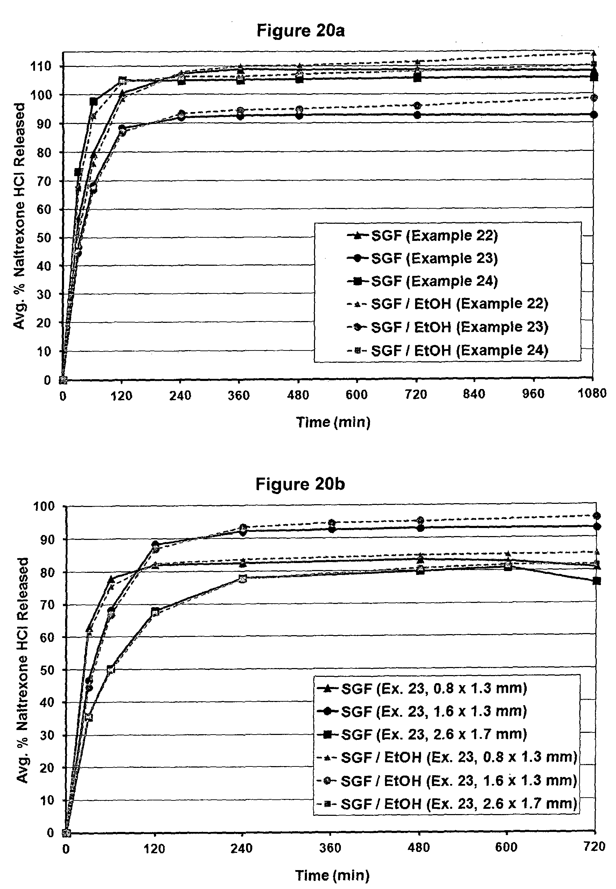

The dissolution results for Examples 22-24 MEMs in capsules are summarized in Figure 20a and Table 20a.

**Table 20a**

| **Ex.** | **Dissolution Media** | **Mean Naltrexone HCl % Released (n=2)** | | | | | | | |
|---|---|---|---|---|---|---|---|---|---|
| | | **30 min** | **60 min** | **120 min** | **240 min** | **360 min** | **480 min** | **720 min** | **1080 min** |
| **22** | **SGF** (normalized) | 56.8 (52.4) | 79.6 (73.5) | 100.6 (93.0) | 107.3 (99.1) | 108.8 (100.5) | 108.6 (100.3) | 108.6 (100.3) | 108.2 (100.0) |
| | **SGF/ EtOH** (normalized) | 51.8 (45.4) | 76.0 (66.6) | 98.7 (86.5) | 107.7 (94.3) | 110.0 (96.4) | 110.2 (96.5) | 111.4 (97.6) | 114.1 (100.0) |
| **23** | **SGF** (normalized) | 46.8 (50.5) | 68.4 (73.8) | 88.4 (95.5) | 92.1 (100.0) | 92.6 (100.3) | 92.9 (100.3) | 92.9 (100.3) | 92.6 (100.0) |
| | **SGF/ EtOH** (normalized) | 44.6 (45.3) | 66.8 (67.7) | 87.0 (88.3) | 93.4 (94.8) | 94.6 (96.0) | 95.1 (96.5) | 96.2 (97.6) | 98.6 (100.0) |
| **24** | **SGF** (normalized) | 73.1 (69.1) | 97.8 (92.4) | 105.0 (99.2) | 104.8 (99.1) | 105.1 (99.3) | 105.4 (99.7) | 105.8 (100.0) | 105.8 (100.0) |
| | **SGF/ EtOH** (normalized) | 67.4 (61.1) | 92.6 (84.0) | 104.5 (94.7) | 106.2 (96.3) | 106.2 (96.3) | 107.1 (97.1) | 108.2 (98.1) | 110.3 (100.0) |

For Example 23, strands of thicker and thinner dimensions were also collected and pelletized. The dissolution results for Example 23 MEMs in capsules with a pellet size smaller than 1.5 mm x 1.5 mm are summarized in Table 20b, dissolution results for Example 23 MEMs in capsules with a pellet size larger than 1.5 mm x 1.5 mm are summarized in Table 20c. The dissolution results for all Example 23 MEMs in capsules are summarized in Figure 20b.

**Table 20b**

| | **MEMs: 0.75 mm ± 0.23 x 1.31 mm ± 0.55** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 63.1 (77.9) | 78.0 (96.3) | 82.1 (101.4) | 82.6 (101.9) | 83.4 (102.9) | 82.8 (102.2) | 81.0 (100.0) |
| **SGF/ EtOH** (normalized) | 61.4 (72.1) | 75.5 (88.6) | 82.6 (97.0) | 83.6 (98.2) | 84.7 (99.5) | 85.1 (99.9) | 85.2 (100.0) |

**Table 20c**

| | **MEMs: 2.56 mm ± 0.12 x 1.73 mm ± 0.20** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 35.4 (46.5) | 50.4 (66.1) | 68.1 (89.2) | 77.9 (102.1) | 79.9 (104.7) | 80.9 (106.0) | 76.3 (100.0) |
| **SGF/ EtOH** (normalized) | 35.9 (43.9) | 49.5 (60.6) | 67.2 (82.2) | 77.7 (95.2) | 80.8 (98.9) | 81.7 (100.0) | 81.7 (100.0) |

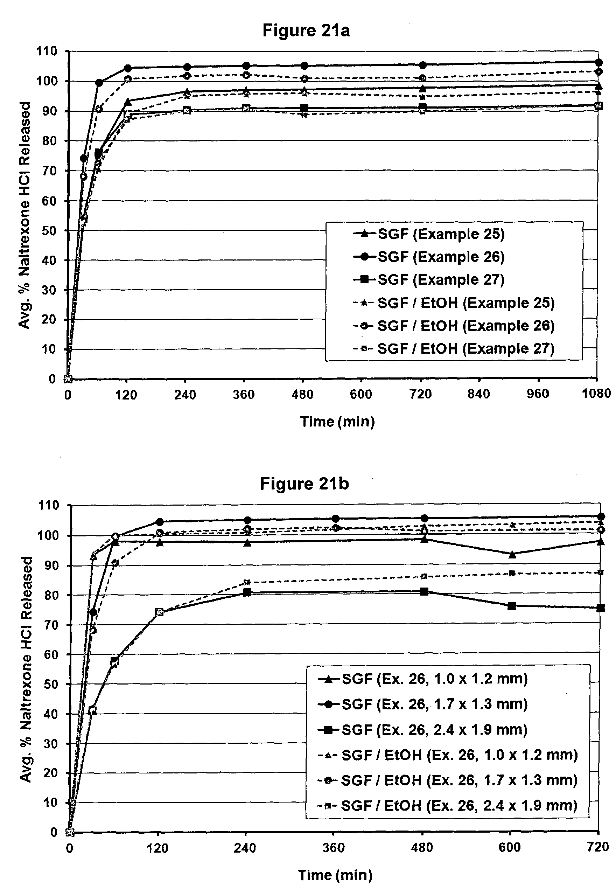

The dissolution results for Examples 25-27 MEMs in capsules are summarized in Figure 21a and Table 21a.

**Table 21a**

| **Ex.** | **Dissolution Media** | **Mean Naltrexone HCl % Released (n=2)** | | | | | | | |
|---|---|---|---|---|---|---|---|---|---|
| | | **30 min** | **60 min** | **120 min** | **240 min** | **360 min** | **480 min** | **720 min** | **1080 min** |
| **25** | **SGF** (normalized) | 54.2 (54.9) | 75.6 (76.7) | 93.4 (94.7) | 96.6 (98.0) | 97.1 (98.5) | 97.2 (98.5) | 97.8 (99.1) | 98.6 (100.0) |
| | **SGF/ EtOH** (normalized) | 52.9 (54.9) | 70.6 (73.3) | 89.3 (92.7) | 95.2 (98.8) | 95.7 (99.3) | 96.0 (99.6) | 94.9 (98.4) | 96.4 (100.0) |
| **26** | **SGF** (normalized) | 74.5 (70.0) | 99.6 (93.7) | 104.5 (98.3) | 104.9 (98.6) | 105.2 (98.9) | 105.2 (98.9) | 105.6 (99.2) | 106.4 (100.0) |
| | **SGF/ EtOH** (normalized) | 68.3 (66.2) | 90.9 (88.1) | 100.8 (97.7) | 101.9 (98.7) | 102.2 (99.0) | 100.9 (97.8) | 101.2 (98.0) | 103.2 (100.0) |
| **27** | **SGF** (normalized) | 54.2 (59.0) | 76.3 (83.1) | 89.0 (96.9) | 90.4 (98.5) | 91.0 (99.2) | 91.0 (99.1) | 91.3 (99.5) | 91.8 (100.0) |
| | **SGF/ EtOH** (normalized) | 52.5 (57.0) | 72.9 (79.3) | 87.2 (94.7) | 90.1 (97.9) | 90.6 (98.4) | 88.9 (96.6) | 89.8 (97.6) | 92.1 (100.0) |

For Example 26, strands of thicker and thinner dimensions were also collected and pelletized. The dissolution results for Example 26 MEMs in capsules with a pellet size smaller than 1.5 mm x 1.5 mm are summarized in Table 21b, dissolution results for Example 26 MEMs in capsules with a pellet size larger than 1.5 mm x 1.5 mm are summarized in Table 21c. The dissolution results for all Example 26 MEMs in capsules are summarized in Figure 21b.

**Table 21b**

| | **MEMs: 1.00 mm ± 0.04 x 1.24 mm ± 0.39** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 93.4 (95.9) | 98.0 (100.6) | 97.8 (100.5) | 97.6 (100.2) | 98.3 (100.9) | 93.2 (95.7) | 97.4 (100.0) |
| **SGF EtOH** (normalized) | 93.8 (90.5) | 99.9 (96.4) | 100.4 (97.0) | 100.6 (97.1) | 102.6 (99.0) | 103.1 (99.5) | 103.6 (100.0) |

**Table 21c**

| | **MEMs: 2.35 mm ± 0.12 x 1.92 mm ± 0.45** | | | | | | |
|---|---|---|---|---|---|---|---|
| **Dissolution Media** | **Mean Naltrexone HCl % Released (n=2)** | | | | | | |
| | **30 min** | **60 min** | **120 min** | **240 min** | **480 min** | **600 min** | **720 min** |
| **SGF** (normalized) | 41.1 (54.8) | 57.8 (77.0) | 74.3 (98.9) | 80.8 (107.7) | 80.9 (107.8) | 75.9 (101.1) | 75.1 (100.0) |
| **SGF/ EtOH** (normalized) | 41.8 (48.1) | 56.4 (65.0) | 74.3 (85.6) | 84.1 (96.8) | 85.8 (98.8) | 86.6 (99.7) | 86.9 (100.0) |

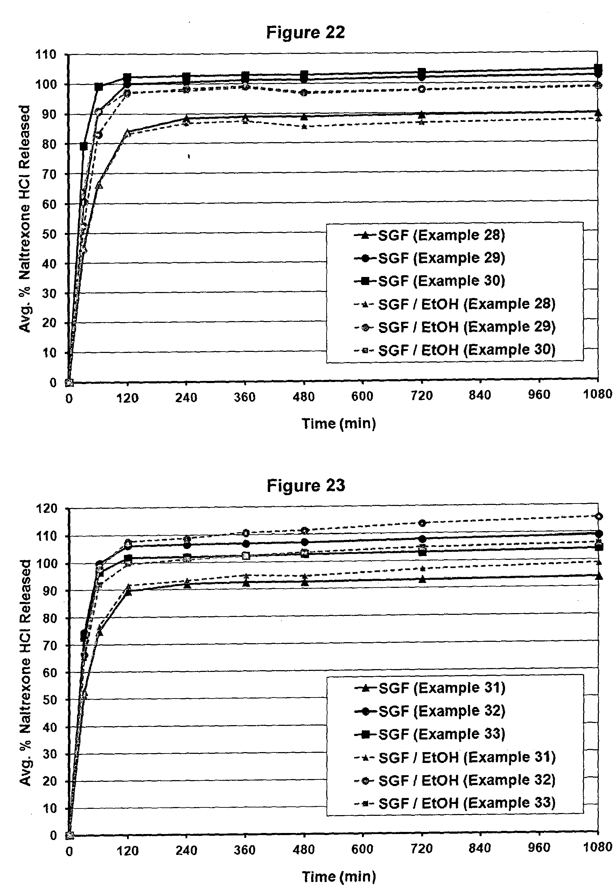

The dissolution results for Examples 28-30 MEMs in capsules are summarized in Figure 22 and Table 22.

**Table 22**

| **Ex.** | **Dissolution Media** | **Mean Naltrexone HCl % Released (n=2)*** | | | | | | | |
|---|---|---|---|---|---|---|---|---|---|
| | | **30 min** | **60 min** | **120 min** | **240 min** | **360 min** | **480 min** | **720 min** | **1080 min** |
| **28** | **SGF** (normalized) | 45.4 (50.5) | 66.7 (74.3) | 84.0 (93.5) | 88.5 (98.5) | 88.8 (98.9) | 89.0 (99.1) | 89.5 (99.6) | 89.8 (100.0) |
| | **SGF/ EtOH** (normalized) | 44.4 (50.6) | 66.4 (75.7) | 83.3 (95.0) | 86.9 (99.0) | 87.5 (99.8) | 85.6 (97.6) | 86.9 (99.0) | 87.7 (100.0) |
| **29** | **SGF** (normalized) | 60.9 (59.4) | 90.8 (88.6) | 100.0 (97.5) | 100.6 (98.2) | 101.1 (98.6) | 101.2 (98.7) | 102.0 (99.5) | 102.5 (100.0) |
| | **SGF/ EtOH** (normalized) | 52.6 (53.3) | 83.2 (84.3) | 96.8 (98.1) | 98.3 (99.6) | 99.0 (100.3) | 97.0 (98.3) | 97.9 (99.2) | 98.7 (100.0) |
| **30** | **SGF** (normalized) | 79.5 (76.1) | 99.2 (95.0) | 102.3 (97.9) | 102.6 (98.2) | 102.9 (98.5) | 102.9 (98.6) | 103.6 (99.2) | 104.4 (100.0) |
| | **SGF/ EtOH** (normalized) | 64.1 (65.0) | 91.1 (92.4) | 97.3 (98.8) | 97.7 (99.1) | 98.7 (100.2) | 96.6 (98.0) | 97.6 (99.1) | 98.5 (100.0) |

| | | | | | | | | | |
|---|---|---|---|---|---|---|---|---|---|
| * n=1 for Example 29. | | | | | | | | | |

The dissolution results for Examples 31-33 MEMs in capsules are summarized in Figure 23 and Table 23.

**Table 23**

| **Ex.** | **Dissolution Media** | **Mean Naltrexone HCl % Released (n=2)** | | | | | | | |
|---|---|---|---|---|---|---|---|---|---|
| | | **30 min** | **60 min** | **120 min** | **240 min** | **360 min** | **480 min** | **720 min** | **1080 min** |
| **31** | **SGF** (normalized) | 52.8 (56.3) | 74.9 (79.9) | 89.6 (95.6) | 92.1 (98.2) | 92.6 (98.8) | 92.6 (98.8) | 93.1 (99.4) | 93.7 (100.0) |
| | **SGF/ EtOH** (normalized) | 51.5 (52.2) | 76.7 (77.8) | 91.6 (92.9) | 93.4 (94.7) | 95.1 (96.4) | 94.8 (96.0) | 97.0 (98.3) | 98.7 (100.0) |
| **32** | **SGF** (normalized) | 74.7 (68.5) | 99.7 (91.5) | 106.1 (97.4) | 106.5 (97.7) | 106.7 (97.9) | 107.1 (98.3) | 107.8 (98.9) | 109.0 (100.0) |
| | **SGF/ EtOH** (normalized) | 66.6 (57.7) | 98.3 (85.2) | 107.6 (93.3) | 108.8 (94.3) | 110.6 (95.9) | 111.2 (96.4) | 113.5 (98.4) | 115.3 (100.0) |
| **33** | **SGF** (normalized) | 73.0 (70.2) | 96.5 (92.7) | 101.7 (97.7) | 101.9 (97.9) | 102.3 (98.3) | 102.7 (98.7) | 103.1 (99.0) | 104.1 (100.0) |
| | **SGF/ EtOH** (normalized) | 65.1 (61.4) | 92.0 (86.8) | 99.6 (93.9) | 101.0 (95.2) | 102.2 (96.4) | 103.4 (97.5) | 104.8 (98.9) | 106.0 (100.0) |

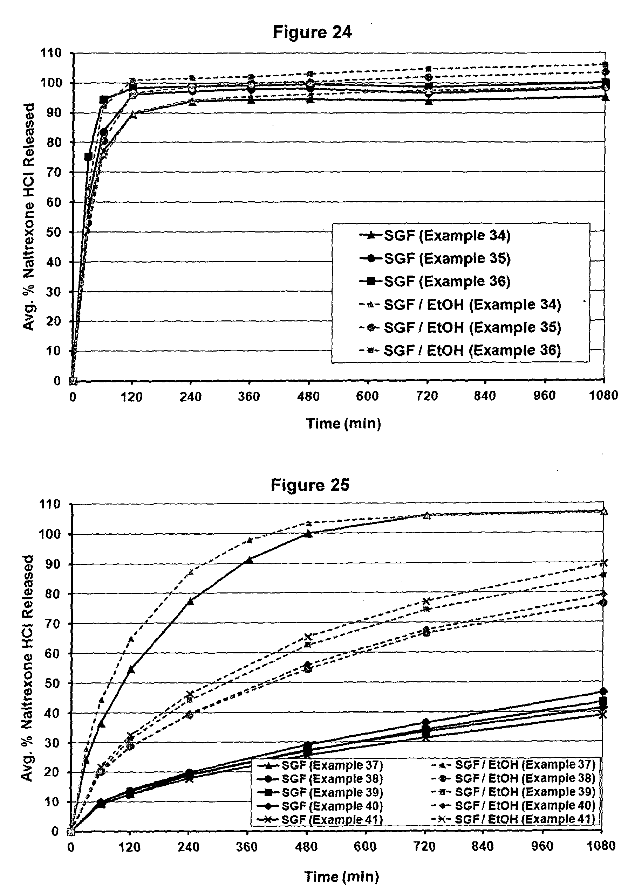

The dissolution results for Examples 34-36 MEMs in capsules are summarized in Figure 24 and Table 24.

**Table 24**

| **Ex.** | **Dissolution Media** | **Mean Naltrexone HCl % Released (n=2)** | | | | | | | |
|---|---|---|---|---|---|---|---|---|---|
| | | **30 min** | **60 min** | **120 min** | **240 min** | **360 min** | **480 min** | **720 min** | **1080 min** |
| **34** | **SGF** (normalized) | 56.4 (59.2) | 77.2 (81.0) | 89.6 (94.0) | 93.8 (98.4) | 94.3 (99.0) | 94.6 (99.3) | 94.0 (98.6) | 95.3 (100.0) |
| | **SGF/ EtOH** (normalized) | 51.0 (51.8) | 75.5 (76.7) | 90.1 (91.5) | 94.3 (95.8) | 95.4 (96.9) | 96.3 (97.8) | 97.4 (98.9) | 98.5 (100.0) |
| **35** | **SGF** (normalized) | 60.7 (61.7) | 83.6 (85.0) | 96.1 (97.7) | 97.3 (98.9) | 97.8 (99.4) | 98.2 (99.8) | 96.6 (98.2) | 98.4 (100.0) |
| | **SGF/ EtOH** (normalized) | 53.3 (51.5) | 80.6 (77.9) | 96.5 (93.2) | 98.7 (95.3) | 99.9 (96.5) | 100.3 (96.9) | 102.0 (98.6) | 103.5 (100.0) |
| **36** | **SGF** (normalized) | 75.4 (75.3) | 94.4 (94.3) | 98.4 (98.3) | 98.9 (98.8) | 99.1 (99.0) | 99.5 (99.4) | 98.7 (98.6) | 100.1 (100.0) |
| | **SGF/ EtOH** (normalized) | 64.5 (60.8) | 92.2 (87.1) | 100.9 (95.3) | 101.7 (96.0) | 102.0 (96.3) | 103.1 (97.3) | 104.7 (98.8) | 105.9 (100.0) |

### EXAMPLES 37 to 41

### Composition

The compositions of the poly(ε-caprolactone) melt-extruded multi-particulates (MEMs) for Example 37 and comparative Examples 38 to 41 are summarized in Table XX below:

**Table XX**

| **Example Number** | **37** | **38** | **39** | **40** | **41** |
|---|---|---|---|---|---|
| **Ingredient** | **Amount (% w/w)** | | | | |
| Naltrexone HCl* | **15** | **15** | **15** | **15** | **15** |
| Poly(ε-caprolactone), **Mn** ~ **42,500** | **69** | **69** | **69** | **69** | **69** |
| Polyethylene oxide, Mw ~ 100,000 (PEO WSR N10) | **15** | - | - | - | - |
| Sodium Alginate | - | **15** | - | - | - |
| Pectin | - | - | | **15** | - |
| Agar | - | - | **15** | - | - |
| Hydroxy ethyl methyl cellulose | - | - | - | - | **15** |
| Butylated Hydroxy Toluene (BHT) | **1** | **1** | **1** | **1** | **1** |
| Total | **100** | **100** | **100** | **100** | **100** |

| | | | | | |
|---|---|---|---|---|---|
| * Amount not corrected for water or impurities. | | | | | |

The manufacturing procedure for Examples 37 to 41 corresponds to the manufacturing procedure for Examples 19 to 36.

The dissolution results for Examples 37-41 MEMs in capsules are summarized in Figure 25 and Table 25.

**Table 25**

| **Ex.** | **Dissolution Media** | **Mean Naltrexone HCl % Released (n=2)*** | | | | | | | |
|---|---|---|---|---|---|---|---|---|---|
| | | **30 min** | **60 min** | **120 min** | **240 min** | **360 min** | **480 min** | **720 min** | **1080 min** |
| **37** | **SGF** | 24.2 | 36.6 | 54.7 | 77.4 | 911.4 | 100.0 | 105.9 | 107.1 |
| | **SGF/ EtOH** | 28.0 | 44.4 | 65.0 | 87.3 | 97.9 | 103.4 | 105.7 | 106.6 |
| **38** | **SGF** | - | 9.8 | 13.8 | 19.8 | - | 29.1 | 36.5 | 46.5 |
| | **SGF/ EtOH** | - | 20.1 | 28.8 | 39.3 | - | 54.6 | 66.5 | 76.2 |
| **39** | **SGF** | - | 9.2 | 12.5 | 18.9 | - | 27.2 | 34.1 | 43.4 |
| | **SGF/ EtOH** | - | 20.4 | 31.2 | 44.3 | - | 62.6 | 74.3 | 85.5 |
| **40** | **SGF** | - | 9.9 | 13.5 | 19.1 | - | 27.3 | 33.5 | 41.3 |
| | **SGF/ EtOH** | - | 20.0 | 28.5 | 39.6 | - | 56.1 | 67.7 | 79.1 |
| **41** | **SGF** | - | 9.3 | 12.5 | 17.9 | - | 25.5 | 31.4 | 38.9 |
| | **SGF/ EtOH** | - | 21.8 | 32.6 | 46.4 | - | 65.4 | 77.1 | 89.6 |

Figure 25 shows that polyethylene oxide is superior to the other tested materials (Sodium Alginate, Pectin, Agar and Hydroxy ethyl methyl cellulose) with respect to providing alcohol resistance.

## Claims

1. A solid extended release pharmaceutical dosage form, comprising a mixture in the form of an extended release matrix formulation, the mixture comprising :
(1) at least one poly(ε-caprolactone) having an approximate number average molecular weight of from about 10,000 to about 200,000, and
(2) at least one polyethylene oxide having an approximate weight average molecular weight of from about 10,000 to less than 1,000,000, and
(3) at least one active agent.

2. The solid extended release pharmaceutical dosage form according to claim 1, wherein the poly(ε-caprolactone) has an approximate number average molecular weight of from about 45,000 to about 200,000, or from about 105,000 to about 200,000.

3. The solid extended release pharmaceutical dosage form according to any one of claim 1 or 2, wherein the poly(ε-caprolactone) is present at an amount of from about 40 weight-% to about 85 weight-% or at an amount of less than 50 weight-% of the extended release matrix formulation.

4. The solid extended release pharmaceutical dosage form according to any one of claims 1 to 3, wherein the polyethylene oxide has an approximate weight average molecular weight of from about 40,000 to less than 1,000,000, or from about 50,000 to about 300,000, or from about 50,000 to about 200,000.

5. The solid extended release pharmaceutical dosage form according to any one of claims 1 to 4, wherein polyethylene oxide is present at an amount of at least about 10 weight-%, or at least about 13 weight-%, or at least about 15 weight-%, or at least about 20 weight-%, or at least about 25 weight-%, or at least about 30 weight-% of the extended release matrix formulation or wherein polyethylene oxide is present at an amount of from about 10 weight-% to about 40 weight-%, or from about 13 weight-% to about 40 weight-%, or from about 15 weight-% to about 40 weight-%, or from about 20 weight-% to about 40 weight-%, or from about 25 weight-% to about 40 weight-%, or from about 30 weight-% to about 40 weight-%, or from about 15 weight-% to about 35 weight-% of the extended release matrix formulation.

6. The solid extended release pharmaceutical dosage form according to any one of claims 1 to 5, wherein the extended release matrix formulation comprises at least one further retardant, preferably selected from the group of long chain (C₈-C₅₀) substituted or unsubstituted hydrocarbons such as fatty acids, fatty alcohols, glyceryl esters of fatty acids such as glyceryl behenate, mineral and vegetable oils and waxes.

7. The solid extended release pharmaceutical dosage form of claim 6, wherein the retardant is present at an amount of from about 0.1 weight-% to 10 weight-% of the extended release matrix formulation, preferably wherein the retardant is glyceryl behenate and is present at an amount of from about 2 weight-% to 7 weight-%.

8. The solid extended release pharmaceutical dosage form according to any one of claims 1 to 7, wherein the active agent is present at an amount of at least about 10 weight-%, or at least about 12.5 weight-%, or at least about 15 weight-% of the extended release matrix formulation or wherein the active agent is present at an amount of from about 10 weight-% to about 30 weight-%, or from about 10 weight-% to about 25 weight-%, or from about 12.5 weight-% to about 25 weight-% of the extended release matrix formulation.

9. The solid extended release pharmaceutical dosage form according to any one of claims 1 to 8, wherein active agent is an opioid analgesic selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, the pharmaceutically acceptable salts, hydrates, solvates, and mixtures of any of the foregoing, and is preferably selected from the group consisting of codeine, morphine, oxycodone, hydrocodone, hydromorphone, oxymorphone, the pharmaceutically acceptable salts, hydrates, solvates, and mixtures of any of the foregoing.

10. The solid extended release pharmaceutical dosage form according to claim 9, wherein the opioid analgesic is oxycodone or a pharmaceutically acceptable salt thereof, preferably oxycodone hydrochloride, and wherein the dosage form preferably comprises from about 5 mg to about 500 mg of oxycodone hydrochloride such as 5 mg, 7.5 mg, 10 mg, 15 mg, 20 mg, 30, mg, 40 mg, 45 mg, 50 mg, 60 mg, or 80 mg, 90 mg, 100 mg, 120 mg or 160 mg of oxycodone hydrochloride and wherein the oxycodone hydrochloride is preferably present at an amount of more than 15 weight-% of the extended release matrix formulation.

11. The solid extended release pharmaceutical dosage form of claim 9, wherein the opioid analgesic is oxymorphone or a pharmaceutically acceptable salt thereof, preferably oxymorphone hydrochloride, and wherein the dosage form preferably comprises from about 1 mg to about 500 mg of oxymorphone hydrochloride such as 5 mg, 7.5 mg, 10 mg, 15 mg, 20 mg, 30 mg, 40 mg, 45 mg, 50 mg 60 mg, or 80 mg, 90 mg, 100 mg, 120 mg or 160 mg of oxymorphone hydrochloride.

12. The solid extended release pharmaceutical dosage form of claim 9, wherein the opioid analgesic is hydromorphone or a pharmaceutically acceptable salt thereof, preferably hydromorphone hydrochloride, and wherein the dosage form preferably comprises from about 1 mg to about 100 mg of hydromorphone hydrochloride such as 2 mg, 4 mg, 5 mg, 8 mg, 12 mg, 15 mg, 16 mg, 24 mg, 25 mg, 32 mg, 48 mg, 50 mg, 64 mg or 75 mg of hydromorphone hydrochloride.

13. The solid extended release pharmaceutical dosage form according to any one of claims 1 to 8, wherein the active agent is an opioid antagonist, preferably selected from the group consisting of naloxone, naltrexone, nalmephene, and pharmaceutically acceptable salts, hydrates solvates, and mixtures of any of the foregoing.

14. The solid extended release pharmaceutical dosage form of claim 13, wherein the opioid antagonist is naltrexone hydrochloride and the dosage form comprises from about 1 mg to about 100 mg of naltrexone hydrochloride such as 2.5 mg, 5 mg, 10 mg, 15 mg, 20 mg, 30 mg, 40 mg, 50 mg or 60 mg of naltrexone hydrochloride and wherein the naltrexone hydrochloride is preferably present at an amount of at least about 10 weight-% of the extended release matrix formulation.

15. The solid extended release pharmaceutical dosage form according to any one of claims 1 to 14, wherein the extended release matrix formulation is in multi particulate form, preferably wherein the multi-particulates have a diameter in the range of from about 0.1 to about 5 mm, or from about 0.1 to about 2 mm, or from about 0.5 to about 2 mm, or from about 2 to about 5 mm.

16. The solid extended release pharmaceutical dosage form according to claim 15, wherein the multi-particulates are disposed in a capsule.

17. The solid extended release pharmaceutical dosage form according to any one of claims 1 to 14, wherein the extended release matrix formulation is in the form of a tablet.

18. The solid extended release pharmaceutical dosage form according to any one of claims 1 to 17, wherein the extended release matrix formulation is thermo-treated preferably cured, or melt-formed such as shaped by a melt extrusion method, a casting method, an injection molding method or by direct compression with simultaneous application of elevated temperature, or
wherein the extended release matrix formulation is shaped by direct compression.

19. The solid extended release pharmaceutical dosage form according to any one of claims 1 to 18, wherein the dosage form provides release rates of the active agent in-vitro when measured by the USP Basket Method at 100 rpm at 900 ml simulated gastric fluid at 37 °C, from about 12.5 % to about 55 % (by wt) active agent released after 60 minutes, from about 25 % to about 65 % (by wt) active agent released after 120 minutes, from about 45 % to about 85 % (by wt) active agent released after 240 minutes, and from about 55 % to about 95 % (by wt) active agent released after 360 minutes.

20. The solid extended release pharmaceutical dosage form according to any one of claims 1 to 19, wherein the dosage form provides an in-vitro dissolution rate of the active agent, when measured by the USP Basket Method at 100 rpm at 900 ml simulated gastric fluid at 37 °C, from about 10 % to about 30 % (by wt) active agent released after 30 minutes, from about 20 % to about 50 % (by wt) active agent released after 60 minutes, from about 30 % to about 65 % (by wt) active agent released after 120 minutes, from about 45 % to about 85 % (by wt) active agent released after 240 minutes, and from about 60 % to about 95 % (by wt) active agent released after 360 minutes.

21. The solid extended release pharmaceutical dosage form according to any one of claim 19 or 20, wherein the active agent is oxycodone hydrochloride, or hydromorphone hydrochloride, or oxymorphone hydrochloride.

22. The solid extended release pharmaceutical dosage form according to any one of claims 1 to 21, wherein the dosage form is resistant to alcohol extraction.

23. The solid extended release pharmaceutical dosage form according to any one of claims 1 to 22, providing an in-vitro dissolution rate, when measured in a USP Apparatus 1 (basket) at 100 rpm in 900 ml simulated gastric fluid (SGF) comprising 40% ethanol at 37° C, **characterized by** the percent amount of active agent released at 30 minutes, or at 60 minutes, or at 120 minutes, or at 240 minutes, or at 360 minutes of dissolution that deviates no more than 20 % points, or no more than 15 % points, or no more than 10 % points, or no more than 5 % points from the corresponding in-vitro dissolution rate measured in a USP Apparatus 1 (basket) at 100 rpm in 900 ml simulated gastric fluid (SGF) at 37° C without ethanol.

24. The solid extended release pharmaceutical dosage form according to any one of claim 19 or 23, wherein the active agent is oxycodone hydrochloride, or hydromorphone hydrochloride, or oxymorphone hydrochloride, or naltrexone hydrochloride and / or
wherein the extended release matrix formulation further comprises at least one retardant.

25. The solid extended release pharmaceutical dosage form according to any one of claims 1 to 24, wherein the dosage form is resistant to crushing,
preferably wherein the dosage form, after crushing for 10 seconds in a coffee mill, provides an amount of material retained by a mesh #30 of at least about 85 %, or at least about 90 %, or at least about 95 % of the initial amount of the dosage form and / or
wherein the dosage form, after crushing for 20 seconds in a coffee mill, provides an amount of material retained by a mesh #30 of at least about 75 %, or at least about 80 %, or at least about 85 %, or at least about 90 % of the initial amount of the dosage form and / or
wherein the dosage form, after crushing for 30 seconds in a coffee mill, provides an amount of material retained by a mesh #30 of at least about 65 %, or at least about 70 %, or at least about 80 %, or at least about 85 % of the initial amount of the dosage form and / or
wherein the dosage form, after crushing for 40 seconds in a coffee mill, provides an amount of material retained by a mesh #30 of at least about 60 %, or at least about 65 %, or at least about 70 %, or at least about 75 %, or at least about 80 % of the initial amount of the dosage form and / or
wherein the dosage form, after crushing for 50 seconds in a coffee mill, provides an amount of material retained by a mesh #30 of at least about 55 %, or at least about 60 %, or at least about 70 %, or at least about 75 % of the initial amount of the dosage form and / or
wherein the dosage form, after crushing for 60 seconds in a coffee mill, provides an amount of material retained by a mesh #30 of at least about 45 %, or at least about 55 %, or at least about 65 %, or at least about 70 %, or at least about 75 %, or at least about 80 %, or at least about 85 % of the initial amount of the dosage form.

26. A process of preparing the solid extended release pharmaceutical dosage form according to any one of claims 1 to 25 comprising the steps of:
1. combining the poly(ε-caprolactone), the polyethylene oxide, the active agent, and optionally one or more other ingredients to form a blend;
2. feeding the blend from step 1 into a single-screw volumetric dispenser;
3. metering the blend from the dispenser into a twin screw extruder and processing the blend at elevated temperature into strands;
4. drawing the strands from step 3 from the extruder and cooling the strands; and
5. pelletizing the cooled strands from step 4 by cutting them into pellets; or providing slices by cutting the cooled strands from step 4 into tablet slices with a blade,
preferably further comprising the steps of:
6. metering the pellets from step 5 into a twin screw extruder and processing them at elevated temperature into strands;
7. drawing and cooling the strands; and
8. pelletizing the cooled strands by cutting into pellets,
preferably wherein the poly(ε-caprolactone) is used in step 1 in the form of flakes or milled material having a diameter of less than or equal to 840 µm.

27. A process of preparing the solid extended release pharmaceutical dosage form according to any one of claims 1 to 25, comprising the steps of:
1. blending the polyethylene oxide, the active agent and optionally one or more other ingredients, except the poly(ε-caprolactone), to form a first composition;
2. feeding the first composition of step 1 to a first hopper of a first volumetric dispenser fitted with a first single-screw assembly;
3. feeding poly(ε-caprolactone) as a second composition to a second hopper of a second volumetric dispenser fitted with a second screw assembly larger than the first screw assembly;
4. calibrating the feed rate of the two dispensers according to the relative proportion of the first and second composition to obtain a total feed rate;
5. metering the first and second compositions into a twin screw extruder and processing the resulting extrudate at elevated temperature into strands;
6. drawing and cooling the strands from step 5; and
7. pelletizing the cooled strands from step 6 by cutting them into pellets.

28. A solid extended release pharmaceutical dosage form obtainable by a process according to any one of claim 26 or 27.

29. A solid extended release pharmaceutical dosage form according to any one of claims 1 to 25 for use in the treatment of pain, wherein the active agent is an opioid analgesic.

30. Use of polyethylene oxide having an approximate weight average molecular weight of from about 10,000 to less than 1,000,000 in an extended release matrix formulation in a solid extended release pharmaceutical dosage form, wherein the extended release matrix formulation further comprises an active agent and poly(ε-caprolactone) for imparting to the solid extended release dosage form resistance to alcohol extraction.

31. Use of poly(ε-caprolactone) having an approximate number average molecular weight of from about 10,000 to about 200,000 in an extended release matrix formulation in a solid extended release pharmaceutical dosage form, wherein the extended release matrix formulation further comprises an active agent and polyethylene oxide having an approximate weight average molecular weight of from about 10,000 to less than 1,000,000 for imparting to the solid extended release dosage form resistance to crushing.

## Patentansprüche

1. Feste pharmazeutische Darreichungsform mit verzögerter Freisetzung, umfassend ein Gemisch in der Form einer Matrix-Formulierung mit verzögerter Freisetzung, wobei das Gemisch Folgendes umfasst
(1) wenigstens ein Poly(ε-caprolacton) mit einem ungefähren zahlenmittleren Molekulargewicht von etwa 10.000 bis etwa 200.000, und
(2) wenigstens ein Polyethylenoxid mit einem ungefähren gewichtsmittleren Molekulargewicht von etwa 10.000 bis weniger als 1.000.000, und
(3) wenigstens einen Wirkstoff.

2. Feste pharmazeutische Darreichungsform mit verzögerter Freisetzung nach Anspruch 1, wobei das Poly(ε-caprolacton) ein ungefähres zahlenmittleres Molekulargewicht von etwa 45.000 bis etwa 200.000 oder von etwa 105.000 bis etwa 200.000 aufweist.

3. Feste pharmazeutische Darreichungsform mit verzögerter Freisetzung nach einem der Ansprüche 1 oder 2, wobei das Poly(ε-caprolacton) in einer Menge von etwa 40 Gew.-% bis etwa 85 Gew.-% oder in einer Menge von weniger als 50 Gew.-% der Matrix-Formulierung mit verzögerter Freisetzung vorliegt.

4. Feste pharmazeutische Darreichungsform mit verzögerter Freisetzung nach einem der Ansprüche 1 bis 3, wobei das Polyethylenoxid ein ungefähres zahlenmittleres Molekulargewicht von etwa 40.000 bis weniger als 1,000,000 oder von etwa 50.000 bis etwa 300.000 oder von etwa 50.000 bis etwa 200.000 aufweist.

5. Feste pharmazeutische Darreichungsform mit verzögerter Freisetzung nach einem der Ansprüche 1 bis 4, wobei Polyethylenoxid in einer Menge von wenigstens etwa ES:AF:JS 10 Gew.-% oder wenigstens etwa 13 Gew.-% oder wenigstens etwa 15 Gew.-% oder wenigstens etwa 20 Gew.-% oder wenigstens etwa 25 Gew.-% oder wenigstens etwa 30 Gew.-% der Matrix-Formulierung mit verzögerter Freisetzung vorliegt, oder wobei Polyethylenoxid in einer Menge von etwa 10 Gew.-% bis etwa 40 Gew.-% oder von etwa 13 Gew.-% bis etwa 40 Gew.-% oder von etwa 15 Gew.-% bis etwa 40 Gew.-% oder von etwa 20 Gew.-% bis etwa 40 Gew.-% oder von etwa 25 Gew.-% bis etwa 40 Gew.-% oder von etwa 30 Gew.-% bis etwa 40 Gew.-% oder von etwa 15 Gew.-% bis etwa 35 Gew.-% der Matrix-Formulierung mit verzögerter Freisetzung vorliegt.

6. Feste pharmazeutische Darreichungsform mit verzögerter Freisetzung nach einem der Ansprüche 1 bis 5, wobei die Matrix-Formulierung mit verzögerter Freisetzung wenigstens ein weiteres Verzögerungsmittel umfasst, vorzugsweise ausgewählt aus der Gruppe langkettiger (C₈-C₅₀) substituierter oder unsubstituierter Kohlenwasserstoffe wie etwa Fettsäuren, Fettalkohole, Glycerylester von Fettsäuren wie etwa Glycerylbehenat, Mineral- und Pflanzenöle oder -wachse.

7. Feste pharmazeutische Darreichungsform mit verzögerter Freisetzung nach Anspruch 6, wobei das Verzögerungsmittel in einer Menge von etwa 0,1 Gew.-% bis 10 Gew.-% der Matrix-Formulierung mit verzögerter Freisetzung vorliegt, wobei das Verzögerungsmittel vorzugsweise Glycerylbehenat ist und in einer Menge von etwa 2 Gew.-% bis 7 Gew.-% vorliegt.

8. Feste pharmazeutische Darreichungsform mit verzögerter Freisetzung nach einem der Ansprüche 1 bis 7, wobei der Wirkstoff in einer Menge von wenigstens etwa 10 Gew.-% oder wenigstens etwa 12.5 Gew.-% oder wenigstens etwa 15 Gew.-% der Matrix-Formulierung mit verzögerter Freisetzung vorliegt oder wobei der Wirkstoff in einer Menge von etwa 10 Gew.-% bis etwa 30 Gew.-% oder von etwa 10 Gew.-% bis etwa 25 Gew.-% oder von etwa 12.5 Gew.-% bis etwa 25 Gew.-% der Matrix-Formulierung mit verzögerter Freisetzung vorliegt.

9. Feste pharmazeutische Darreichungsform mit verzögerter Freisetzung nach einem der Ansprüche 1 bis 8, wobei der Wirkstoff ein Opioidanalgetikum ist, ausgewählt aus der Gruppe bestehend aus Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl und Derivaten, Hydrocodon, Hydromorphone, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Papaveretum, Pentazocin, Phenadoxon, Phenomorphan, Phenazocin, Phenoperidin, Piminodin, Piritramide, Propheptazin, Promedol, Properidin, Propoxyphen, Sufentanil, Tilidin, Tramadol, den pharmazeutisch verträglichen Salzen, Hydraten, Solvaten und Gemischen beliebiger der Vorstehenden, und vorzugsweise ausgewählt ist aus der Gruppe bestehend aus Codein, Morphin, Oxycodon, Hydrocodon, Hydromorphon, Oxymorphon, den pharmazeutisch verträglichen Salzen, Hydraten, Solvaten und Gemischen beliebiger der Vorstehenden.

10. Feste pharmazeutische Darreichungsform mit verzögerter Freisetzung nach Anspruch 9, wobei das Opioidanalgetikum Oxycodon oder ein pharmazeutisch verträgliches Salz davon ist, vorzugsweise Oxycodonhydrochlorid, und wobei die Darreichungsform vorzugsweise etwa 5 mg bis etwa 500 mg Oxycodonhydrochlorid umfasst, wie etwa 5 mg, 7,5 mg, 10 mg, 15 mg, 20 mg, 30, mg, 40 mg, 45 mg, 50 mg, 60 mg oder 80 mg, 90 mg, 100 mg, 120 mg oder 160 mg Oxycodonhydrochlorid und wobei das Oxycodonhydrochlorid vorzugsweise in einer Menge von mehr als 15 Gew.-% der Matrix-Formulierung mit verzögerter Freisetzung vorliegt.

11. Feste pharmazeutische Darreichungsform mit verzögerter Freisetzung nach Anspruch 9, wobei das Opioidanalgetikum Oxymorphon oder ein pharmazeutisch verträgliches Salz davon ist, vorzugsweise Oxymorphonhydrochlorid, und wobei die Darreichungsform vorzugsweise etwa 1 mg bis etwa 500 mg Oxymorphonhydrochlorid umfasst, wie etwa 5 mg, 7,5 mg, 10 mg, 15 mg, 20 mg, 30 mg, 40 mg, 45 mg, 50 mg 60 mg oder 80 mg, 90 mg, 100 mg, 120 mg oder 160 mg Oxymorphonhydrochlorid.

12. Feste pharmazeutische Darreichungsform mit verzögerter Freisetzung nach Anspruch 9, wobei das Opioidanalgetikum Hydromorphon oder ein pharmazeutisch verträgliches Salz davon ist, vorzugsweise Hydromorphonhydrochlorid, und wobei die Darreichungsform vorzugsweise etwa 1 mg bis etwa 100 mg Hydromorphonhydrochlorid umfasst, wie etwa 2 mg, 4 mg, 5 mg, 8 mg, 12 mg, 15 mg, 16 mg, 24 mg, 25 mg, 32 mg, 48 mg, 50 mg, 64 mg oder 75 mg Hydromorphonhydrochlorid.

13. Feste pharmazeutische Darreichungsform mit verzögerter Freisetzung nach einem der Ansprüche 1 bis 8, wobei der Wirkstoff ein Opioidantagonist ist, vorzugsweise ausgewählt aus der Gruppe bestehend aus Naloxon, Naltrexon, Nalmephen und pharmazeutisch verträglichen Salzen, Hydraten, Solvaten und Gemischen beliebiger der Vorstehenden.

14. Feste pharmazeutische Darreichungsform mit verzögerter Freisetzung nach Anspruch 13, wobei der Opioidantagonist Naltrexonhydrochlorid ist und die Darreichungsform etwa 1 mg bis etwa 100 mg Naltrexonhydrochlorid umfasst, wie etwa 2,5 mg, 5 mg, 10 mg, 15 mg, 20 mg, 30 mg, 40 mg, 50 mg oder 60 mg Naltrexonhydrochlorid, und wobei das Naltrexonhydrochlorid vorzugsweise in einer Menge von wenigstens etwa 10 Gew.-% der Matrix-Formulierung mit verzögerter Freisetzung vorliegt.

15. Feste pharmazeutische Darreichungsform mit verzögerter Freisetzung nach einem der Ansprüche 1 bis 14, wobei die Matrix-Formulierung mit verzögerter Freisetzung in Multipartikelform vorliegt, wobei die Multipartikel vorzugsweise einen Durchmesser im Bereich von etwa 0,1 bis etwa 5 mm oder von etwa 0,1 bis etwa 2 mm oder von etwa 0,5 bis etwa 2 mm oder von etwa 2 bis etwa 5 mm aufweisen.

16. Feste pharmazeutische Darreichungsform mit verzögerter Freisetzung nach Anspruch 15, wobei die Multipartikel in einer Kapsel angeordnet sind.

17. Feste pharmazeutische Darreichungsform mit verzögerter Freisetzung nach einem der Ansprüche 1 bis 14, wobei die Matrix-Formulierung mit verzögerter Freisetzung in Form einer Tablette vorliegt.

18. Feste pharmazeutische Darreichungsform mit verzögerter Freisetzung nach einem der Ansprüche 1 bis 17, wobei die Matrix-Formulierung mit verzögerter Freisetzung wärmebehandelt, vorzugsweise gehärtet wird, oder schmelzgeformt wird, wie etwa geformt durch ein Schmelzextrusionsverfahren, ein Gießverfahren, ein Spritzgießverfahren oder durch direkte Komprimierung mit gleichzeitiger Anwendung erhöhter Temperatur, oder
wobei die Matrix-Formulierung mit verzögerter Freisetzung durch direkte Komprimierung geformt wird.

19. Feste pharmazeutische Darreichungsform mit verzögerter Freisetzung nach einem der Ansprüche 1 bis 18, wobei die Darreichungsform Freisetzungsraten des Wirkstoffs in vitro, bei Messung nach der USP-Korbmethode bei 100 U/min mit 900 ml simuliertem Magensaft bei 37 °C, von etwa 12,5 % bis etwa 55 % (nach Gewicht) freigesetztem Wirkstoff nach 60 Minuten, von etwa 25 % bis etwa 65 % (nach Gewicht) freigesetztem Wirkstoff nach 120 Minuten, von etwa 45 % bis etwa 85 % (nach Gewicht) freigesetztem Wirkstoff nach 240 Minuten und von etwa 55 % bis etwa 95 % (nach Gewicht) freigesetztem Wirkstoff nach 360 Minuten bereitstellt.

20. Feste pharmazeutische Darreichungsform mit verzögerter Freisetzung nach einem der Ansprüche 1 bis 19, wobei die Darreichungsform eine In-vitro-Auflösungsrate des Wirkstoffs bei Messung nach der USP-Korbmethode bei 100 U/min mit 900 ml simuliertem Magensaft bei 37 °C von etwa 10 % bis etwa 30 % (nach Gewicht) freigesetztem Wirkstoff nach 30 Minuten, von etwa 20 % bis etwa 50 % (nach Gewicht) freigesetztem Wirkstoff nach 60 Minuten, von etwa 30 % bis etwa 65 % (nach Gewicht) freigesetztem Wirkstoff nach 120 Minuten, von etwa 45 % bis etwa 85 % (nach Gewicht) freigesetztem Wirkstoff nach 240 Minuten und von etwa 60 % bis etwa 95 % (nach Gewicht) freigesetztem Wirkstoff nach 360 Minuten bereitstellt.

21. Feste pharmazeutische Darreichungsform mit verzögerter Freisetzung nach einem der Ansprüche 19 oder 20, wobei der Wirkstoff Oxycodonhydrochlorid oder Hydromorphonhydrochlorid oder Oxymorphonhydrochlorid ist.

22. Feste pharmazeutische Darreichungsform mit verzögerter Freisetzung nach einem der Ansprüche 1 bis 21, wobei die Darreichungsform gegenüber Alkoholextraktion beständig ist.

23. Feste pharmazeutische Darreichungsform mit verzögerter Freisetzung nach einem der Ansprüche 1 bis 22, die eine In-vitro-Auflösungsrate bei Messung in einer USP-Vorrichtung 1 (Korb) bei 100 U/min in 900 ml simuliertem Magensaft (SGF) umfassend 40 % Ethanol bei 37 °C bereitstellt, die **gekennzeichnet ist durch** die prozentuale Menge an Wirkstoff, die bei 30 Minuten oder bei 60 Minuten oder bei 120 Minuten oder bei 240 Minuten oder bei 360 Minuten der Auflösung freigesetzt wird, die um nicht mehr als 20 % Punkte oder nicht mehr als 15 % Punkte oder nicht mehr als 10 % Punkte oder nicht mehr als 5 % Punkte von der entsprechenden In-vitro-Auflösungsrate abweicht, die in einer USP-Vorrichtung 1 (Korb) bei 100 U/min in 900 ml simuliertem Magensaft (SGF) bei 37 °C ohne Ethanol gemessen wird.

24. Feste pharmazeutische Darreichungsform mit verzögerter Freisetzung nach einem der Ansprüche 19 oder 23, wobei der Wirkstoff Oxycodonhydrochlorid oder Hydromorphonhydrochlorid oder Oxymorphonhydrochlorid oder Naltrexonhydrochlorid ist, und/oder
wobei die Matrix-Formulierung mit verzögerter Freisetzung ferner wenigstens ein Verzögerungsmittel umfasst.

25. Feste pharmazeutische Darreichungsform mit verzögerter Freisetzung nach einem der Ansprüche 1 bis 24, wobei die Darreichungsform gegenüber Zerkleinern beständig ist, wobei die Darreichungsform vorzugsweise nach Zerkleinern für 10 Sekunden in einer Kaffeemühle eine Materialmenge, die von einem Gewebe #30 zurückgehalten wird, von etwa 85 % oder wenigstens etwa 90 % oder wenigstens etwa 95 % der Anfangsmenge der Darreichungsform bereitstellt, und/oder
wobei die Darreichungsform nach Zerkleinern für 20 Sekunden in einer Kaffeemühle eine Materialmenge, die von einem Gewebe #30 zurückgehalten wird, von wenigstens etwa 75 % oder wenigstens etwa 80 % oder wenigstens etwa 85 % oder wenigstens etwa 90 % der Anfangsmenge der Darreichungsform bereitstellt, und/oder
wobei die Darreichungsform nach Zerkleinern für 30 Sekunden in einer Kaffeemühle eine Materialmenge, die von einem Gewebe #30 zurückgehalten wird, von wenigstens etwa 65 % oder wenigstens etwa 70 % oder wenigstens etwa 80 % oder wenigstens etwa 85 % der Anfangsmenge der Darreichungsform bereitstellt, und/oder
wobei die Darreichungsform nach Zerkleinern für 40 Sekunden in einer Kaffeemühle eine Materialmenge, die von einem Gewebe #30 zurückgehalten wird, von wenigstens etwa 60 % oder wenigstens etwa 65 % oder wenigstens etwa 70 % oder wenigstens etwa 75 % oder wenigstens etwa 80 % der Anfangsmenge der Darreichungsform bereitstellt, und/oder
wobei die Darreichungsform nach Zerkleinern für 50 Sekunden in einer Kaffeemühle eine Materialmenge, die von einem Gewebe #30 zurückgehalten wird, von wenigstens etwa 55 % oder wenigstens etwa 60 % oder wenigstens etwa 70 % oder wenigstens etwa 75 % der Anfangsmenge der Darreichungsform bereitstellt, und/oder
wobei die Darreichungsform nach Zerkleinern für 60 Sekunden in einer Kaffeemühle eine Materialmenge, die von einem Gewebe #30 zurückgehalten wird, von wenigstens etwa 45 % oder wenigstens etwa 55 % oder wenigstens etwa 65 % oder wenigstens etwa 70 % oder wenigstens etwa 75 % oder wenigstens etwa 80 % oder wenigstens etwa 85 % der Anfangsmenge der Darreichungsform bereitstellt.

26. Verfahren zum Herstellen der festen pharmazeutischen Darreichungsform mit verzögerter Freisetzung nach einem der Ansprüche 1 bis 25, folgende Schritte umfassend:
1. Vereinigen des Poly(ε-caprolactons), des Polyethylenoxids, des Wirkstoffs und wahlweise eines oder mehrerer weiterer Zutaten, um eine Mischung zu bilden;
2. Zuführen der Mischung aus Schritt 1 in einen volumetrischen Spender mit einzelner Schnecke;
3. Abmessen der Mischung vom Spender in einen Doppelschneckenextruder und Verarbeiten der Mischung bei erhöhter Temperatur zu Strängen;
4. Ziehen der Stränge aus Schritt 3 aus dem Extruder und Abkühlen der Stränge; und
5. Pelletieren der abgekühlten Stränge aus Schritt 4 durch Schneiden derselben in Pellets; oder Bereitstellen von Scheiben durch Schneiden der abgekühlten Stränge aus Schritt 4 in Tablettenscheiben mit einer Klinge,
vorzugsweise ferner folgende Schritte umfassend:
6. Abmessen der Pellets aus Schritt 5 in einen Doppelschneckenextruder und Verarbeiten derselben bei erhöhter Temperatur zu Strängen;
7. Ziehen und Abkühlen der Stränge; und
8. Pelletieren der abgekühlten Stränge durch Schneiden in Pellets,
wobei das Poly(ε-caprolacton) in Schritt 1 vorzugsweise in der Form von Flocken oder gemahlenem Material mit einem Durchmesser von weniger als oder gleich 840 µm verwendet wird.

27. Verfahren zum Herstellen der festen pharmazeutischen Darreichungsform mit verzögerter Freisetzung nach einem der Ansprüche 1 bis 25, folgende Schritte umfassend:
1. Mischen des Polyethylenoxids, des Wirkstoffs und wahlweise einer oder mehrerer weiterer Zutaten mit Ausnahme des Poly(ε-caprolactons), um eine erste Zusammensetzung zu bilden;
2. Zuführen der ersten Zusammensetzung aus Schritt 1 in einen ersten Trichter eines ersten volumetrischen Spenders, der mit einer ersten Einzelschneckenbaugruppe versehen ist;
3. Zuführen von Poly(ε-caprolacton) als zweite Zusammensetzung in einen zweiten Trichter eines zweiten volumetrischen Spenders, der mit einer zweiten Einzelschneckenbaugruppe versehen ist, die größer als die erste Einzelschneckenbaugruppe ist;
4. Kalibrieren der Zuführrate der zwei Spender gemäß der relativen Proportion der ersten und zweiten Zusammensetzung, um eine Gesamtzuführrate zu erlangen;
5. Abmessen der ersten und zweiten Zusammensetzung in einen Doppelschneckenextruder und Verarbeiten des resultierenden Extrudats bei erhöhter Temperatur zu Strängen;
6. Ziehen und Abkühlen der Stränge aus Schritt 5; und
7. Pelletieren der abgekühlten Stränge aus Schritt 6 durch Schneiden derselben in Pellets.

28. Feste pharmazeutische Darreichungsform mit verzögerter Freisetzung, die durch ein Verfahren nach einem der Ansprüche 26 oder 27 erlangt werden kann.

29. Feste pharmazeutische Darreichungsform mit verzögerter Freisetzung nach einem der Ansprüche 1 bis 25 zur Verwendung bei der Behandlung von Schmerz, wobei der Wirkstoff ein Opiodanalgetikum ist.

30. Verwendung von Polyethylenoxid mit einem ungefähren gewichtsmittleren Molekulargewicht von etwa 10.000 bis weniger als 1.000.000 in einer Matrix-Formulierung mit verzögerter Freisetzung in einer festen pharmazeutischen Darreichungsform mit verzögerter Freisetzung, wobei die Matrix-Formulierung mit verzögerter Freisetzung ferner einen Wirkstoff und Poly(ε-caprolacton) umfasst, um der festen pharmazeutischen Darreichungsform mit verzögerter Freisetzung Beständigkeit gegenüber Alkoholextraktion zu verleihen.

31. Verwendung von Poly(ε-caprolacton) mit einem ungefähren zahlenmittleren Molekulargewicht von etwa 10.000 bis etwa 200.000 in einer Matrix-Formulierung mit verzögerter Freisetzung in einer festen pharmazeutischen Darreichungsform mit verzögerter Freisetzung, wobei die Matrix-Formulierung mit verzögerter Freisetzung ferner einen Wirkstoff und Polyethylenoxid mit einem ungefähren gewichtsmittleren Molekulargewicht von etwa 10.000 bis weniger als 1.000.000 umfasst, um der festen pharmazeutischen Darreichungsform mit verzögerter Freisetzung Beständigkeit gegenüber Zerkleinern zu verleihen.

## Revendications

1. Forme posologique pharmaceutique solide à libération prolongée, comprenant un mélange sous la forme d'une formulation matricielle à libération prolongée, le mélange comprenant :
(1) au moins une poly(ε-caprolactone) ayant un poids moléculaire moyen approximatif en nombre d'environ 10 000 à environ 200 000, et
(2) au moins un oxyde de polyéthylène ayant un poids moléculaire moyen approximatif en poids d'environ 10 000 à moins de 1 000 000, et
(3) au moins un agent actif.

2. Forme posologique pharmaceutique solide à libération prolongée selon la revendication 1, dans laquelle la poly(ε-caprolactone) a un poids moléculaire moyen approximatif en nombre d'environ 45 000 à environ 200 000, ou d'environ 105 000 à environ 200 000.

3. Forme posologique pharmaceutique solide à libération prolongée selon l'une quelconque de la revendication 1 ou 2, dans laquelle la poly(ε-caprolactone) est présente en une quantité d'environ 40 % en poids à environ 85 % en poids ou en une quantité inférieure à 50 % en poids de la formulation matricielle à libération prolongée.

4. Forme posologique pharmaceutique solide à libération prolongée selon l'une quelconque des revendications 1 à 3, dans laquelle l'oxyde de polyéthylène a un poids moléculaire moyen approximatif en poids d'environ 40 000 à moins de 1 000 000, ou d'environ 50 000 à environ 300 000, ou d'environ 50 000 à environ 200 000.

5. Forme posologique pharmaceutique solide à libération prolongée selon l'une quelconque des revendications 1 à 4, dans laquelle l'oxyde de polyéthylène est présent en une quantité d'au moins environ 10 % en poids, ou d'au moins environ 13 % en poids, ou d'au moins environ 15 % en poids, ou d'au moins environ 20 % en poids, ou d'au moins environ 25 % en poids, ou d'au moins environ 30 % en poids de la formulation matricielle à libération prolongée ou dans laquelle l'oxyde de polyéthylène est présent en une quantité d'environ 10 % en poids à environ 40 % en poids, ou d'environ 13 % en poids à environ 40 % en poids, ou d'environ 15 % en poids à environ 40 % en poids, ou d'environ 20 % en poids à environ 40 % en poids, ou d'environ 25 % en poids à environ 40 % en poids, ou d'environ 30 % en poids à environ 40 % en poids, ou d'environ 15 % en poids à environ 35 % en poids de la formulation matricielle à libération prolongée.

6. Forme posologique pharmaceutique solide à libération prolongée selon l'une quelconque des revendications 1 à 5, dans laquelle la formulation matricielle à libération prolongée comprend au moins un autre retardateur, de préférence choisi dans le groupe constitué des hydrocarbures à longue chaîne (en C₈ à C₅₀), substitués ou non substitués, tels que les acides gras, les alcools gras, les esters glycéryliques d'acides gras tels que le béhénate de glycéryle, les huiles minérales et végétales et les cires.

7. Forme posologique pharmaceutique solide à libération prolongée selon la revendication 6, dans laquelle le retardateur est présent en une quantité d'environ 0,1 % en poids à 10 % en poids de la formulation matricielle à libération prolongée, de préférence dans laquelle le retardateur est le béhénate de glycéryle et est présent en une quantité d'environ 2 % en poids à 7 % en poids.

8. Forme posologique pharmaceutique solide à libération prolongée selon l'une quelconque des revendications 1 à 7, dans laquelle l'agent actif est présent en une quantité d'au moins environ 10 % en poids, ou d'au moins environ 12,5 % en poids, ou d'au moins environ 15 % en poids de la formulation matricielle à libération prolongée ou dans laquelle l'agent actif est présent en une quantité d'environ 10 % en poids à environ 30 % en poids, ou d'environ 10 % en poids à environ 25 % en poids, ou d'environ 12,5 % en poids à environ 25 % en poids de la formulation matricielle à libération prolongée.

9. Forme posologique pharmaceutique solide à libération prolongée selon l'une quelconque des revendications 1 à 8, dans laquelle l'agent actif est un analgésique opioïde choisi dans le groupe constitué de l'alfentanil, de l'allylprodine, de l'alphaprodine, de l'aniléridine, de la benzylmorphine, de la bézitramide, de la buprénorphine, du butorphanol, du clonitazène, de la codéine, de la désomorphine, du dextromoramide, de la dézocine, de la diampromide, de la diamorphone, de la dihydrocodéine, de la dihydromorphine, du diménoxadol, du dimépheptanol, du diméthylthiambutène, du butyrate de dioxaphétyl, de la dipipanone, de l'eptazocine, de l'éthoheptazine, de l'éthylméthylthiambutène, de l'éthylmorphine, de l'étonitazène, de l'éthorphine, de la dihydroéthorphine, du fentanyl et de ses dérivés, de l'hydrocodone, de l'hydromorphone, de l'hydroxypéthidine, de l'isométhadone, de la cétobémidone, du lévorphanol, du levophénacylmorphane, du lofentanil, de la mépéridine, du meptazinol, de la métazocine, de la méthadone, du métopon, de la morphine, de la myrophine, de la narcéine, de la nicomorphine, du norlévorphanol, de la norméthadone, de la nalorphine, du nalbuphène, de la normorphine, de la norpipanone, de l'opium, de l'oxycodone, de l'oxymorphone, du papaveretum, de la pentazocine, de la phénadoxone, du phénomorphane, de la phénazocine, de la phénopéridine, de la piminodine, de la piritramide, de la propheptazine, du promédol, de la propéridine, du propoxyphène, du sufentanil, de la tilidine, du tramadol, des sels, hydrates, solvates pharmaceutiquement acceptables, et des mélanges de composés quelconques parmi les précédents, et est de préférence choisi dans le groupe constitué de la codéine, de la morphine, de l'oxycodone, de l'hydrocodone, de l'hydromorphone, de l'oxymorphone, des sels, hydrates, solvates pharmaceutiquement acceptables, et des mélanges de composés quelconques parmi les précédents.

10. Forme posologique pharmaceutique solide à libération prolongée selon la revendication 9, dans laquelle l'analgésique opioïde est l'oxycodone ou un sel pharmaceutiquement acceptable de celle-ci, de préférence le chlorhydrate d'oxycodone, et dans laquelle la forme posologique comprend de préférence d'environ 5 mg à environ 500 mg de chlorhydrate d'oxycodone tel que 5 mg, 7,5 mg, 10 mg, 15 mg, 20 mg, 30, mg, 40 mg, 45 mg, 50 mg, 60 mg, ou 80 mg, 90 mg, 100 mg, 120 mg ou 160 mg de chlorhydrate d'oxycodone et dans laquelle le chlorhydrate d'oxycodone est de préférence présent en une quantité de plus de 15 % en poids de la formulation matricielle à libération prolongée.

11. Forme posologique pharmaceutique solide à libération prolongée selon la revendication 9, dans laquelle l'analgésique opioïde est l'oxymorphone ou un sel pharmaceutiquement acceptable de celle-ci, de préférence le chlorhydrate d'oxymorphone, et dans laquelle la forme posologique comprend de préférence d'environ 1 mg à environ 500 mg de chlorhydrate d'oxymorphone tel que 5 mg, 7,5 mg, 10 mg, 15 mg, 20 mg, 30 mg, 40 mg, 45 mg, 50 mg 60 mg, ou 80 mg, 90 mg, 100 mg, 120 mg ou 160 mg de chlorhydrate d'oxymorphone.

12. Forme posologique pharmaceutique solide à libération prolongée selon la revendication 9, dans laquelle l'analgésique opioïde est l'hydromorphone ou un sel pharmaceutiquement acceptable de celle-ci, de préférence le chlorhydrate d'hydromorphone, et dans laquelle la forme posologique comprend de préférence d'environ 1 mg à environ 100 mg de chlorhydrate d'hydromorphone tel que 2 mg, 4 mg, 5 mg, 8 mg, 12 mg, 15 mg, 16 mg, 24 mg, 25 mg, 32 mg, 48 mg, 50 mg, 64 mg ou 75 mg de chlorhydrate d'hydromorphone.

13. Forme posologique pharmaceutique solide à libération prolongée selon l'une quelconque des revendications 1 à 8, dans laquelle l'agent actif est un agoniste opioïde, de préférence choisi dans le groupe constitué de la naloxone, de la naltrexone, du nalméfène, et des sels, hydrates, solvates pharmaceutiquement acceptables, et des mélanges de composés quelconques parmi les précédents.

14. Forme posologique pharmaceutique solide à libération prolongée selon la revendication 13, dans laquelle l'agoniste opioïde est le chlorhydrate de naltrexone et la forme posologique comprend d'environ 1 mg à environ 100 mg de chlorhydrate de naltrexone tel que 2,5 mg, 5 mg, 10 mg, 15 mg, 20 mg, 30 mg, 40 mg, 50 mg ou 60 mg de chlorhydrate de naltrexone et dans laquelle le chlorhydrate de naltrexone est de préférence présent en une quantité d'au moins environ 10 % en poids de la formulation matricielle à libération prolongée.

15. Forme posologique pharmaceutique solide à libération prolongée selon l'une quelconque des revendications 1 à 14, dans laquelle la formulation matricielle à libération prolongée se présente sous une forme multiparticulaire, de préférence dans laquelle les multiparticules ont un diamètre dans la plage d'environ 0,1 à environ 5 mm, ou d'environ 0,1 à environ 2 mm, ou d'environ 0,5 à environ 2 mm, ou d'environ 2 à environ 5 mm.

16. Forme posologique pharmaceutique solide à libération prolongée selon la revendication 15, dans laquelle les multiparticules sont disposées dans une capsule.

17. Forme posologique pharmaceutique solide à libération prolongée selon l'une quelconque des revendications 1 à 14, dans laquelle la formulation matricielle à libération prolongée se présente sous la forme d'un comprimé.

18. Forme posologique pharmaceutique solide à libération prolongée selon l'une quelconque des revendications 1 à 17, dans laquelle la formulation matricielle à libération prolongée est traitée thermiquement, de préférence durcie, ou formée à l'état fondu, par exemple mise en forme par un procédé d'extrusion de matière fondue, un procédé de moulage, un procédé de moulage par injection ou par compression directe avec application simultanée d'une température élevée, ou
dans laquelle la formulation matricielle à libération prolongée est mise en forme par compression directe.

19. Forme posologique pharmaceutique solide à libération prolongée selon l'une quelconque des revendications 1 à 18, dans laquelle la forme posologique fournit des taux de libération de l'agent actif *in vitro* lorsqu'ils sont mesurés par la méthode du panier selon l'USP à 100 tr/min à 900 ml de liquide gastrique simulé à 37 °C, d'environ 12,5 % à environ 55 % (en poids) d'agent actif libéré après 60 minutes, d'environ 25 % à environ 65 % (en poids) d'agent actif libéré après 120 minutes, d'environ 45 % à environ 85 % (en poids) d'agent actif libéré après 240 minutes, et d'environ 55 % à environ 95 % (en poids) d'agent actif libéré après 360 minutes.

20. Forme posologique pharmaceutique solide à libération prolongée selon l'une quelconque des revendications 1 à 19, dans laquelle la forme posologique fournit un taux de dissolution *in vitro* de l'agent actif, lorsqu'il est mesuré par la méthode du panier selon l'USP à 100 tr/min à 900 ml de liquide gastrique simulé à 37 °C, d'environ 10 % à environ 30 % (en poids) d'agent actif libéré après 30 minutes, d'environ 20 % à environ 50 % (en poids) d'agent actif libéré après 60 minutes, d'environ 30 % à environ 65 % (en poids) d'agent actif libéré après 120 minutes, d'environ 45 % à environ 85 % (en poids) d'agent actif libéré après 240 minutes, et d'environ 60 % à environ 95 % (en poids) d'agent actif libéré après 360 minutes.

21. Forme posologique pharmaceutique solide à libération prolongée selon l'une quelconque de la revendication 19 ou 20, dans laquelle l'agent actif est le chlorhydrate d'oxycodone, ou le chlorhydrate d'hydromorphone, ou le chlorhydrate d'oxymorphone.

22. Forme posologique pharmaceutique solide à libération prolongée selon l'une quelconque des revendications 1 à 21, dans laquelle la forme posologique est résistante à l'extraction à l'alcool.

23. Forme posologique pharmaceutique solide à libération prolongée selon l'une quelconque des revendications 1 à 22, fournissant un taux de dissolution *in vitro,* lorsqu'il est mesuré dans un appareil USP 1 (panier) à 100 tr/min dans 900 ml de liquide gastrique simulé (LGS) comprenant 40 % d'éthanol à 37 °C, **caractérisé par** la quantité en pourcentage d'agent actif libéré après 30 minutes, ou après 60 minutes, ou après 120 minutes, ou après 240 minutes, ou après 360 minutes de dissolution, qui ne s'écarte pas plus de 20 % en points, ou de pas plus de 15 % en points, ou de pas plus de 10 % en points, ou de pas plus de 5 % en points par rapport au taux de dissolution correspondant *in vitro* mesuré dans un appareil USP 1 (panier) à 100 tr/min dans 900 ml de liquide gastrique simulé (LGS) à 37 °C sans éthanol.

24. Forme posologique pharmaceutique solide à libération prolongée selon l'une quelconque de la revendication 19 ou 23, dans laquelle l'agent actif est le chlorhydrate d'oxycodone, ou le chlorhydrate d'hydromorphone, ou le chlorhydrate d'oxymorphone, ou le chlorhydrate de naltrexone et/ou
dans laquelle la formulation matricielle à libération prolongée comprend en outre au moins un retardateur.

25. Forme posologique pharmaceutique solide à libération prolongée selon l'une quelconque des revendications 1 à 24, dans laquelle la forme posologique est résistante au broyage, de préférence dans laquelle la forme posologique, après broyage pendant 10 secondes dans un moulin à café, fournit une quantité de matière retenue par une maille n° 30 d'au moins environ 85 %, ou d'au moins environ 90 %, ou d'au moins environ 95 % de la quantité initiale de la forme posologique et/ou
dans laquelle la forme posologique, après broyage pendant 20 secondes dans un moulin à café, fournit une quantité de matière retenue par une maille n° 30 d'au moins environ 75 %, ou d'au moins environ 80 %, ou d'au moins environ 85 %, ou d'au moins environ 90 % de la quantité initiale de la forme posologique et/ou
dans laquelle la forme posologique, après broyage pendant 30 secondes dans un moulin à café, fournit une quantité de matière retenue par une maille n° 30 d'au moins environ 65 %, ou d'au moins environ 70 %, ou d'au moins environ 80 %, ou d'au moins environ 85 % de la quantité initiale de la forme posologique et/ou
dans laquelle la forme posologique, après broyage pendant 40 secondes dans un moulin à café, fournit une quantité de matière retenue par une maille n° 30 d'au moins environ 60 %, ou d'au moins environ 65 %, ou d'au moins environ 70 %, ou d'au moins environ 75 %, ou d'au moins environ 80 % de la quantité initiale de la forme posologique et/ou
dans laquelle la forme posologique, après broyage pendant 50 secondes dans un moulin à café, fournit une quantité de matière retenue par une maille n° 30 d'au moins environ 55 %, ou d'au moins environ 60 %, ou d'au moins environ 70 %, ou d'au moins environ 75 % de la quantité initiale de la forme posologique et/ou
dans laquelle la forme posologique, après broyage pendant 60 secondes dans un moulin à café, fournit une quantité de matière retenue par une maille n° 30 d'au moins environ 45 %, ou au moins d'environ 55 %, ou d'au moins environ 65 %, ou d'au moins environ 70 %, ou d'au moins environ 75 %, ou d'au moins environ 80 %, ou d'au moins environ 85 % de la quantité initiale de la forme posologique.

26. Procédé de préparation de la forme posologique pharmaceutique solide à libération prolongée selon l'une quelconque des revendications 1 à 25 comprenant les étapes suivantes :
1. combinaison de la poly(ε-caprolactone), de l'oxyde de polyéthylène, de l'agent actif, et facultativement d'un ou plusieurs autres ingrédients pour former un mélange ;
2. introduction du mélange de l'étape 1 dans un distributeur volumétrique à simple vis ;
3. dosage du mélange à partir du distributeur dans une extrudeuse à double vis et traitement du mélange à température élevée pour former des brins ;
4. étirage des brins de l'étape 3 à partir de l'extrudeuse et refroidissement des brins ; et
5. granulation des brins refroidis de l'étape 4 en les découpant en pastilles ; ou obtention de tranches en découpant les brins refroidis de l'étape 4 en tranches de comprimé à l'aide d'une lame,
de préférence comprenant en outre les étapes suivantes :
6. dosage des pastilles de l'étape 5 dans une extrudeuse à double vis et traitement de celles-ci à température élevée pour former des brins ;
7. étirage et refroidissement des brins ; et
8. granulation des brins refroidis en les découpant en pastilles,
de préférence dans lequel la poly(ε-caprolactone) est utilisée à l'étape 1 sous la forme de paillettes ou de matière broyée ayant un diamètre inférieur ou égal à 840 µm.

27. Procédé de préparation de la forme posologique pharmaceutique solide à libération prolongée selon l'une quelconque des revendications 1 à 25, comprenant les étapes suivantes :
1. malaxage de l'oxyde de polyéthylène, de l'agent actif et facultativement d'un ou plusieurs autres ingrédients, à l'exception de la poly(ε-caprolactone), pour former une première composition ;
2. introduction de la première composition de l'étape 1 dans une première trémie d'un premier distributeur volumétrique équipé d'un premier assemblage à simple vis ;
3. introduction de la poly(ε-caprolactone) en tant que seconde composition dans une seconde trémie d'un second distributeur volumétrique équipé d'un second assemblage à vis plus grand que le premier assemblage à vis ;
4. étalonnage du taux d'alimentation des deux distributeurs en fonction de la proportion relative de la première et de la seconde composition pour obtenir un taux d'alimentation total ;
5. dosage des première et seconde compositions dans une extrudeuse à double vis et traitement de l'extrudat résultant à température élevée pour former des brins ;
6. étirage et refroidissement des brins de l'étape 5 ; et
7. granulation des brins refroidis de l'étape 6 en les découpant en pastilles.

28. Forme posologique pharmaceutique solide à libération prolongée pouvant être obtenue par un procédé selon l'une quelconque des revendications 26 ou 27.

29. Forme posologique pharmaceutique solide à libération prolongée selon l'une quelconque des revendications 1 à 25 pour utilisation dans le traitement de la douleur, dans laquelle l'agent actif est un analgésique opioïde.

30. Utilisation d'oxyde de polyéthylène ayant un poids moléculaire moyen approximatif en poids d'environ 10 000 à moins de 1 000 000 dans une formulation matricielle à libération prolongée sous une forme posologique pharmaceutique solide à libération prolongée, dans laquelle la formulation matricielle à libération prolongée comprend en outre un agent actif et de la poly(ε-caprolactone) permettant de conférer à la forme posologique solide à libération prolongée une résistance à l'extraction à l'alcool.

31. Utilisation de poly(ε-caprolactone) ayant un poids moléculaire moyen approximatif en nombre d'environ 10 000 à environ 200 000 dans une formulation matricielle à libération prolongée sous une forme posologique pharmaceutique solide à libération prolongée, dans laquelle la formulation matricielle à libération prolongée comprend en outre un agent actif et de l'oxyde de polyéthylène ayant un poids moléculaire moyen approximatif en poids d'environ 10 000 à moins de 1 000 000 permettant de conférer à la forme posologique solide à libération prolongée une résistance au broyage.
